(19) Europäisches Patentamt
European Patent Office
Office européen des brevets

(11) EP 1 718 613 B1

(12) # EUROPEAN PATENT SPECIFICATION

(45) Date of publication and mention of the grant of the patent:
**03.09.2008 Bulletin 2008/36**

(21) Application number: **05715507.9**

(22) Date of filing: **22.02.2005**

(51) Int Cl.:
***C07D 213/74*** *(2006.01)* ***A61K 31/44*** *(2006.01)*
***A61P 25/06*** *(2006.01)* ***A61P 25/08*** *(2006.01)*

(86) International application number:
**PCT/EP2005/001938**

(87) International publication number:
**WO 2005/080342 (01.09.2005 Gazette 2005/35)**

(54) **PYRIDINE DERIVATIVES AND THEIR USE AS CB2 RECEPTOR MODULATORS**

PYRIDINDERIVATE UND DEREN VERWENDUNG ALS MODULATOREN DES CB2-REZEPTORS

DERIVES DE PYRIDINE ET LEUR UTILISATION EN TANT QUE MODULATEURS DU RECEPTEUR CB2

(84) Designated Contracting States:
**AT BE BG CH CY CZ DE DK EE ES FI FR GB GR HU IE IS IT LI LT LU MC NL PL PT RO SE SI SK TR**
Designated Extension States:
**HR LV**

(30) Priority: **24.02.2004 GB 0404104**
**12.11.2004 GB 0425069**

(43) Date of publication of application:
**08.11.2006 Bulletin 2006/45**

(73) Proprietor: **GLAXO GROUP LIMITED**
**Greenford, Middlesex UB6 ONN (GB)**

(72) Inventors:
- **EATHERTON, Andrew John, GlaxoSmithKline Harlow, Essex CM19 5AW (GB)**
- **GIBLIN, Gerard Martin Paul, GlaxoSmithKline Harlow, Essex CM19 5AW (GB)**
- **MITCHELL, William Leonard, GlaxoSmithKline Harlow, Essex CM19 5AW (GB)**
- **NAYLOR, Alan, GlaxoSmithKline Harlow, Essex CM19 5AW (GB)**

(74) Representative: **Knight, Lucie Viktoria**
**GlaxoSmithKline Services Unlimited**
**980 Great West Road**
**Brentford, Middlesex TW8 9GS (GB)**

(56) References cited:
**EP-A- 0 576 357** **US-A- 5 112 820**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 718 613 B1

## Description

The present invention relates to novel pyridine derivatives, pharmaceutical compositions containing these compounds and their use in the treatment of diseases, particularly pain, which diseases are caused directly or indirectly by an increase or decrease in activity of the cannabinoid receptor.

Cannabinoids are a specific class of psychoactive compounds present in Indian cannabis (*Cannabis sativa*), including about sixty different molecules, the most representative being cannabinol, cannabidiol and several isomers of tetrahydrocannabinol. Knowledge of the therapeutic activity of cannabis dates back to the ancient dynasties of China, where, 5,000 years ago, cannabis was used for the treatment of asthma, migraine and some gynaecological disorders. These uses later became so established that, around 1850, cannabis extracts were included in the US Pharmacopaeia and remained there until 1947.

Cannabinoids are known to cause different effects on various systems and/or organs, the most important being on the central nervous system and on the cardiovascular system. These effects include alterations in memory and cognition, euphoria, and sedation. Cannabinoids also increase heart rate and vary systemic arterial pressure. Peripheral effects related to bronchial constriction, immunomodulation, and inflammation have also been observed. The capability of cannabinoids to reduce intraocular pressure and to affect respiratory and endocrine systems is also well documented. See e.g. L.E. Hollister, Health Aspects of Cannabis, Pharmacological Reviews, Vol. 38, pp. 1-20, (1986). More recently, it was found that cannabinoids suppress the cellular and humoral immune responses and exhibit antiinflammatory properties. Wirth et al., Antiinflammatory Properties ofCannabichrome, Life Science, Vol. 26, pp. 1991-1995, (1980).

In spite of the foregoing benefits, the therapeutic use of cannabis is controversial, both due to its relevant psychoactive effects (causing dependence and addiction), and due to manifold side effects that have not yet been completely clarified. Although work in this field has been ongoing since the 1940's, evidence indicating that the peripheral effects of cannabinoids are directly mediated, and not secondary to a CNS effect, has been limited by the lack of receptor characterization, the lack of information concerning an endogenous cannabinoid ligand and, until recently, the lack of receptor subtype selective compounds.

The first cannabinoid receptor was found to be mainly located in the brain, in neural cell lines, and, only to a lesser extent, at the peripheral level. In view of its location, it was called the central receptor ("CB1"). See Matsuda et al., "Structure of a Cannabinoid Receptor and Functional Expression of the Cloned cDNA," Nature, Vol. 346, pp. 561-564 (1990. The second cannabinoid receptor ("CB2") was identified in the spleen, and was assumed to modulate the non psychoactive effects of the cannabinoids. See Munro et el., "Molecular Characterization of a Peripheral Receptor for Cannabinoids," Nature, Vol. 365, pp. 61-65 (1993).

Recently, some compounds have been prepared which are capable of acting as agonists on both the cannabinoid receptors. For example, use of derivatives of dihydroxypyrrole-(1,2,3-d,e)-1,4-benzoxazine in the treatment of glaucoma and the use of derivatives of 1,5-diphenyl-pyrazole as immunomodulators or psychotropic agents in the treatment of various neuropathologies, migraine, epilepsy, glaucoma, etc are known. See U.S. Patent No. 5,112,820 and EP 576357, respectively. However, because these compounds are active on both the CB1 and CB2 receptor, they can lead to serious psychoactive effects.

The foregoing indications and the preferential localization of the CB2 receptor in the immune system confirms a specific role of CB2 in modulating the immune and antiinflammatory response to stimuli of different sources.

The total size of the patient population suffering from pain is vast (almost 300 million), dominated by those suffering from back pain, osteo-arthritic pain and post-operative pain. Neuropathic pain (associated with neuronal lesions such as those induced by diabetes, HIV, herpes infection, or stroke) occurs with lower, but still substantial prevalence, as does cancer pain.

The pathogenic mechanisms that give rise to pain symptoms can be grouped into two main categories:

- those that are components of inflammatory tissue responses (Inflammatory Pain);
- those that result from a neuronal lesion of some form (Neuropathic Pain).

Chronic inflammatory pain consists predominantly of osteoarthritis, chronic low back pain and rheumatoid arthritis. The pain results from acute and on-going injury and/or inflammation. There may be both spontaneous and provoked pain.

There is an underlying pathological hypersensitivity as a result of physiological hyperexcitability and the release of inflammatory mediators which further potentiate this hyperexcitability. CB2 receptors are expressed on inflammatory cells (T cells, B cells, macrophages, mast cells) and mediate immune suppression through inhibition of cellular interaction/ inflammatory mediator release. CB2 receptors may also be expressed on sensory nerve terminals and therefore directly inhibit hyperalgesia.

The role of CB2 in immunomodulation, inflammation, osteoporosis, cardiovascular, renal and other disease conditions is now being examined. In light of the fact that cannabinoids act on receptors capable of modulating different

functional effects, and in view of the low homology between CB2 and CB1, the importance of developing a class of drugs selective for the specific receptor sub-type is evident The natural or synthetic cannabinoids currently available do not fulfil this function because they are active on both receptors.

Based on the foregoing, there is a need for compounds which are capable of selectively modulating the receptor for cannabinoids and, therefore, the pathologies associated with such receptors. Thus, CB2 modulators offer a unique approach toward the pharmacotherapy of immune disorders, inflammation, osteoporosis, renal ischemia and other pathophysiological conditions.

The present invention provides novel pyridine derivatives of formula (I) and pharmaceutically acceptable derivatives thereof, pharmaceutical compositions containing these compounds or derivatives, and their use as CB2 receptor modulators, which are useful in the treatment of a variety of disorders.

The present invention further comprises a method for treating disease mediated by CB2 receptors in an animal, including humans, which comprises administering to an animal in need thereof an effective amount of a compound of formula (I) or a pharmaceutically acceptable derivative thereof.

The invention provides compounds of formula (I):

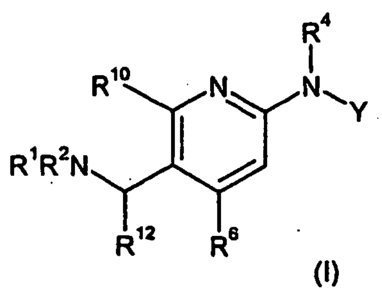

(I)

wherein:

Y is phenyl, unsubstituted or substituted with one, two or three substituents;
$R^1$ is selected from hydrogen, $C_{1-6}$ alkyl, $C_{3-6}$ cycloalkyl, or halosubstituted$C_{1-6}$ alkyl;
$R^2$ is $(CH_2)_mR^3$ where m is 0 or 1;
or $R^1$ and $R^2$ together with N to which they are attached form an unsubstituted or substituted 4- to 8- membered non-aromatic heterocyclyl ring;
$R^3$ is an unsubstituted or substituted 4- to 8- membered non-aromatic heterocyclyl group, an unsubstituted or substituted $C_{3-8}$ cycloalkyl group, an unsubstituted or substituted straight or branched $C_{1-10}$ alkyl, an unsubstituted or substituted $C_{5-7}$ cycloalkenyl, $R^5$ or $R^3$ is an unsubstituted or substituted 5- to 6- membered aromatic heterocyclyl group, or group A:

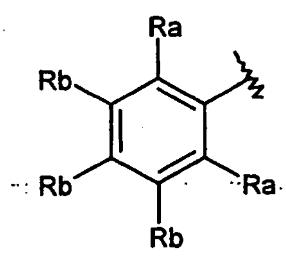

(A)

$R^4$ is selected from hydrogen, $C_{1-6}$ alkyl, $C_{3-6}$ cycloalkyl, or halosubstituted$C_{1-6}$ alkyl, $COCH_3$, or $SO_2Me$;
$R^5$ is

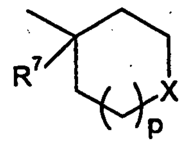

wherein p is 0, 1 or 2, and X is $CH_2$, O, S, SO or $SO_2$;

$R^6$ is halo, an substituted or unsubstituted $(C_{1-6})$alkyl, substituted or unsubstituted $(C_{3-6})$cycloalkyl, or a 4- to 7-membered non aromatic heterocyclyl group and $R^{10}$ is hydrogen or $R^{10}$ is halo, an substituted or unsubstituted $(C_{1-6})$alkyl, substituted or unsubstituted $(C_{3-6})$cycloalkyl, or a 4- to 7- membered non aromatic heterocyclyl group and $R^6$ is hydrogen:

$R^7$ is OH, $C_{1-6}$alkoxy, $NR^{8a}R^{8b}$, $NHCOR^9$, $NHSO_2R^9$, $SOqR^9$;

$R^{8a}$ is H or $C_{1-6}$alkyl;

$R^{8b}$ is H or $C_{1-6}$alkyl;

$R^9$ is $C_{1-6}$alkyl;

$R^{12}$ is hydrogen or $C_{1-6}$alkyl;

q is 0, 1 or 2;

Ra can be independently selected from hydrogen, fluoro, chloro or trifluoromethyl;

Rb can be independently be selected from hydrogen, $C_{1-6}$ alkyl, $C_{1-6}$ alkoxy, halo$C_{1-6}$ alkoxy, hydroxy, cyano, halo, sulfonyl, $CONH_2$, COOH or $NHCOOC_{1-6}$alkyl;

and pharmaceutically acceptable derivatives thereof.

In one embodiment Y is a substituted phenyl. In one embodiment Y is substituted by 1 or 2 substituents.

In one embodiment $R^1$ is hydrogen.

In one embodiment $R^4$ is $C_{1-6}$ alkyl or hydrogen, for example methyl or hydrogen, alternatively hydrogen.

In one embodiment $R^6$ is isopropyl, cyclopropyl, trifluoromethyl, *t*-butyl or cyclopentyl.

In one embodiment $R^6$ is isopropyl, cyclopropyl, trifluoromethyl or *t*-butyl.

In one embodiment $R^{10}$ is hydrogen

In one embodiment $R^7$ is OH.

In one embodiment X is $CH_2$.

When Y is substituted, the substituent or substituents may be selected from: $C_{1-6}$ alkyl, halosubstituted$C_{1-6}$ alkyl, $C_{1-6}$ alkoxy, a hydroxy group, a cyano group, halo, a $C_{1-6}$alkyl sulfonyl group, $-CONH_2$,$-NHCOCH_3$, -COOH, halosubstituted $C_{1-6}$ alkoxy, or $SO_2NR^{8a}R^{8b}$ wherein $R^{8a}$ and $R^{8b}$ are as defined above.

In one embodiment Y is substituted by halo, cyano, methyl, trifluoromethyl, methoxy or trifluoromethoxy.

In one embodiment $R^3$ is an unsubstituted or substituted 4- to 8- membered non-aromatic heterocyclyl group, an unsubstituted or substituted $C_{3-8}$ cycloalkyl group or a straight or branched $C_{1-6}$alkyl group.

In one embodiment $R^3$ is an unsubstituted or substituted 4- to 8- membered non-aromatic heterocyclyl group, or an unsubstituted or substituted $C_{3-8}$ cycloalkyl group.

In one particular embodiment $R^3$ is pyridinyl, pyrimidinyl, imidazoyl, oxadiazoyl, triazolyl or pyrazinyl any of which can be unsubstituted or substituted or is group A.

In one particular embodiment $R^3$ is pyridinyl, pyrimidinyl, imidazoyl, oxadiazoyl, triazolyl or pyrazinyl any of which can be unsubstituted or substituted or is group A.

In one particular embodiment $R^3$ is group A, pyridinyl or pyrimidinyl.

In a further embodiment $R^3$ is group A or pyridinyl.

In one embodiment when $R^3$ is an unsubstituted or substituted 4- to 8- membered non-aromatic heterocyclyl group it can be morpholinyl, tertahydropyranyl, thiomorpholine-s,s-dioxide, or tetrahydrothiopyran-1,1-dioxide.

In one particular embodiment Rb is selected from halo, methoxy, and cyano.

In one embodiment compounds of formula (I) are compounds of formula (Ia):

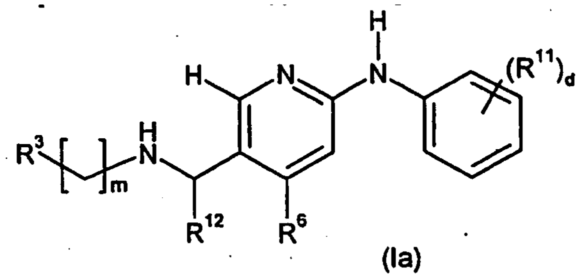

(Ia)

wherein

$R^3$ is an unsubstituted or substituted 4- to 8- membered non-aromatic heterocyclyl group, an unsubstituted or substituted $C_{3-8}$ cycloalkyl group or a straight or branched $C_{1-6}$alkyl group;
$R^6$ is isopropyl, cyclopropyl, trifluoromethyl, *t*-butyl or cyclopentyl.
$R^{11}$ is selected from halo, cyano, methyl, trifluoromethyl, methoxy or trifluoromethoxy;
$R^{12}$ is hydrogen or $C_{1-6}$alkyl;
d is 0, 1, 2 or 3;
m is 0 or 1
and pharmaceutically acceptable derivatives thereof.

In one embodiment $R^3$ is $(C_{3-6})$cycloakl for example cyclobutyl, cyclopropyl or cyclopentyl.

In one embodiment $R^3$ is tetroahydropyran.

In one embodiment compounds of formula (I) are compounds of formula (Ib):

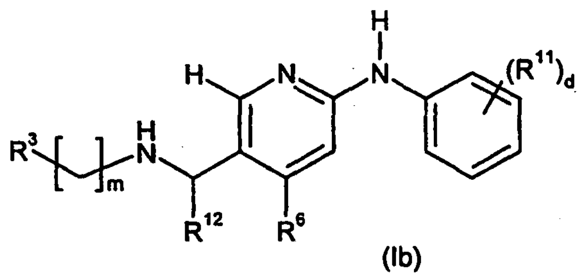

(Ib)

$R^3$ is an optionally substituted 5- to 6- membered aromatic heterocyclyl group, or group A:
$R^6$ is isopropyl, cyclopropyl, trifluoromethyl, *t*-butyl or cyclopentyl;
$R^{11}$ is selected from halo, cyano, methyl, trifluoromethyl, methoxy or trifluoromethoxy;
$R^{12}$ is hydrogen or $C_{1-6}$alkyl;
d is 0, 1,2 or 3;
m is 0 or 1;
and pharmaceutically acceptable derivatives thereof.

In one embodiment compounds of formula (I) are compounds of formula (Ic):

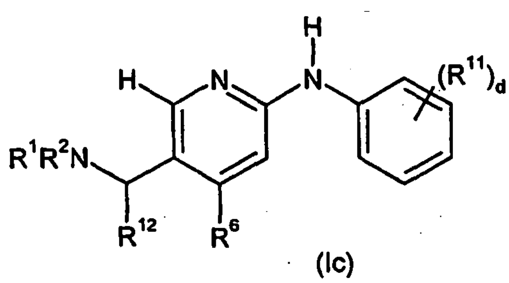

(Ic)

$R^1$ and $R^2$ together with N to which they are attached form an unsubstituted or substituted 4- to 8- membered non-aromatic heterocyclyl ring;
$R^6$ is isopropyl, cyclopropyl, trifluoromethyl, *t*-butyl or cyclopentyl;
$R^{11}$ is selected from halo, cyano, methyl, trifluoromethyl, methoxy or trifluoromethoxy;

$R^{12}$ is hydrogen or $C_{1-6}$alkyl;
d is 0, 1, 2 or 3;
and pharmaceutically acceptable derivatives thereof.

In one embodiment $R^6$ is isopropyl

In one embodiment $R^{12}$ is hydrogen or methyl, suitably hydrogen.

In one embodiment $R^1$ and $R^2$ together with N to which they are attached form an azetinyl, pyrrolidinyl, piperidinyl, morpholinyl or thiomorpholinyl ring.

When $R^1$ and $R^2$ together with N to which they are attached form a 4- to 8- membered non-aromatic heterocyclyl ring which is substituted, or when $R^3$ is substituted, the substituent or substituents may be selected from: $C_{1-6}$ alkyl, $C_{1-6}$ alkoxy, a hydroxy group, halosubstituted $C_{1-6}$alkyl, halosubstituted $C_{1-6}$alkox, a cyano group, halo or a sulfonyl group, methylsulfonyl, $NR^{8a}R^{8b}$, $CONH_2$, $NHCOCH_3$, (=O), COOH, $CONHCH_3$ and $NHSO_2CH_3$ wherein $R^{8a}$ and $R^{8b}$ are as described above.

In one particular embodiment when $R^3$ is an 5- to 6- membered aromatic heterocyclyl group the substituents are halo, methoxy, and cyano.

When $R^1$ and $R^2$ together with N to which they are attached form a 4- to 8- membered non-aromatic heterocyclyl ring which is substituted, or when $R^3$ is substituted there can be 1, 2 or 3 substituents.

When $R^6$ is substitued by 1, 2 or 3 substitutents the substituent or substituents may be selected from OH, halo, cyano, $C_{1-6}$alkoxy, $NR^{8a}R^{8b}$, $NHCOR^9$, $NHSO_2R^9$, $SOqR^9$; wherein $R^{8a}$, $R^{8b}$, $R^9$, and q are defined above.

In one particular embodiment the compounds are selective for CB2 over CB 1. Preferably the compounds are 50 fold selective i.e. compounds of formula (I) have an $EC_{50}$ value at the cloned human cannabinoid CB2 receptor of at least 50 times the $EC_{50}$ values at the cloned human cannabinoid CB1 receptor or have less than 10% efficacy at the CB1 receptor.

The invention is described using the following definitions unless otherwise indicated.

The term "pharmaceutically acceptable derivative" means any pharmaceutically acceptable salt, ester, salt of such ester or solvate of the compounds of formula (I), or any other compound which upon administration to the recipient is capable of providing (directly or indirectly) a compound of formula (I) or an active metabolite or residue thereof.

It will be appreciated by those skilled in the art that compounds of formula (I) may be modified to provide pharmaceutically acceptable derivatives thereof at any of the functional groups in the compounds, and that the compounds of formula (I) may be derivatised at more than one position.

It will be appreciated that, for pharmaceutical use, the salts referred to above will be physiologically acceptable salts, but other salts may find use, for example in the preparation of compounds of formula (I) and the physiological acceptable salts thereof. Pharmaceutically acceptable salts include those described by Berge, Bighley and Monkhouse , J. Pharm. Sci., 1977, 66, 1-19. The term "pharmaceutically acceptable salts" includes salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc, and the like. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, trishydroxylmethyl amino methane, tripropyl amine, tromethamine, and the like. When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like.

Examples of pharmaceutically acceptable salts include the ammonium, calcium, magnesium, potassium, and sodium salts, and those formed from maleic, fumaric, benzoic, ascorbic, pamoic, succinic, hydrochloric, sulfuric, bismethylenesalicylic, methanesulfonic, ethanedisulfonic, propionic, tartaric, salicylic, citric, gluconic, aspartic, stearic, palmitic, itaconic, glycolic, p-aminobenzoic, glutamic, benzenesulfonic, cyclohexylsulfamic, phosphoric and nitric acids.

The terms 'halogen or halo' are used to represent fluorine, chlorine, bromine or iodine.

The term 'alkyl' as a group or part of a group means a straight or branched chain alkyl group or combinations thereof, for example a methyl, ethyl, n-propyl, i-propyl, n-butyl, s-butyl, t-butyl, pentyl, hexyl, 1,1-dimethylethyl, or combinations thereof.

The term 'alkoxy' as a group or as part of a group means a straight, branched or cyclic chain alkyl group having an oxygen atom attached to the chain, for example a methoxy, ethoxy, n-propoxy, i-propoxy, n-butoxy, s-butoxy, t-butoxy group, pentoxy, hexyloxy group, cyclopentoxy or cyclohexyloxy group.

The term 'cycloalkyl' means a closed 4- to 8- membered non-aromatic ring, for example cyclobutyl, cyclopentyl,

cyclohexyl or cycloheptyl, or cyclooctyl.

The term 'cycloalkenyl' means a closed non-aromatic carbon ring containing 1 or more double bonds, for example cyclobutenyl, cyclopentenyl, cyclohexenyl or cycloheptenyl, or cyclooctenyl.

The term 'alkynyl' as a group or part of a group means a straight or branched chain carbon chain or combinations containing 1 or more triple carbon bonds for example a ethynyl, propynyl, butynyl, pentynyl, hexynyl or combinations thereof.

The term 'aryl' means a 5- or 6- membered aromatic ring, for example phenyl, or a 7- to 12-membered bicyclic ring system where at least one of the rings is aromatic, for example naphthyl.

When $R^1$ and $R^2$ taken together with the N to which they are attached form an optionally substituted non-aromatic heterocyclyl ring, the ring may optionally contain 1, 2, 3 or 4 further hetero atoms. The ring may be saturated or unsaturated. In one embodiment the further hetero atoms are selected from oxygen, nitrogen or sulphur. An example of a 4 membered heterocyclyl ring is azetidinyl Examples of 5- membered heterocyclyl rings include pyrrolidinyl, Examples of 6-membered heterocyclyl rings are morpholinyl, piperizinyl, piperidinyl, tetrahydropyridinyl. An additional example is thiomorpholinyl. Examples of a 7- membered heterocyclyl ring are azapine or oxapine. Examples of 8-membered heterocyclyl rings are azacyclooctanyl, azaoxacyclooctanyl or azathiacyclooctanyl.

When $R^3$ or $R^6$ is an optionally substituted non-aromatic heterocyclyl group, the ring may contain 1, 2, 3, or 4 hetero atoms. In one embodiment the hetero atoms are selected from oxygen, nitrogen or sulphur. Examples of 4-membered groups are 2- or 3- azetidinyl, oxetanyl, thioxetanyl, thioxetanyl-s-oxide and thioxetanyl-s,s-dioxide. Examples of 5- membered heterocyclyl groups in this instance include dioxalanyl, pyrrolidinyl, tetrahydrofuranyl, tetrahydrothiophenyl and tetrahydrothiophenyl-s,s-dioxide. Examples of 6-membered heterocyclyl groups are morpholinyl, piperidinyl, piperazinyl, tetrahydropyranyl, tetrahydrothiopyranyl, tetrahydrothiopyranyl-s,s-dioxide, thiomorpholinyl, thiomorpholinyl-s,s-dioxide, tetrahydropyridinyl dioxanyl, and tetrahydro-thiopyran 1,1 dioxide. Examples of a 7- membered heterocyclyl ring are azapine or oxapine. Examples of 8- membered groups are azacyclooctanyl, azaoxacyclooctanyl or azathiacyclooctanyl, oxacylcooctanyl, or thiacyclooctanyl.

When $R^3$ is an aromatic heterocyclyl group, the ring may contain 1, 2, 3, or 4 hetero atoms. In one particular embodiment the hetero atoms are selected from oxygen, nitrogen or sulphur. Examples of 5- membered heterocyclyl groups in this instance include furanyl, dioxalanyl, pyrrolyl, oxazolyl, thiazolyl, imidazolyl, oxadiazolyl, thiadiazolyl, triazolyl, triazinyl, isothiazolyl, isoxazolyl, thienyl, pyrazolyl or tetrazolyl. Examples of 6-membered heterocyclyl groups are pyridyl, pyrizinyl, pyrimidinyl, pyrazinyl, triazinyl, or tetrazinyl.

Compounds of formula (I) when $R^{12}$ is hydrogen can be prepared as set forth in the following scheme:

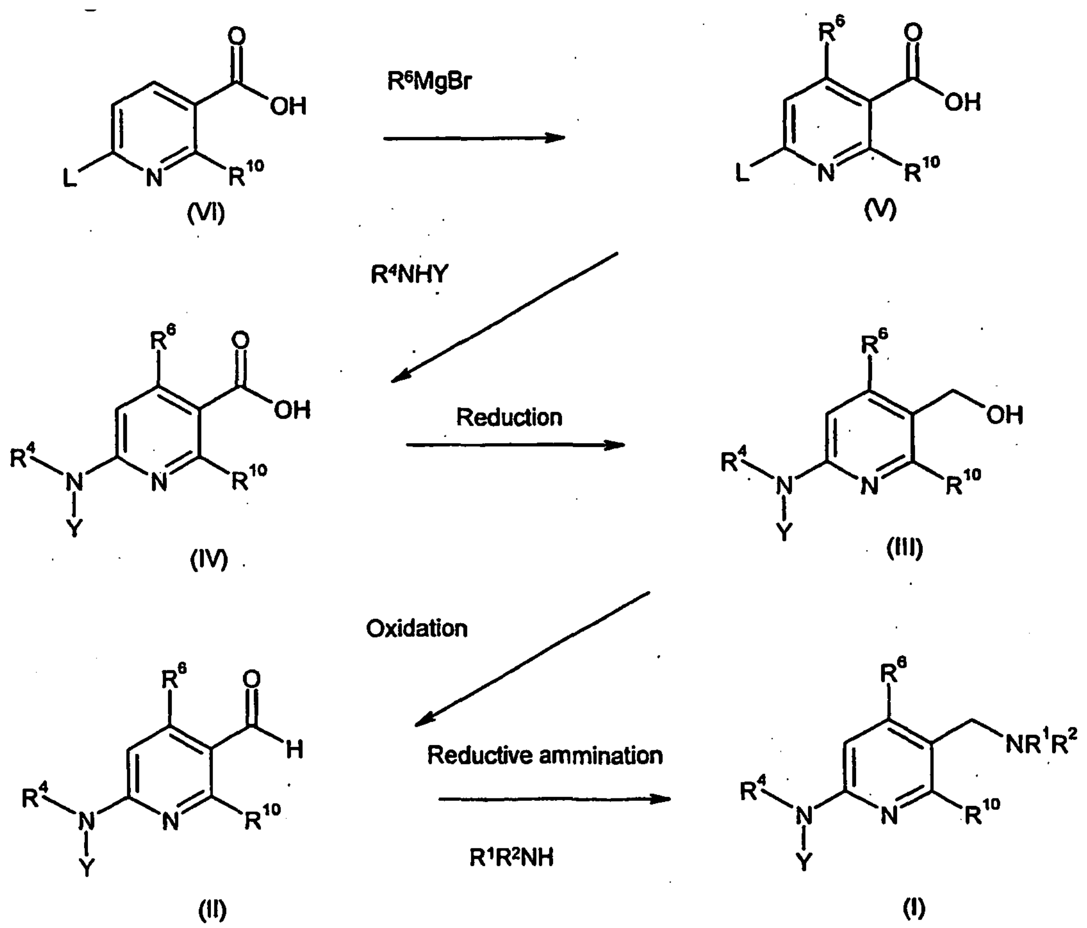


wherein L is a leaving group, for example halo, and $R^1$, $R^2$, $R^4$, $R^6$, $R^{10}$ and Y are as defined for compounds of formula (I).

Compounds of formula (I) when $R^{12}$ is hydrogen can be prepared as set forth in the following scheme 2:

Scheme 2

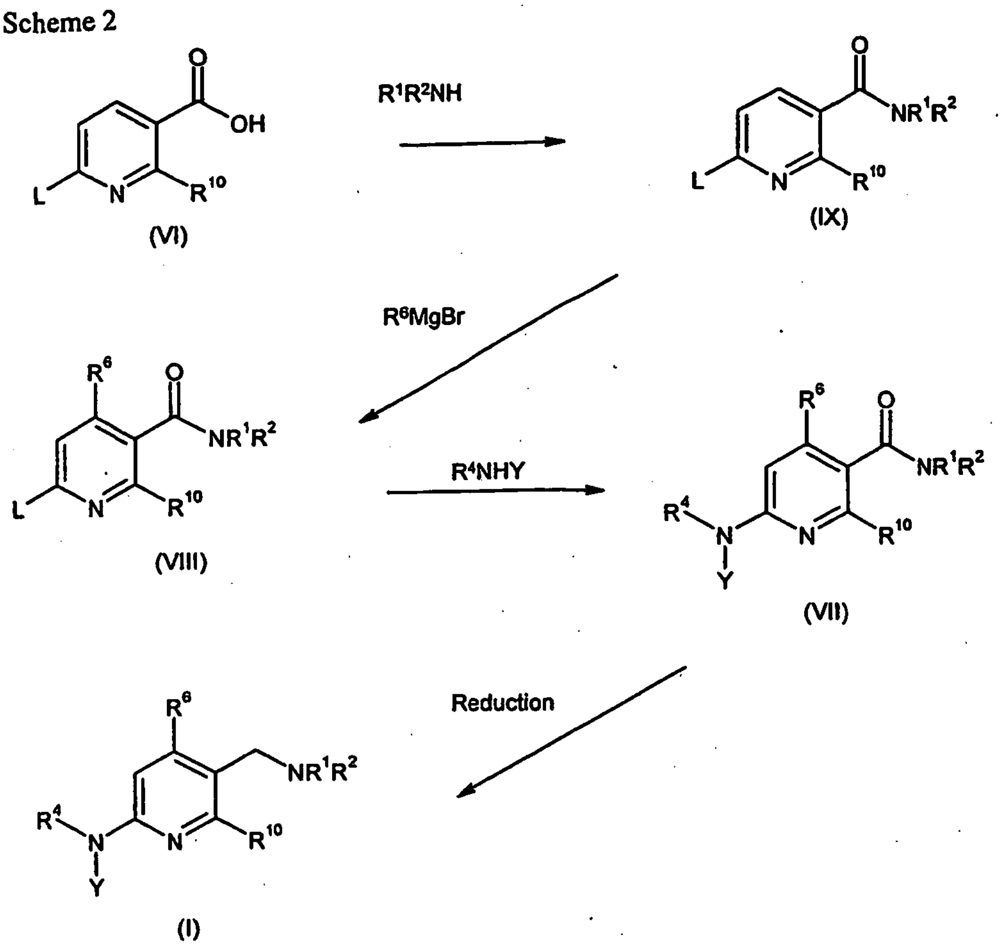


wherein L is a leaving group, for example halo, and $R^1$, $R^2$, $R^4$, $R^6$, $R^{10}$ and Y are as defined for compounds of formula (I).

Compounds of formula (I) where $R^{12}$ is other than hydrogen can be prepared by the following scheme 3 from compounds of formula (II) (prepared as set out in scheme 1):

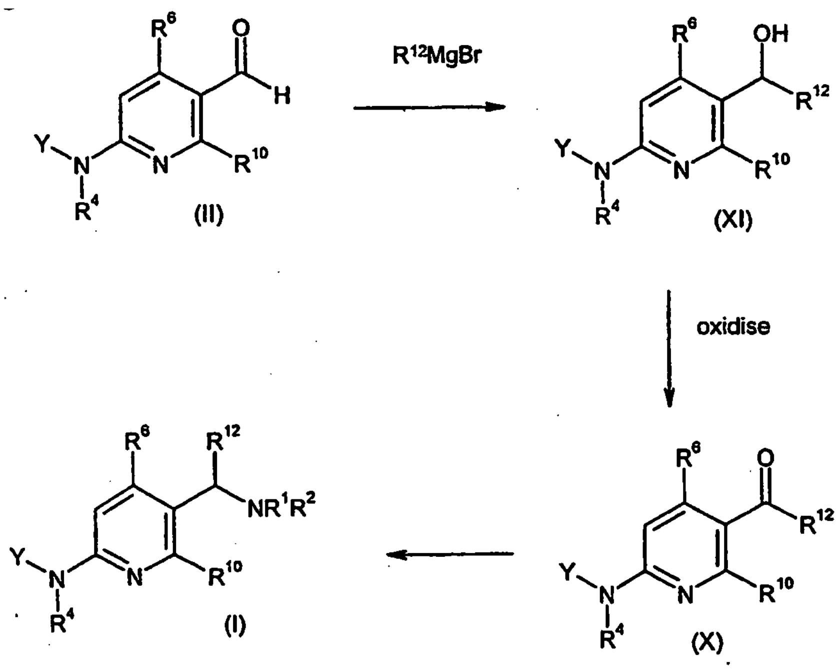

wherein $R^1$, $R^2$, $R^4$, $R^6$, $R^{10}$, $R^{12}$ and Y are as defined for compounds of formula (I) except $R^{12}$ is not hydrogen.

It is to be understood that the present invention encompasses all isomers of compounds of formula (I) and their pharmaceutically acceptable derivatives, including all geometric, tautomeric and optical forms, and mixtures thereof (e.g. racemic mixtures). Where additional chiral centres are present in compounds of formula (I), the present invention includes within its scope all possible diastereoismers, including mixtures thereof. The different isomeric forms may be separated or resolved one from the other by conventional methods, or any given isomer may be obtained by conventional synthetic methods or by stereospecific or asymmetric syntheses.

The subject invention also includes isotopically-labeled compounds, which are identical to those recited in formulas I and following, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine, iodine, and chlorine, such as $^3H$, $^{11}C$, $^{14}C$, $^{18}F$, $^{123}I$ and $^{125}I$.

Compounds of the present invention and pharmaceutically acceptable salts of said compounds that contain the aforementioned isotopes and/or other isotopes of other atoms are within the scope of the present invention. Isotopically-labeled compounds of the present invention, for example those into which radioactive isotopes such as $^3H$, $^{14}C$ are incorporated, are useful in drug and/or substrate tissue distribution assays. Tritiated, i.e., $^3H$, and carbon-14, i.e., $^{14}C$, isotopes are particularly preferred for their ease of preparation and detectability. $^{11}C$ and $^8F$ isotopes are particularly useful in PET (positron emission tomography), and $^{125}I$ isotopes are particularly useful in SPECT (single photon emission computerized tomography), all useful in brain imaging: Further, substitution with heavier isotopes such as deuterium, i.e., $^2H$, can afford certain therapeutic advantages resulting from greater metabolic stability, for example increased *in vivo* half-life or reduced dosage requirements and, hence, may be preferred in some circumstances. Isotopically labeled compounds of formula I and following of this invention can generally be prepared by carrying out the procedures disclosed in the Schemes and/or in the Examples below, by substituting a readily available isotopically labeled reagent for a non-isotopically labeled reagent.

The compounds of formula (I) may be prepared in crystalline or non-crystalline form, and, if crystalline, may optionally be hydrated or solvated. This invention includes within its scope stoichiometric hydrates or solvates as well as compounds containing variable amounts of water and/or solvent.

The compounds of the invention bind to the CB2 receptor, and therefore may be useful in treating CB2 receptor mediated diseases.

In view of their ability to bind to the CB2 receptor, the compounds of the invention may be useful in the treatment of the disorders that follow. Thus, the compounds of formula (I) may be useful as analgesics. For example they may be useful in the treatment of chronic inflammatory pain (e.g. pain associated with rheumatoid arthritis, osteoarthritis, rheumatoid spondylitis, gouty arthritis and juvenile arthritis) including the property of disease modification and joint structure preservation; musculoskeletal pain; lower back and neck pain; sprains and strains; neuropathic pain; sympathetically maintained pain; myositis; pain associated with cancer and fibromyalgia; pain associated with migraine; pain associated with influenza or other viral infections, such as the common cold; rheumatic fever; pain associated with functional bowel disorders such as non-ulcer dyspepsia, non-cardiac chest pain and irritable bowel syndrome; pain associated with myocardial ischemia; post operative pain; headache; toothache; and dysmenorrhea.

The compounds of the invention may also be useful disease modification or joint structure preservation in multiple sclerosis, rheumatoid arthritis, osteo-arthritis, rheumatoid spondylitis, gouty arthritis and juvenile arthritis.

The compounds of the invention may be particularly useful in the treatment of neuropathic pain. Neuropathic pain syndromes can develop following neuronal injury and the resulting pain may persist for months or years, even after the original injury has healed. Neuronal injury may occur in the peripheral nerves, dorsal roots, spinal cord or certain regions in the brain. Neuropathic pain syndromes are traditionally classified according to the disease or event that precipitated them. Neuropathic pain syndromes include: diabetic neuropathy; sciatica; non-specific lower back pain; multiple sclerosis pain; fibromyalgia; HIV-related neuropathy; post-herpetic neuralgia; trigeminal neuralgia; and pain resulting from physical trauma, amputation, cancer, toxins or chronic inflammatory conditions. These conditions are difficult to treat and although several drugs are known to have limited efficacy, complete pain control is rarely achieved. The symptoms of neuropathic pain are incredibly heterogeneous and are often described as spontaneous shooting and lancinating pain, or ongoing, burning pain. In addition, there is pain associated with normally non-painful sensations such as "pins and needles" (paraesthesias and dysesthesias), increased sensitivity to touch (hyperesthesia), painful sensation following innocuous stimulation (dynamic, static or thermal allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia).

The compounds of formula (I) may also be useful in the treatment of fever.

The compounds of formula (I) may also be useful in the treatment of inflammation, for example in the treatment of skin conditions (e.g. sunburn, burns, eczema, dermatitis, psoriasis); ophthalmic diseases such as glaucoma, retinitis, retinopathies, uveitis and of acute injury to the eye tissue (e.g. conjunctivitis); lung disorders (e.g. asthma, bronchitis,

emphysema, allergic rhinitis, respiratory distress syndrome, pigeon fancier's disease, farmer's lung, chronic obstructive pulmonary disease, (COPD); gastrointestinal tract disorders (e.g. aphthous ulcer, Crohn's disease, atopic gastritis, gastritis varialoforme, ulcerative colitis, coeliac disease, regional ileitis, irritable bowel syndrome, inflammatory bowel disease, gastroesophageal reflux disease); organ transplantation; other conditions with an inflammatory component such as vascular disease, migraine, periarteritis nodosa, thyroiditis, aplastic anaemia, Hodgkin's disease, sclerodoma, myaesthenia gravis, multiple sclerosis, sorcoidosis, nephrotic syndrome, Bechet's syndrome, polymyositis, gingivitis, myocardial ischemia, pyrexia, systemic lupus erythematosus, tendinitis, bursitis, and Sjogren's syndrome.

The compounds of formula (I) may also be useful in the treatment of bladder hyperrelexia following bladder inflammation.

The compounds of formula (I) may also be useful in the treatment of immunological diseases such as autoimmune diseases, immunological deficiency diseases or organ transplantation. The compounds of formula (I) may also be effective in increasing the latency of HIV infection.

The compounds of formula (I) may also be useful in the treatment of diseases of abnormal platelet function (e.g. occlusive vascular diseases).

The compounds of formula (I) may also be useful in the treatment of neuritis, heart burn, dysphagia, pelvic hypersensitivity, urinary incontinence, cystitis or pruritis.

The compounds of formula (I) may also be useful for the preparation of a drug with diuretic action.

The compounds of formula (I) may also be useful in the treatment of impotence or erectile dysfunction.

The compounds of formula (I) may also be useful for attenuating the hemodynamic side effects of non-steroidal anti-inflammatory drugs (NSAID's) and cyclooxygenase-2 (COX-2) inhibitors.

The compounds of formula (I) may also be useful in the treatment of neurodegenerative diseases and neurodegeneration such as dementia, particularly degenerative dementia (including senile dementia, Alzheimer's disease, Pick's disease, Huntingdon's chorea, Parkinson's disease and Creutzfeldt-Jakob disease, motor neuron disease); vascular dementia (including multi-infarct dementia); as well as dementia associated with intracranial space occupying lesions; trauma; infections and related conditions (including HIV infection); dementia in Parkinson's disease ; metabolism; toxins; anoxia and vitamin deficiency; and mild cognitive impairment associated with ageing, particularly Age Associated Memory Impairment. The compounds may also be useful for the treatment of amyotrophic lateral sclerosis (ALS) and neuroinflamation.

The compounds of formula (I) may also be useful in neuroprotection and in the treatment of neurodegeneration following stroke, cardiac arrest, pulmonary bypass, traumatic brain injury, spinal cord injury or the like.

The compounds of formula (I) may also be useful in the treatment of tinnitus.

The compounds of formula (I) may also be useful in the treatment of psychiatric disease for example schizophrenia, depression (which term is used herein to include bipolar depression, unipolar depression, single or recurrent major depressive episodes with or without psychotic features, catatonic features, melancholic features, atypical features or postpartum onset, seasonal affective disorder, dysthymic disorders with early or late onset and with or without atypical features, neurotic depression and social phobia, depression accompanying dementia for example of the Alzheimer's type, schizoaffective disorder or the depressed type, and depressive disorders resulting from general medical conditions including, but not limited to, myocardial infarction, diabetes, miscarriage or abortion, *etc*), anxiety disorders (including generalised anxiety disorder and social anxiety disorder), panic disorder, agoraphobia, social phobia, obsessive compulsive disorder and post-traumatic stress disorder, memory disorders, including dementia, amnesic disorders and age-associated memory impairment, disorders of eating behaviours, including anorexia nervosa and bulimia nervosa, sexual dysfunction, sleep disorders (including disturbances of circadian rhythm, dyssomnia, insomnia, sleep apnea and narcolepsy), withdrawal from abuse of drugs such as of cocaine, ethanol, nicotine, benzodiazepines, alcohol, caffeine, phencyclidine (phencyclidine-like compounds), opiates (e.g., heroin, morphine), amphetamine or amphetamine-related drugs (e.g. dextroamphetamine, methylamphetamine) or a combination thereof.

The compounds of formula (I) may also be useful in preventing or reducing dependence on, or preventing or reducing tolerance or reverse tolerance to, a dependence - inducing agent. Examples of dependence inducing agents include opioids (e.g. morphine), CNS depressants (e.g. ethanol), psychostimulants (e.g. cocaine) and nicotine.

The compounds of formula (I) may also be useful in the treatment of kidney dysfunction (nephritis, particularly mesangial proliferative glomerulonephritis, nephritic syndrome), liver dysfunction (hepatitis, cirrhosis), gastrointestinal dysfunction (diarrhoea) and colon cancer.

The term "treatment" or "treating" as used herein includes the treatment of established disorders and also includes the prophylaxis thereof. The term " prophylaxis" is used herein to mean preventing symptoms in an already afflicted subject or preventing recurrance of symptoms in an afflicted subject and is not limited to complete prevention of an affliction.

According to a further aspect of the invention, we provide a compound of formula (I) or a pharmaceutically acceptable derivative thereof for use in human or veterinary medicine.

According to another aspect of the invention, we provide a compound of formula (I) or a pharmaceutically

acceptable derivative thereof for use in the treatment of a condition which is mediated by the activity of cannabinoid 2 receptors.

In one embodiment the pain is selected from inflammatory pain, viseral pain, cancer pain, neuropathic pain, lower back pain, muscular sceletal, post operative pain, acute pain and migraine. For example, the inflammatory pain is pain associated with rheumatoid arthritis or osteoarthritis.

According to one aspect of the invention there is provided a compound of formula (I) or a pharmaceutically acceptable derivative thereof for use as a medicament in the treatment of pain.

According to another aspect of the invention there is provided the use of a compound of formula (I) or a pharmaceutically acceptable derivative thereof for the manufacture of a therapeutic agent for the treatment or prevention of a condition such as an immune disorder, an inflammatory disorder, pain, rheumatoid arthritis, multiple sclerosis, osteoarthritis or osteoporosis.

In order to use a compound of formula (I) or a pharmaceutically acceptable derivative thereof for the treatment of humans and other mammals it is normally formulated in accordance with standard pharmaceutical practice as a pharmaceutical composition. Therefore in another aspect of the invention is provided a pharmaceutical composition comprising a compound of formula (I) or a pharmaceutically acceptable derivative thereof adapted for use in human or veterinary medicine.

As used herein, "modulator" means both antagonist, partial or full agonist and inverse agonist. In one embodiment the present modulators are agonists.

Compounds of formula (I) and their pharmaceutically acceptable derivatives may be administered in a standard manner for the treatment of the indicated diseases, for example orally, parentarally, sub-lingually, dermally, intranasally, transdermally, rectally, via inhalation or via buccal administration.

Compounds of formula (I) and their pharmaceutically acceptable derivatives which are active when given orally can be formulated as liquids, tablets, capsules and lozenges. A liquid formulation will generally consist of a suspension or solution of the compound or salt in a liquid carrier for example, ethanol, olive oil, glycerine, glucose (syrup) or water with a flavouring, suspending, or colouring agent. Where the composition is in the form of a tablet, any pharmaceutical carrier routinely used for preparing solid formulations may be used. Examples of such carriers include magnesium stearate, terra alba, talc, gelatin, acacia, stearic acid, starch, lactose and sucrose. Where the composition is in the form of a capsule, any routine encapsulation is suitable, for example using the aforementioned carriers or a semi solid e.g. mono di-glycerides of capric acid, Gelucire™ and Labrasol™, or a hard capsule shell e.g gelatin. Where the composition is in the form of a soft shell capsule e.g. gelatin, any pharmaceutical carrier routinely used for preparing dispersions or suspensions may be considered, for example aqueous gums or oils, and are incorporated in a soft capsule shell.

Typical parenteral compositions consist of a solution or suspension of a compound or derivative in a sterile aqueous or non-aqueous carrier optionally containing a parenterally acceptable oil, for example polyethylene glycol, polyvinylpyrrolidone, lecithin, arachis oil or sesame oil.

Typical compositions for inhalation are in the form of a solution, suspension or emulsion that may be administered as a dry powder or in the form of an aerosol using a conventional propellant such as dichlorodifluoromethane or trichlorofluoromethane.

A typical suppository formulation comprises a compound of formula (I) or a pharmaceutically acceptable derivative thereof which is active when administered in this way, with a binding and/or lubricating agent, for example polymeric glycols, gelatins, cocoa-butter or other low melting vegetable waxes or fats or their synthetic analogs.

Typical dermal and transdermal formulations comprise a conventional aqueous or non-aqueous vehicle, for example a cream, ointment, lotion or paste or are in the form of a medicated plaster, patch or membrane.

In one embodiment the composition is in unit dosage form, for example a tablet, capsule or metered aerosol dose, so that the patient may administer a single dose.

Each dosage unit for oral administration contains suitably from 0.01 mg to 500 mg/Kg, and for example from 0.01 mg to 100 mg/Kg, and each dosage unit for parenteral administration contains suitably from 0.001 mg to 100 mg/Kg, of a compound of formula(I) or a pharmaceutically acceptable derivative thereof calculated as the free acid. Each dosage unit for intranasal administration contains suitably 1-400 mg and suitably 10 to 200 mg per person. A topical formulation contains suitably 0.01 to 5.0% of a compound of formula (I).

The daily dosage regimen for oral administration is suitably about 0.01 mg/Kg to 1000 mg/Kg, of a compound of formula(I) or a pharmaceutically acceptable derivative thereof calculated as the free acid. The daily dosage regimen for parenteral administration is suitably about 0.001 mg/Kg to 200 mg/Kg, of a compound of formula (I) or a pharmaceutically acceptable derivative thereof calculated as the free acid. The daily dosage regimen for intranasal administration and oral inhalation is suitably about 10 to about 500 mg/person. The active ingredient may be administered from 1 to 6 times a day, sufficient to exhibit the desired activity.

It may be advantageous to prepare the compounds of the present invention as nanoparticles. This may improve the oral bioavailability of the compounds. For the purposes of the present invention "nanoparticulate" is defined as solid particles with 50% of the particles having a particle size of less than 1$\mu$m, for example less than 0.75$\mu$m

The particle size of the solid particles of compound (I) may be determined by laser diffraction. A suitable machine for determining particle size by laser diffraction is a Lecotrac laser particle size analyser, using an HELOS optical bench fitted with a QUIXEL dispersion unit.

Numerous processes for the synthesis of solid particles in nanoparticulate form are known. Typically these processes involve a milling process, for example a wet milling process in the presence of a surface modifying agent that inhibits aggregation and/or crystal growth of the nanoparticles once created. Alternatively these processes may involve a precipitation process, for example, a process of precipitation in an aqueous medium from a solution of the drug in a non-aqueous solvent.

Accordingly, in a further aspect, the present invention provides a process for preparing compound (I) in nanoparticulate form as hereinbefore defined, which process comprises milling or precipitation.

Representative processes for the preparation of solid particles in nanoparticulate form are described in the patents and publications listed below. U.S. Patent No. 4,826,689 to Violanto & Fischer, U. S. Patent No. 5,145,684 to Liversidge et al U.S Patent No. 5,298,262 to Na & Rajagopalan, U.S. Patent No. 5,302,401 Liversidge et al U.S. Patent No. 5,336,507 to Na & Rajagopalan, U.S. Patent No. 5,340,564 to Illig & Sarpotdar U.S. Patent No. 5,346,702 to Na Rajagopalan, U.S. Patent No. 5,352,459 to Hollister et al U.S. Patent No. 5,354,560 to Lovrecich, U.S. Patent No. 5,384,124 to Courteille et al, U.S. Patent No. 5,429,824 to June, U.S. Patent No. 5,503,723 to Ruddy et al, U.S. Patent No. 5,510 118 to Bosch et al, U.S. Patent No. 5,518 to Bruno et al, U.S. Patent No. 5,518,738 to Eickhoff et al, U.S. Patent No. 5,534,270 to De Castro, U.S. Patent No. 5,536,508 to Canal et al, U.S. Patent No. 5,552,160 to Liversidge et al, U.S. Patent No. 5,560,931 to Eickhoff et al, U.S. Patent No. 5,560,932 to Bagchi et al, U.S. Patent No. 5,565,188 to Wong et al, U.S. Patent No. 5,571,536 to Eickhoff et al, U.S. Patent No. 5,573,783 to Desieno & Stetsko, U.S Patent No. 5,580,579 to Ruddy et al, U.S. Patent No 5,585,108 to Ruddy et al, U.S. Patent No. 5,587,143 to Wong, U.S. Patent No. 5,591456 to Franson et al, U.S. Patent No. 5,622,938 to Wong, U.S. Patent No 5,662,883 to Bagchi et al, U.S. Patent No. 5,665,331 to Bagchi et al, U.S Patent No. 5,718,919 to Ruddy et al, U.S. Patent No. 5,747,001 to Wiedmann et al, WO93/25190, WO96/24336, WO 97/14407, WO 98/35666, WO 99/65469, WO 00/18374, WO 00/27369, WO 00/30615 and WO 01/41760.

Such processes may be readily adapted for the preparation of compound (I) in nanoparticulate form. Such processes form a further aspect of the invention.

The process of the present invention may use a wet milling step carried out in a mill such as a dispersion mill in order to produce a nanoparticulate form of the compound. The present invention may be put into practice using a conventional wet milling technique, such as that described in Lachman et al., The Theory and Practice of Industrial Pharmacy, Chapter 2, "Milling" p.45 (1986).

In a further refinement, WO02/00196 (SmithKline Beecham plc) describes a wet milling procedure using a mill in which at least some of the surfaces are made of nylon (polyamide) comprising one or more internal lubricants, for use in the preparation of solid particles of a drug substance in nanoparticulate form.

In another aspect the present invention provides a process for preparing compounds of the invention in nanoparticulate form comprising wet milling a suspension of compound in a mill having at least one chamber and agitation means, said chamber(s) and/or said agitation means comprising a lubricated nylon, as described in WO02/00196.

The suspension of a compound of the invention for use in the wet milling is typically a liquid suspension of the coarse compound in a liquid medium. By "suspension" is meant that the compound is essentially insoluble in the liquid medium. Representative liquid media include an aqueous medium. Using the process of the present invention the average particle size of coarse compound of the invention may be up to 1mm in diameter. This advantageously avoids the need to pre-process the compound.

In a further aspect of the invention the aqueous medium to be subjected to the milling comprises compound (I) present in from about 1% to about 40% w/w, suitably from about 10% to about 30% w/w, for example about 20% w/w.

The aqueous medium may further comprise one or more pharmaceutically acceptable watersoluble carriers which are suitable for steric stabilisation and the subsequent processing of compound (I) after milling to a pharmaceutical composition, e.g. by spray drying. Pharmaceutically acceptable excipients most suitable for steric stabilisation and spray-drying are surfactants such as poloxamers, sodium lauryl sulphate and polysorbates etc; stabilisers such as celluloses e.g. hydroxypropylmethyl cellulose; and carriers such as carbohydrates e.g. mannitol.

In a further aspect of the invention the aqueous medium to be subjected to the milling may further comprise hydroxypropylmethyl cellulose (HPMC) present from about 0.1 to about 10% w/w.

The process of the present invention may comprise the subsequent step of drying compound of the invention to yield a powder.

Accordingly, in a further aspect, the present invention provides a process for preparing a pharmaceutical composition contain a compound of the present invention which process comprises producing compound of formula (I) in nanoparticulate form optionally followed by drying to yield a powder.

A further aspect of the invention is a pharmaceutical composition comprising a compound of formula (I) or a pharmaceutically acceptable deriviate thereof in which the compound of formula (I) or a pharmaceutically acceptable

deriviate thereof is present in solid particles in nanoparticulate form, in admixture with one or more pharmaceutically acceptable carriers or excipients.

By "drying" is meant the removal of any water or other liquid vehicle used during the process to keep compound of formula (I) in liquid suspension or solution. This drying step may be any process for drying known in the art, including freeze drying, spray granulation or spray drying. Of these methods spray drying is particularly preferred. All of these techniques are well known in the art. Spray drying/fluid bed granulation of milled compositions is carried out most suitably using a spray dryer such as a Mobile Minor Spray Dryer [Niro, Denmark], or a fluid bed drier, such as those manufactured by Glatt, Germany.

In a further aspect the invention provides a pharmaceutical composition as hereinbefore defined, in the form of a dried powder, obtainable by wet milling solid particles of compound of formaula (I) followed by spray-drying the resultant suspension.

In one embodiment, the pharmaceutical composition as hereinbefore defined, further comprises HPMC present in less than 15% w/w, for example, in the range 0.1 to 10% w/w.

The $CB_2$ receptor compounds for use in the instant invention may be used in combination with other therapeutic agents, for example COX-2 inhibitors, such as celecoxib, deracoxib, rofecoxib, valdecoxib, parecoxib or COX-189; 5-lipoxygenase inhibitors; NSAID's, such as aspirin, diclofenac, indomethacin, nabumetone or ibuprofen; leukotriene receptor antagonists; DMARD's such as methotrexate; adenosine A1 receptor agonists; sodium channel blockers, such as lamotrigine; NMDA receptor modulators, such as glycine receptor antagonists; gabapentin and related compounds; tricyclic antidepressants such as amitriptyline; neurone stabilising antiepileptic drugs; mono-aminergic uptake inhibitors such as venlafaxine; opioid analgesics; local anaesthetics; $5HT_1$ agonists, such as triptans, for example sumatriptan, naratriptan, zolmitriptan, eletriptan, frovatriptan, almotriptan or rizatriptan; $EP_1$ receptor ligands, $EP_4$ receptor ligands; $EP_2$ receptor ligands; $EP_3$ receptor ligands; $EP_4$ antagonists; $EP_2$ antagonists and $EP_3$ antagonists; bradykinin receptor ligands and vanilloid receptor ligand, antirheumatoid arthritis drugs, for example anti TNF drugs e.g. enbrel, remicade, anti-IL-1 drugs, or DMARDS e.g. leflunamide. When the compounds are used in combination with other therapeutic agents, the compounds may be administered either sequentially or simultaneously by any convenient route.

Additional COX-2 inhibitors are disclosed in US Patent Nos. 5,474,995 US5,633,272; US5,466,823, US6,310,099 and US6,291,523; and in WO 96/25405, WO 97/38986, WO 98/03484, WO 97/14691, WO99/12930, WO00/26216, WO00/52008, WO00/38311, WO01/58881 and WO02/18374.

Compounds of the present invention may be administered in combination with other active substances such as 5HT3 antagonists, NK-1 antagonists, serotonin agonists, selective serotonin reuptake inhibitors (SSRI), noradrenaline re-uptake inhibitors (SNRI), tricyclic antidepressants and/or dopaminergic antidepressants.

Suitable 5HT3 antagonists which may be used in combination of the compound of the inventions include for example ondansetron, granisetron, metoclopramide.

Suitable serotonin agonists which may be used in combination with the compound of the invention include sumatriptan, rauwolscine, yohimbine, metoclopramide.

Suitable SSRIs which may be used in combination with the compound of the invention include fluoxetine, citalopram, femoxetine, fluvoxamine, paroxetine, indalpine, sertraline, zimeldine.

Suitable SNRIs which may be used in combination with the compound of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

Compounds of the present invention may be used in combination with PDE4 inhibitors. The PDE4 inhibitor useful in this invention may be any compound that is known to inhibit the PDE4 enzyme or which is discovered to act as a PDE4 inhibitor, and which is only or essentially only a PDE4 inhibitor, not compounds which inhibit to a degree of exhibiting a therapeutic effect other members of the PDE family as well as PDE4. Generally it is preferred to use a PDE4 antagonist which has an $IC_{50}$ ratio of about 0.1 or greater as regards the $IC_{50}$ for the PDE4 catalytic form which binds rolipram with a high affinity divided by the $IC_{50}$ for the form which binds rolipram with a low affinity. Compounds of the present invention or combinations with PDE4 can be used in treating inflammation and as bronchodilators.

It turns out that there are at least two binding forms on human monocyte recombinant PDE 4 (hPDE 4) at which inhibitors bind. One explanation for these observations is that hPDE 4 exists in two distinct forms. One binds the likes of rolipram and denbufylline with a high affinity while the other binds these compounds with a low affinity. The preferred PDE4 inhibitors of for use in this invention will be those compounds which have a salutary therapeutic ratio, i.e., compounds which preferentially inhibit cAMP catalytic activity where the enzyme is in the form that binds rolipram with a low affinity, thereby reducing the side effects which apparently are linked to inhibiting the form which binds rolipram with a high affinity. Another way to state this is that the preferred compounds will have an $IC_{50}$ ratio of about 0.1 or greater as regards the $IC_{50}$ for the PDE 4 catalytic form which binds rolipram with a high affinity divided by the $IC_{50}$ for the form

which binds rolipram with a low affinity.

Reference is made to U.S. patent 5,998,428, which describes these methods in more detail.

Suitably are those PDE4 inhibitors which have an $IC_{50}$ ratio of greater than 0.5, and particularly those compounds having a ratio of greater than 1.0.

A further aspect of the invention is an CB2 modulator in combination with a PDE4 inhibitor and pharmaceutical compositions comprising said combination.

A further aspect of the invention is a method of treating lung disorders for example asthma, bronchitis, emphysema, allergic rhinitis, respiratory distress syndrome, pigeon fancier's disease, farmer's lung, chronic obstructive pulmonary disease, (COPD) and cough or a disorder which can be treated with a broncodilator which comprises administering to a mammal including man, an effective amount of a CB modulator or a pharmaceutically acceptable derivative therefore and an effective amount of a PDE4 inhibitor or a pharmaceutically acceptable derivative thereof.

An additional aspect of the invention is the use of an effective amount of a CB2 modulator or a pharmaceutically acceptable derivative therefore and an effective amount of a PDE4 inhibitor or a pharmaceutically acceptable derivative thereof in the manufacture of a medicament in the treatment of lung disorders for example asthma, bronchitis, emphysema, allergic rhinitis, respiratory distress syndrome, pigeon fancier's disease, farmer's lung, chronic obstructive pulmonary disease, (COPD) and cough or for the manufacture of a brocodilator.

When used herein cough can have a number of forms and includes productive, non-productive, hyper-reactive, asthma and COPD associated.

A further aspect of the invention is a patient pack comprsing an effective amount of a CB 2 modulator or a pharmaceutically acceptable derivative therefore and an effective amount of a PDE4 inhibitor or a pharmaceutically acceptable derivative

Possible PDE4 compounds are *cis* [cyano-4-(3-cyclopentyloxy-4-methoxyphenyl)cyclohexan-1-carboxylate] also known as cilomilast or Ariflo®, 2-carbomethoxy-4-cyano-4-(3-cyclopropylmethoxy-4-difluoromethoxyphenyl)cyclohexan-1-one, and *cis* [4-cyano-4-(3-cyclopropylmethoxy-4-difluoromethoxyphenyl)cyclohexan-1-ol]. They can be made by the processed described in US patents 5,449,686 and 5,552,438. Other PDE4 inhibitors, specific inhibitors, which can be used in this invention are AWD-12-281 from ASTA MEDICA (Hofgen, N. *et al*. 15th EFMC Int Symp Med Chem (Sept 6-10, Edinburgh) 1998, Abst P.98); a 9-benzyladenine derivative nominated NCS-613 (INSERM); D-4418 from Chiroscience and Schering-Plough; a benzodiazepine PDE4 inhibitor identified as CI-1018 (PD-168787; Parke-Davis/Warner-Lambert); a benzodioxole derivative Kyowa Hakko disclosed in WO 9916766; V-11294A from Napp (Landells, L.J. et al. Eur Resp J [Annu Cong Eur Resp Soc (Sept 19-23, Geneva) 1998] 1998, 12(Suppl. 28): Abst P2393); roflumilast (CAS reference No 162401-32-3) and a pthalazinone (WO 99/47505) from Byk-Gulden (now Altana); or a compound identified as T-440 (Tanabe Seiyaku; Fuji, K. et al. J Pharmacol Exp Ther, 1998, 284(1): 162).

Additional PDE4 inhibitors are disclosed on pages 2 to 15 of WO01/13953. Specifically selected are arofylline, atizoram, BAY-19-8004, benafentrine, BYK-33043, CC-3052, CDP-840, cipamfylline, CP-220629, CP-293121, D-22888, D-4396, denbufylline, filaminast, GW-3600, ibudilast, KF-17625, KS-506-G, laprafylline, NA-0226A, NA-23063A, ORG-20241, ORG-30029, PDB-093, pentoxifylline, piclamilast, rolipram, RPR-117658, RPR-122818, RPR-132294, RPR-132703, RS-17597, RS-25344-000, SB-207499, SB210667, SB211572, SB-211600, SB212066, SB212179, SDZ-ISQ-844, SDZ-MNS-949, SKF-107806, SQ-20006, T-2585, tibenelast, tolafentrine, UCB-29646, V-11294A, Y-58997, YM-976 and zardaverine.

In one embodiment the PDE4 inhibitor is selected from cilomilast, AWD-12-281, NCS-613, D-4418, CI-1018, V-11294A, roflumilast or T-440.

It will be appreciated that the compounds of any of the above combinations or compositions may be administered simultaneously (either in the same or different pharmaceutical formulations), separately or sequentially.

The invention thus provides, in a further aspect, a combination comprising a compound of formula (I) or a pharmaceutically acceptable derivative thereof together with a further therapeutic agent or agents.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations. When a compound of formula (I) or a pharmaceutically acceptable derivative thereof is used in combination with a second therapeutic agent active against the same disease state the dose of each compound may differ from that when the compound is used alone. Appropriate doses will be readily appreciated by those skilled in the art.

**Determination of cannabinoid CB1 Receptor Agonist Activity**

The cannabinoid CB1 receptor agonist activity of the compounds of formula (I) was determined in accordance with the following experimental method.

### Experimental Method

Yeast (*Saccharomyces cerevisiae*) cells expressing the human cannabinoid CB1 receptor were generated by integration of an expression cassette into the *ura3* chromosomal locus of yeast strain MMY23. This cassette consisted of DNA sequence encoding the human CB1 receptor flanked by the yeast GPD promoter to the 5' end of CB1 and a yeast transcriptional terminator sequence to the 3' end of CB1. MMY23 expresses a yeast/mammalian chimeric G-protein alpha subunit in which the C-terminal 5 amino acids of Gpa1 are replaced with the C-terminal 5 amino acids of human G$\alpha$i3 (as described in Brown et al. (2000), Yeast 16:11-22). Cells were grown at 30˚C in liquid Synthetic Complete (SC) yeast media (Guthrie and Fink (1991), Methods in Enzymology, Vol. 194) lacking uracil, tryptophan, adenine and leucine to late logarithmic phase (approximately 6 $OD_{600}$/ml).

Agonists were prepared as 10 mM stocks in DMSO. $EC_{50}$ values (the concentration required to produce 50% maximal response) were estimated using dilutions of between 3- and 5-fold (BiomekFX, Beckman) into DMSO. Agonist solutions in DMSO (1% final assay volume) were transferred into black, clear bottom, microtitre plates from NUNC (96- or 384-well). Cells were suspended at a density of 0.2 $OD_{600}$/ml in SC media lacking histidine, uracil, tryptophan, adenine and leucine and supplemented with 10mM 3-aminotriazole, 0.1M sodium phosphate pH 7.0, and 20$\mu$M fluorescein di-$\beta$-D-glucopyranoside (FDGlu). This mixture (50ul per well for 384-well plates, 200ul per well for 96-well plates) was added to agonist in the assay plates (Multidrop 384, Labsystems). After incubation at 30˚C for 24 hours, fluorescence resulting from degradation of FDGlu to fluorescein due to exoglucanase, an endogenous yeast enzyme produced during agonist-stimulated cell growth, was determined using a Spectrofluor microtitre plate reader (Tecan; excitation wavelength: 485nm; emission wavelength: 535nm). Fluorescence was plotted against compound concentration and iteratively curve fitted using a four parameter fit to generate a concentration effect value. Efficacy ($E_{max}$) was calculated from the equation

$$E_{max} = Max_{[compound\ X]} - Min_{[compound\ X]} / Max_{[HU210]} - Min_{[HU210]}\ x\ 100\%$$

where $Max_{[compound\ X]}$ and $Min_{[ccmpound\ X]}$ are the fitted maximum and minimum respectively from the concentration effect curve for compound X, and $Max_{[HU210]}$ and $Min_{[HU210]}$ are the fitted maximum and minimum respectively from the concentration effect curve for (6aR, 10aR)-3-(1,1'-Dimethylheptyl)-6a,7,10,10a-tetrahydro-1-hydroxy-6,6-dimethyl-6H-dibenzo[b,d]pyran-9-methanol (HU210; available from Tocris). Equieffective molar ratio (EMR) values were calculated from the equation

$$EMR = EC_{50\ [compound\ X]} / EC_{50\ [HU210]}$$

Where $EC_{50\ [compound\ X]}$ is the $EC_{50}$ of compound X and $EC_{50\ [HU210]}$ is the $EC_{50}$ of HU210.

Compounds of the Examples tested according to this method had $EC_{50}$ values >1,000nM and/or an efficacy of <50% at the cloned human cannabinoid CB1 receptor.

### Determination of cannabinoid CB2 Receptor Agonist Activity

The cannabinoid CB2 receptor agonist activity of the compounds of formula (I) was determined in accordance with the following experimental method.

### Experimental Method

Yeast (*Saccharomyces cerevisiae*) cells expressing the human cannabinoid CB2 receptor were generated by integration of an expression cassette into the *ura3* chromosomal locus of yeast strain MMY23. This cassette consisted of DNA sequence encoding the human CB2 receptor flanked by the yeast GPD promoter to the 5' end of CB2 and a yeast transcriptional terminator sequence to the 3' end of CB2. MMY23 expresses a yeast/mammalian chimeric G-protein alpha subunit in which the C-terminal 5 amino acids of Gpa1 are replaced with the C-terminal 5 amino acids of human G$\alpha$3 (as described in Brown et al. (2000), Yeast 16:11-22). Cells were grown at 30˚C in liquid Synthetic Complete (SC) yeast media (Guthrie and Fink (1991), Methods in Enzymology, Vol. 194) lacking uracil, tryptophan, adenine and leucine to late logarithmic phase (approximately 6 $OD_{600}$/ml).

Agonists were prepared as 10 mM stocks in DMSO. $EC_{50}$ values (the concentration required to produce 50% maximal response) were estimated using dilutions of between 3- and 5-fold (BiomekFX, Beckman) into DMSO. Agonist solutions in DMSO (1% final assay volume) were transferred into black, clear bottom, microtitre plates from NUNC (96- or 384-well). Cells were suspended at a density of 0.2 $OD_{600}$/ml in SC media lacking histidine, uracil, tryptophan, adenine

and leucine and supplemented with 10mM 3-aminotriazole, 0.1M sodium phosphate pH 7.0, and 20M fluorescein di-β-D-glucopyranoside (FDGlu). This mixture (50ul per well for 384-well plates, 200ul per well for 96-well plates) was added to agonist in the assay plates (Multidrop 384, Labsystems). After incubation at 30°C for 24 hours, fluorescence resulting from degradation of FDGlu to fluorescein due to exoglucanase, an endogenous yeast enzyme produced during agonist-stimulated cell growth, was determined using a Spectrofluor microtitre plate reader (Tecan; excitation wavelength: 485nm; emission wavelength: 535nm). Fluorescence was plotted against compound concentration and iteratively curve fitted using a four parameter fit to generate a concentration effect value. Efficacy ($E_{max}$) was calculated from the equation

$$E_{max} = Max_{[compound\ X]} - Min_{[compound\ X]} / Max_{[HU210]} - Min_{[HU210]} \times 100\%$$

where $Max_{[compound\ X]}$ and $Min_{[compound\ X]}$ are the fitted maximum and minimum respectively from the concentration effect curve for compound X, and $Max_{[HU210]}$ and $Min_{[HU210]}$ are the fitted maximum and minimum respectively from the concentration effect curve for (6aR,10aR)-3-(1,1'-Dimethylheptyl)-6a,7,10,10a-tetrahydro-1-hydroxy-6,6-dimethyl-6H-dibenzo[b,d]pyran-9-methanol (HU210; available from Tocris). Equieffective molar ratio (EMR) values were calculated from the equation

$$EMR = EC_{50\ [compound\ X]} / EC_{50\ [HU210]}$$

Where $EC_{50\ [compound\ X]}$ is the $EC_{50}$ of compound X and $EC_{50\ [HU210]}$ is the $EC_{50}$ of HU210.

The compounds of Examples 1 to 5, 13-23, 32, 44, 46-52, 55 to 63 tested according to this method had an $EC_{50}$ values of <300nM and efficacy value of >50% at the cloned human cannabinoid CB2 receptor.

The compound of Example 11, 12, 24-31, 33, 34, 37, 54 and 65 tested according to this method had an $EC_{50}$ values between 300nM and 1000nM and efficacy value of >50% at the cloned human cannabinoid CB2 receptor.

The compounds of Examples 6 to 10, 35, 36, 38 to 43, 45, 53 and 64 tested according to this method had an $EC_{50}$ values >1000nM and/or efficacy value <50% at the cloned human cannabinoid CB2 receptor.

The following examples are illustrative, but not limiting of the embodiments of the present invention.

Abbreviations:

AcOH (acetic acid), Bn (benzyl), Bu, Pr, Me, Et (butyl, propyl, methyl ethyl), DMSO (dimethyl sulfoxide), DCM (dichloromethane), DME (1,2-dimethoxyethane), DMF (N,N-dimethylformamide), EDC (1-(3-dimethylaminopropyl)-3-ethylearbodiimide), EtOAc (ethyl acetate), EtOH (ethanol), HPLC (High pressure liquid chromatography), LC/MS (Liquid chromatography/Mass spectroscopy), MDAP (Mass Directed AutoPurification), MeCN (acetonitrile), MeOH (methanol), NMR (Nuclear Magnetic Resonance (spectrum)), NMP (n-methyl pyrrolidone), SPE (Solid Phase Extraction), THF (tetrahydrofuran), s, d, t, q, m, br (singlet, doublet, triplet, quartet, multiplet, broad.)

## Conditions, Hardware, and Software used for Mass-directed Autopurification

### Hardware

Waters 600 gradient pump, Waters 2700 sample manager, Waters Reagent Manager, Micromass ZMD mass spectrometer, Gilson 202 - fraction collector, Gilson Aspec - waste collector.

### Software

Micromass Masslynx version 3.5

### Column

The column used is typically a Supelco ABZ+ column whose dimensions are 10mm internal diameter by 100mm in length. The stationary phase particle size is 5μm.

### Solvents

A. Aqueous solvent = Water + 0.1% Formic Acid
B. Organic solvent = MeCN: Water 95:5 +0.05% Formic Acid

Make up solvent = MeOH: Water 80:20 +50mMol Ammonium Acetate
Needle rinse solvent = MeOH: Water: DMSO 80:10:10

## Methods

Five methods are used depending on the analytical retention time of the compound of interest. They all have a flow rate of 20ml/min and a 15-minute runtime, which comprises of a 10-minute gradient followed by a 5-minute column flush and re-equilibration step.

Method 1 MDP 1.5-2.2 = 0-30%B
Method 2 MDP 2.0-2.8 = 5-30% B
Method 3 MDP 2.5-3.0 = 15-55%B
Method 4 MDP 2.8-4.0 = 30-80% B
Method 5 MDP 3.8-5.5 = 50-90% B

## Conditions used for Analytical LCMS Systems

## Hardware

Agilent 1100 gradient pump
Agilent 1100 Autosampler
Agilent 1100 PDA Dectector
Agilent 1100 Degasser
Micromass ZQ mass spectrometer
PL-ELS 1000

## Software

Micromass Masslynx versions 3.5/4.0

## Column

The column used is a Supelcosil ABZ+PLUS, the dimensions of which are 4.6mm x 33mm. The stationary phase particle size is 3m.

## Solvents

A : Aqueous solvent = 10mMol Ammonium Acetate + 0.1 % Formic Acid
B : Organic solvent = 95 %Acetonitrile + 0.05% Formic Acid

## Method

The generic method used has 5.5 minute runtime, which comprises of a 4.7-minute gradient (0-100% B) followed by a 0.6 minute column flush and 0.2 minute re-equilibration step.

## Flow rate

The above method has a flow rate of 3ml/mins

**Conditions used for NMR**

**Hardware**

Bruker 400MHz Ultrashield
Bruker B-ACS60 Autosampler
Bruker Advance 400 Console

**Software**

User interface - NMR Kiosk
Controlling software - XWin NMR version 3.0

**Conditions used for the Biotage Horizon.**

Column: Biotage C18HS 25+S
Fraction volume: 9ml UV Threshold : 0.03AU
Solvent A= Water , B= Acetonitrile
Gradient:

| Volume(ml) | A | B |
|---|---|---|
| 0 | 70% | 30% |
| 240 | 0% | 100% |

**Intermediate 1: 6-Chloro-4-cyclopropyl-nicotinic acid**

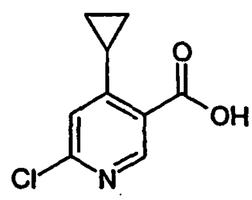

6-Chloronicotinic acid (15.8g, 100mmol) was dissolved in dry THF (100ml) and cooled to 0˚C. A 0.5M solution of cyclopropylmagnesium bromide in THF (500ml, 250mmol) was added dropwise over 75mins and the mixture was stirred at 0˚C for 1hr then at room temperature overnight. The solution was cooled to -60˚C and glacial acetic acid (25ml) was added over 15mins, followed by manganese(III) acetate dihydrate (53.6g, 200mmol). The mixture was stirred at -60˚C for 30mins then at room temperature for 3hr. The mixture was filtered and the filtrate was evaporated. The resultant gum was taken up in DCM (500ml) and water (500ml), separated and the aqueous was extracted with DCM (250ml). The combined organic layers were washed with water (250ml), then extracted with a mixture of water (150ml) and 2M sodium hydroxide (70ml). The extract was washed with DCM (50ml), then acidified with conc hydrochloric acid. The resultant solid was filtered off, washed with water and dried to give the title compound as a brown solid (5.24g). LC/MS , t =1.97 min, Molecular ion observed (MH+) = 198 consistent with the molecular formula $C_9H_8{}^{35}ClNO_2$

**Intermediate 2: 6-(3-Chloro-phenylamino)-4-cyclopropyl-nicotinic acid**

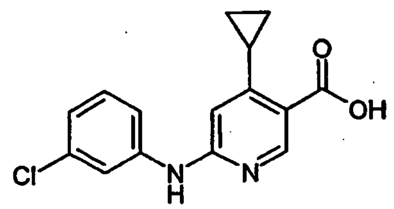

A mixture of 6-chloro-4-cyclopropyl-nicotinic acid (5.22g, 26.4mmol) and 3-chloroaniline (25ml) was heated on an oil bath at 150°C for 2hrs. The reaction mixture was cooled and taken up in a mixture of water (40ml), 2M sodium hydroxide (30ml) and ether (100ml). The layers were separated and the aqueous was washed with ether (2 x 100ml). The aqueous was acidified with concentrated hydrochloric acid, the solid was filtered off, washed with water and dried to give the title compound as a brown solid (6.38g)
LC/MS t = 3.44min, Molecular ion observed (MH+) = 289 consistent with the molecular formula $C_{15}H_{13}{}^{35}ClN_2O_2$

**Intermediate 3: [6-(3-Chloro-phenylamino)-4-cyclopropyl-pyridine-3-yl]-methanol**

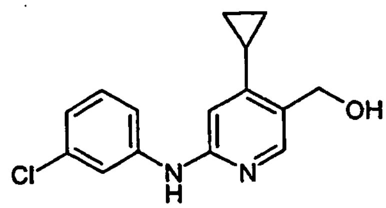

Isobutylchloroformate (2.5ml, 19.3mmol) and N-methylmorpholine (2.2ml, 19.3mmol) were successively added to a stirred solution of 6-(3-chloro-phenylamino)-4-cyclopropyl-nicotinic acid (5.56g, 19.3mmol) in 1,2 dimethoxyethane (125ml) at -20°C and stirring was continued for 30 minutes at -20°C. The precipitate was filtered off. discarded and a solution of $NaBH_4$ (1.46g, 38.5mmol) in water (10ml) was added over 5mins at 0°C. The reaction mixture was stirred for 30 minutes, quenched with water and extracted with ethyl acetate. The combined organic extracts were washed with brine, dried ($MgSO_4$), and evaporated. The resultant gum was purified by chromatography on silica gel (dichloromethane/ ether 1:1) to give the title compound as a cream solid (3.44g)
LC/MS, t = 2.57min, Molecular ion observed (MH+) = 275 consistent with the molecular formula $C_{15}H_{15}{}^{35}ClN_2O$

**Intermediate 4: 6-(3-Chloro-phenylamino)-4-cyclopropyl-pyndine-3-carbaldehyde**

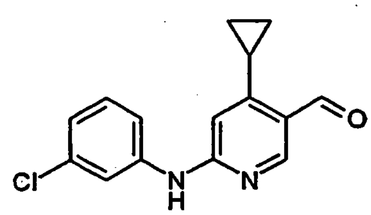

Manganese(IV)oxide, and sodium chloride were added to a stirred solution of [6-(3-chloro-phenylamino)-4-cyclopropyl-pyridine-3-yl]-methanol (3.44g) in dichloromethane (40ml). After stirring at 21°C for 18 hours, the precipitate was filtered off, washed with dichloromethane, and dried in vacuo to yield the title compound as a yellow solid (3.31g)
LC/MS, t = 3.65min, Molecular ion observed (MH+) = 273 consistent with the molecular formula $C_{15}H_{13}{}^{35}ClN_2O$

**Intermediate 5: 6-(3-Chloro-phenylamino)-N-cyclobutylmethyl-4-cyclopropyl-nicotinamide.**

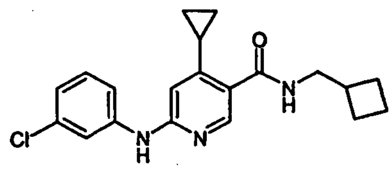

In a manner similar to Intermediate 10, 6-chloro-N-cyclobutyimethyl-4-cyclopropyl-nicotinamide (80mg, 0.3mmol) and 3-chloroaniline (77mg, 0.6mmol) were reacted and the crude product purified by chromatography on the Biotage Horizon to give the title compound (59mg).
LC/MS, t = 3.46 min, Molecular ion observed (MH+) = 356 consistent with the molecular formula $C_{20}H_{22}{}^{35}ClN_3O$

**Intermediate 6: 6-Chloro-N-cyclobutylmethyl-4-isopropyl-nicotinamide**

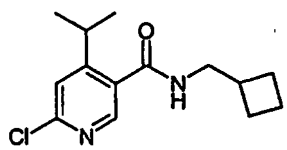

To a solution of 6-chloro-*N*-cyclobutylmethyl-nicotinamide (2.00 g) in dry tetrahydrofuran (5 ml) was added dropwise at 0° under nitrogen a 2.0M solution of isopropylmagnesium chloride in THF (13.5ml) and the solution stirred at room temperature for 15 hours. It was cooled to 0° and dry methanol added dropwise and the solution stirred for 15 minutes. 2,3-Dichloro-5,6-dicyano-1,4-benzoquinone was added and the mixture stirred at room temperature for 30 minutes then evaporated under reduced pressure. The residual liquid was warmed to 50° and t-butyl methyl ether added. The mixture was stirred under reflux for 1 hour then at room temperature for 1 hour and filtered. The filtrate was evaporated and the residue purified using Biotage chromatography (Merck 9385 silica gel), eluting with 1:4 ethyl acetate:isohexane to afford the title compound (1.31g). $[MH^+]$ 267 consistent for $C_{14}H_{19}ClN_2O$

**Intermediate 7: N-Cyclobutylmethyl-4-isopropyl-6-m-tolylamino-nicotinamide**

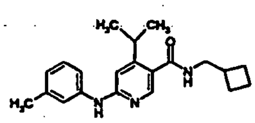

A mixture of 6-chloro-N-cyclobutylmethyl-4-isopropyl-nicotinamide (80mg) and 3-methylphenylamine was irradiated under microwave conditions at 180°C for 1 hour. The crude product was diluted with dichloromethane (2ml) and the solution applied to a Sep-Pack column of silica gel. This was eluted firstly with dichloromethane, followed by dichloromethane/ether 5:1 to give the title compound (48mg).
LC/MS t = 3.39min, $[MH^+]$ 338 consistent with the molecular formula $C_{21}H_{27}N_3O$

**Intermediate 8: 6-Chloro-N-(tetrahydro-pyran-4-ylmethyl)-nicotinamide**

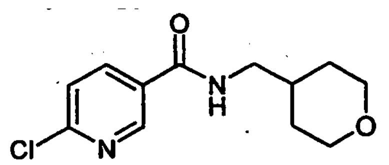

Triethylamine (3.4ml) was added to a solution of 6-chloronicotinoyl chloride (1.9 g, ex Lancaster) and C-(tetrahydro-pyran-4-yl)-methylamine (1.65 g) in dry dichloromethane (30 ml) dropwise at 0° under nitrogen over 15 min. The solution was stirred at 0° for 1 hour. Dichloromethane was removed under reduced pressure and ethyl acetate (50ml) added. The solution was washed with water (3 x 25 ml), dried ($MgSO_4$) and evaporated. The residue was purified using Biotage chromatography over silica gel (Merck 9385) eluting with EtOAc, 3:1, to give the title compound (1.46g).
NMR (DMSO-d6) δ 1.1-1.25 (2H, m), 1.60 (2H, d), 1.79 (1H, m), 3.17 (2H, t), 3.26 (2H, t), 3.83 (2H, d of d), 7.64 (1H, d), 8.23 (1H, d of d), 8.75 (1H, t), 8.82 (1H, s).

**Intermediate 9 : 6-Chloro-4-isopropyl-N-(tetrahydro-pyran-4-ylmethyl)-nicotinamide**

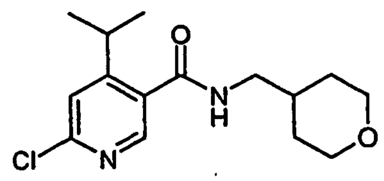

In a manner similar to Intermediate 6, 6-chloro-N-(tetrahydro-pyran-4-ylmethyl)-nicotinamide (1.46 g) and 2.0M isopropylmagnesium chloride in tetrahydrofuran (8.5 ml) afforded the title compound (624 mg).
NMR (DMSO-d6) δ 1.1-1.25 (2H, m), 1.19 (6H, d), 1.60 (2H, d), 1.75 (1H, m), 3.14 (2H, t), 3.21 (1H, m), 3.27 (2H, t), 3.85 (2H, d of d), 7.54 (1H, d), 8.26 (1H, s), 8.63 (1H, t).
LC/MS t = 2.4 min, [$MH^+$] 297 consistent with the molecular formula $C_{15}H_{21}{}^{35}ClN_2O_2$

**Intermediate 10 : 6-(3-Chloro-4-trifluoromethoxy-phenylanmino)-4-isopropyl-N-(tetrahydropyran-4-ylmethyl)-nicotinamide**

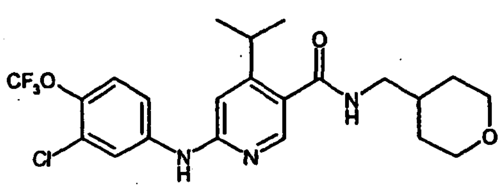

A mixture of 6-chloro-4-isopropyl-N-(tetrahydropyran-4-ylmethyl)-nicotinamide (100 mg), 3-chloro-4-trifluoromethoxyaniline (ex Lancaster, 143 mg), methanesulfonic acid (44 μl) in 1,4-dioxane (0.8 ml) was irradiated under microwave conditions at 180° for 30 minutes. Purification of the crude mixture by MDAP detailed at the beginning of the experimental section afforded the title compound as a white solid (109 mg).
NMR (DMSO-d6) δ 1.16-1.22 (8H, d,m), 1.60-1.63 (2H, d), 1.75 (1H, m), 3.12 (2H, t), 3.26 (2H, t), 3.41 (1H, m), 3.85 (2H, d), 6.80 (1H, s), 7.45 (1H, d), 7.58 (1H, dd), 8.15 (1H, s), 8.25 (1H, d), 8.36 (1H, t), 9.58 (1H, s).
LC/MS t = 3.63 min, [$MH^+$] 472, consistent with molecular formula $C_{22}H_{25}{}^{35}Cl\ F_3N_3O_3$

**Intermediate 11: 6-Chloro-*N*-cyclohexylmethyl-nicotinamide**

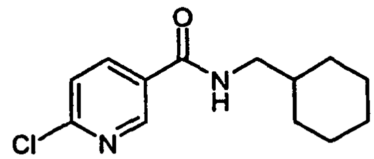

To a solution of 6-chloronicotinoyl chloride (1.5 g, ex Lancaster) in dry dichloromethane (15 ml) was added dropwise at 0˚ under nitrogen a solution of cyclohexanemethanamine (1.11 ml, ex Lancaster) and triethylamine (1.5 ml) in dry dichloromethane (15 ml) over 1 hour. The solution was stirred at 0˚ for 1 hour. Dichloromethane was removed under reduced pressure and ethyl acetate (30 ml) added. The solution was washed with water (3 x 20 ml), dried ($MgSO_4$) and evaporated to afford 6-chloro-*N*-cyclohexylmethyl-nicotinamide (1.96g).
NMR (DMSO-d6) δ 0.85-1.0 (2H, m), 1.1-1.25 (3H, m), 1.54 (1H, m), 1.55-1.75 (5H, m), 3.11 (2H, t), 7.64 (1H, d), 8.23 (1H, d of d), 8.69 (1H, t), 8.82 (1H, s).
LC/MS t = 2.9 min, Molecular ion observed [$MH^+$] 253 consistent with molecular formula $C_{13}H_{17}\,^{35}ClN_2O$

**Intermediate 12: 6-Chloro-*N*-cyclohexylmethyl-4-isopropyl-nicotinamide**

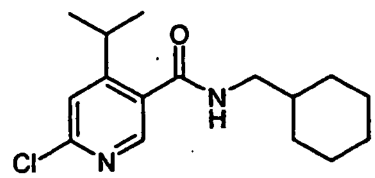

To a solution of 6-chloro-*N*-cyclohexylmethyl-nicotinamide (0.89 g) in dry tetrahydrofuran (5 ml) was added dropwise at 0˚ under nitrogen a 2.0M solution of isopropylmagnesium chloride (5.3 ml, ex Aldrich) and the solution stirred at room temperature for 15 hours. It was cooled to 0˚ and dry methanol (0.86 ml) added dropwise and the solution stirred for 15 minutes. 2,3-Dichloro-5,6-dicyano-1,4-benzoquinone (0.88 g) was added and the mixture stirred at room temperature for 30 minutes then evaporated under reduced pressure to ca. 6 ml. The residual liquid was warmed to 50˚ and t-butyl methyl ether (20 ml) added. The mixture was stirred under reflux for 1 hour then at room temperature for 1 hour and filtered. The filtrate was evaporated and the residue purified using Biotage chromatography (Merck 9385 silica gel) with 1:4 ethyl acetate:isohexane to afford 6-chloro-*N*-cyclohexylmethyl-4-isopropyl-nicotinamide (886 mg).
NMR (DMSO-d6) δ 0.85-1.0 (2H, m), 1.1-1.25 (3H, m), 1.19 (6H, d), 1.50 (1H, m), 1.55-1.75 (5H, m), 3.08 (2H, t), 3.22 (1H, m), 7.53 (1H, s), 8.24 (1H, s), 8.57 (1H, t).
LC/MS, t = 3.2 min, Molecular ion observed [$MH^+$] = 295 consistent with the molecular formula $C_{16}H_{23}\,^{35}ClN_2O$.

**Intermediate 13: 6-Chloro-N-cyclopentylmethyl-nicotinamide**

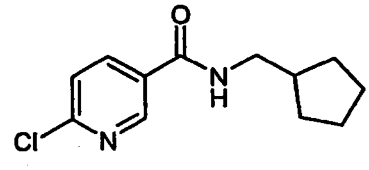

In a manner similar to Intermediate 11, 6-chloronicotinoyl chloride (0.50 g) and cyclopentanemethylamine hydrochloride (385 mg) afforded the title compound (534 mg).

NMR (DMSO-d6) δ 1.2-1.3 (2H, m), 1.45-1.65 (4H, m), 1.65-1.75 (2H, m), 2.13 (1H, m), 3.20 (2H, t), 7.64 (1H, d), 8.23 (1H, d of d), 8.74 (1H, t), 8.82 (1H, s).
LC/MS t = 2.7 min, [MH+] 239, consistent with the molecular formula $C_{12}H_{15}{}^{35}ClN_2O$

**Intermediate 14: 6-(3-Chloro-phenylamino)-4-(1-hydroxy-methyl-ethyl)-N-(tetrahydropyran-4-ylmethyl)-nicotinamide**

**a) 6-Chloro-1,1,dimethyl-1H-furo[3,4-c]pyridin-3-one**

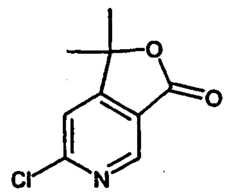

To a solution of 2,2,6,6,-tetramethylpiperidine (ex Aldrich, 13.44g) in tetrahydrofuran (90ml) at - 55°C under nitrogen was added dropwise 1.6M butyl lithium in hexane (ex Aldrich, 80ml). After 30 minutes a solution of 6-chloronicotinic acid (ex Aldrich, 5g) in tetrahydrofuran (40ml) was added dropwise and the solution stirred at -71°C for 2 hours. The solution was treated with acetone (23ml) and then allowed to warm to room temperature. The solvent was removed under reduced pressure and the residue dissolved in water (100ml) and acidified to pH 3 with concentrated hydrochloric acid. The precipitated white solid was filtered off washed with water and dried to afford the title compound (4.42g).
NMR (DMSO-d6) δ 1.65 (6H, s), 8.11 (1H, s), 8.91 (1H, s)
LC/MS t = 2.0 min, [NH+] 198, consistent with molecular formula $C_9H_8{}^{35}ClNO_2$

**b) 6-(2,4-Dichloro-phenylamino)-1,1-dimethyl-1H-furo[3,4-c]pyridin-3-one**

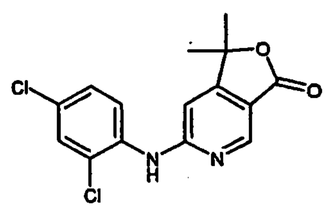

A mixture of 6-chloro-1,1-dimethyl-1H-furo[3,4-c]pyridin-3-one (500mg), 2,4-dichloroaniline (ex Lancaster, 822mg), methanesulfonic acid (329μl) in 1,4-dioxane (3ml) was irradiated under microwave conditions at 180° for 30 minutes. The solid was dissolved in ethyl acetate (50ml) and washed with 5% sodium bicarbonate and water. The organic layer was dried over anhydrous magnesium sulphate, filtered and evaporated under reduced pressure. Purified by trituration with isohexane followed by trituration with ether to afford the title compound as a pale brown solid (360mg).
NMR (DMSO-d6) δ 1.58 (6H, s), 6.93 (1H, s), 7.44 (1H, dd), 7.69 (1H, d), 7.77 (1H, d), 8.55 (1H, s), 9.40 (1H, s).
LC/MS t = 3.3 min, [MH+] 323, consistent with molecular formula $C_{15}H_{12}{}^{35}Cl_2N_2O_2$

**c) 6-(2,4-Dichloro-phenylamino)-4-(1-hydroxy-methyl-ethyl)-N-(tetrahydropyran-4-ylmethyl)-nicotinamide**

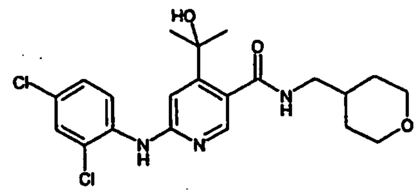

To a solution of 4-aminomethyltetrahydropyran (ex Combi-Blocks, Inc, 267mg) in dry dichloromethane (10ml) under nitrogen, was added dropwise 2.0M trimethylaluminium in hexane (ex Aldrich, 1.16ml) and the solution stirred for 15 minutes. Then 6-(2,4-dichloro-phenylamino)-1,1,dimethyl-1H-furo[3,4-c]pyridin-3-one (150mg) was added and the mixture stirred at 40˚C overnight. A further portion of 4-aminomethyltetrahydropyran (107mg) and 2.0M trimethylaluminium in hexane (466μl) in dry dichloromethane (5ml) was added and the mixture stirred for 24h. The solvent was evaporated under reduced pressure and the residue partitioned between ethyl acetate (20ml) and water (3 x 10ml) and the aqueous layer separated. The organic layer was dried over anhydrous magnesium sulphate, filtered and evaporated under reduced pressure. Purified using the Biotage Horizon system detailed at the beginning of the experimental section to afford the title compound as a white solid (118mg).
NMR (DMSO-d6) δ 1.17-1.23 (2H, m), 1.46 (6H, s), 1.60-1.63 (2H, d), 1.79 (1H, m), 3.11 (2H, t), 3.28 (2H, t), 3.85 (2H, d), 6.07 (1H, s), 7.23 (1H, s), 7.36 (1H, dd), 7.61 (1H, d), 8.06 (2H, t), 8.68 (1H, t), 8.78 (1H, s).
LC/MS t = 3.1 min, [MH$^+$] 437, consistent with molecular formula $C_{21}H_{22}{}^{35}Cl_2N_3O_3$

**Intermediate 15: 6-Chloro-N-cyclopentylmethyl-4-isopropyl-nicotinamide**

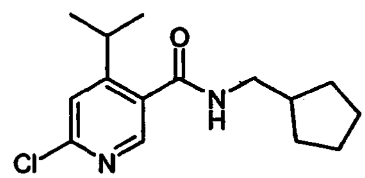

In a manner similar to Intermediate 12, 6-chloro-N-(cyclopentylmethyl)-nicotinamide (532 mg) and 2.0M isopropylmagnesium chloride in tetrahydrofuran (3.4 ml) afforded the title compound (166 mg).
NMR (DMSO-d6) δ 1.19 (6H, d), 1.2-1.3 (2H, m), 1.45-1.65 (4H, m), 1.65-1.75 (2H, m), 2.10 (1H, m), 3.17 (2H, t), 3.21 (1H, m), 7.53 (1H, s), 8.23 (1H, s), 8.61 (1H, t).
LC/MS t = 3.1 min, [MH$^+$] 281, consistent with the molecular formula $C_{15}H_{21}{}^{35}ClN_2O$.

**Intermediate 16: 6-Chloro-N-(tetrahydro-pyran-4-ylmethyl)-nicotinamide**

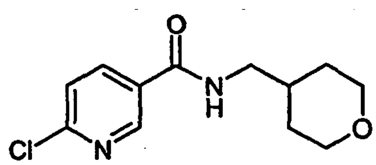

A suspension containing (tetrahydro-pyran-4-yl)-methylamine (13g) in dry dichloromethane (100ml) with triethylamine (35ml) was added dropwise at 0˚C under nitrogen over 1 hour to a stirred solution of 6-chloronicotinoyl chloride (15g, ex Lancaster) in dry dichloromethane (150ml). The solution was stirred at 0˚C for 1 hour, allowed to warm to room temperature and then stirred at room temperature for 1 hour. Dichloromethane was removed under reduced pressure and ethyl acetate (500ml) added. The solution was washed with water (3x100ml), dried ($MgSO_4$), and evaporated to afford 6-chloro-N-(tetrahydro-pyran-4-ylmethyl)-nicotinamide (18.7g)
NMR ($CDCl_3$) δ 1.27-1.38 (2H, m), 1.57-1.64 (2H, m), 1.75-1.90 (1H, m), 3.25-3.37 (4H, m), 3.92 (2H, dd), 6.30 (1H,

bs), 7.35 (1H, d), 8.01 (1H, d), 8.66 (1H, d)
LC/MS, t = 1.75 min, Molecular ion observed [MH+] = 255 consistent with the molecular formula $C_{12}H_{15}{}^{35}ClN_2O_2$

**Intermediate 17: 6-Chloro-4-cyclopentyl-N-(tetrahydro-pyran-4-ylmethyl)-nicotinamide**

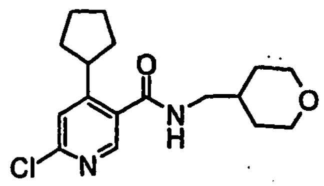

To a solution of 6-chloro-N-(tetrahydro-pyran-4-ylmethyl)-nicotinamide (7 g) in dry tetrahydrofuran (50 ml) was added dropwise at 0°C under nitrogen, cyclopentylmagnesium chloride (2M solution in diethyl ether, 42 ml, ex Aldrich) and the solution stirred at room temperature for 15 hours. It was cooled to 0°C and dry methanol (20 ml) added dropwise and the solution stirred for 15 minutes. 2,3-Dichloro-5,6-dicyano-1,4-benzoquinone (6.9 g) was added and the mixture stirred at room temperature for 1 hour then evaporated under reduced pressure to ca. 20 ml. The residual semi-solid was treated with ethyl acetate (3x100ml) and warmed to 50°C. The solids were filtered off and the filtrate was evaporated and the residue purified using Biotage Horizon (gradient 10% to 50% ethyl acetate and isohexane) to afford 6-chloro-4-cyclopentyl-N-(tetrahydro-pyran-4-ylmethyl)-nicotinamide (5.2g).
NMR ($CDCl_3$) δ 1.24-139 (2H, m), 1.42-1.53 (2H, m), 1.55-1.69 (4H, m), 1.70-1.89 (3H, m), 1.99-2.08 (2H, m), 3.25-3.38 (5H, m), 3.93 (2H, dd), 5.96-6.04 (1H, m), 7.21 (1H, s), 8.20 (1H, s).
LC/MS, t = 2.74 min, Molecular ion observed [MH+] = 323 consistent with the molecular formula $C_{17}H_{23}{}^{35}ClN_2O_2$

**Intermediate 18: C-cyclobutyl-methylamine hydrochloride**

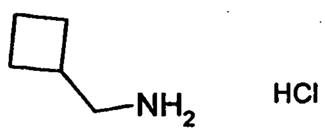

A solution of borane-tetrahydrofuran complex (1M in tetrahydrofuran, 120ml) was added over 10 minutes to a solution of cyclobutanecarbonitrile (8.1g) [Lancaster] in dry tetrahydrofuran (20ml) under nitrogen at room temperature. The solution was refluxed overnight, then cooled to 20°. Methanol (150ml) was added dropwise over 15 minutes keeping the temperature below 25°. The mixture was cooled to 0° and dry hydrogen chloride was bubbled through for 30 minutes. The resulting mixture was refluxed for 1.5 hours, evaporated and the residue re-evaporated twice from methanol. Ether (150ml) was added and the resulting solid was filtered off. The solid was taken up in hot isopropanol (50ml), filtered and hot acetonitrile (30ml) added. On cooling, the solid was filtered off to give the title compound (5.7g)
NMR (DMSO-d6) 81.8 (4H, m), 2.0 (2H, m), 2.54 (1H, m), 2.80 (2H, d), 8.0 (3H, s)

**Intermediate 19: 6-Chloro-N-cyclobutylmethyl-nicotinamide**

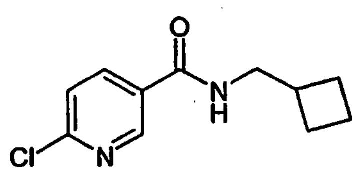

A stirred mixture of 6-chloronicotinoyl chloride (1.9g, ex-Lancaster) and C-cyclobutyl-methylamine hydrochloride (1.52g) in dry dichloromethane (30 ml) was cooled to 0˚ C and then triethylamine (3.4 ml) was added dropwise over 5 min at 0˚C. The mixture was stirred at 0˚C for 15 min, then at ambient temperature for 90 min. The solution was washed with water (30 ml), then with water acidified to pH 5 with aqueous 2N hydrochloric acid, and then with water. The dried ($MgSO_4$) organic layer was evaporated to give the title compound (2.02g).
NMR (DMSO-d6) δ 1.71 (2H, m), 1.82 (2H, m), 1.99 (2H, m), 2.52 (1H, m excess), 3.31 (2H, t), 7.64 (1H, d), 8.22 (1H, d of d), 8.71 (1H, t), 8.81 (1H, d).
LC/MS t = 2.51 min, Molecular ion observed [$MH^+$] = 225 consistent with the molecular formula $C_{11}H_{13}{}^{35}ClN_2O$

**Intermediate 20: 6-Chloro-4-cyclopropyl-N-(tetrahydro-pyran-4-ylmethyl)-nicotinamide**

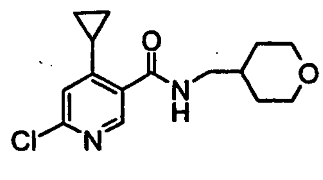

To a solution of 6-chloro-N-(tetrahydro-pyran-4-ylmethyl)-nicotinamide (3.5 g) in dry tetrahydrofuran (25 ml) was added dropwise at 0˚C under nitrogen a 0.5M, in tetrahydrofuran, a solution of cyclopropylmagnesium chloride (82 ml, ex Aldrich) and the solution stirred at room temperature for 15 hours. It was cooled to 0˚C and dry methanol (10 ml) added dropwise and the solution stirred for 15 minutes. 2,3-Dichloro-5,6-dicyano-1,4-benzoquinone (3.1 g) was added and the mixture stirred at room temperature for 1 hour then evaporated under reduced pressure to ca. 6 ml. The residual semi-solid was warmed to 50˚C with ethyl acetate (3x100ml). The solids were filtered off and the filtrate was evaporated. The residue was purified using Biotage Horizon (gradient 10% to 50% ethyl acetate and isohexane) to afford 6-chloro-4-cyclopropyl-N-(tetrahydropyran-4-ylmethyl)-nicotinamide (1.2g).
NMR ($CDCl_3$) δ 0.82-0.95 (2H, m), 1.16-1.28 (2H, m), 1.35-1.48 (2H, m), 1.69 (2H, dd), 1.85-1.98 (1H, m), 2.28-2.38 (1H, m), 3.35-3.47 (4H, m), 4.03 (2H, dd), 6.19 (1H, bs), 6.79 (1H, s), 8.34 (1H, s)
LC/MS, t = 2.20 min, Molecular ion observed [$MH^+$] = 295 consistent with the molecular formula $C_{15}H_{19}{}^{35}ClN_2O_2$

**Intermediate 21: 6-Chloro-N-cyclobutylmethyl-4-cyclopropyl-nicotinamide**

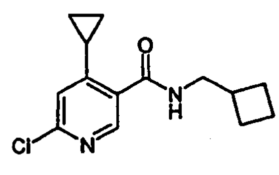

In a manner similar to Intermediate 20, 6-chloro-N-cyclobutylmethyl-nicotinamide (9g) gave the title compound (4.4g)
NMR (DMSO-d6) δ 0.91 (2H, m), 1.08 (2H, m), 1.75 (2H, m), 1.82 (2H, m), 2.00 (2H, m), 2.17 (1H, m), 2.52 (1H, m excess), 3.28 (2H, t), 6.99 (1H, s), 8.20 (1H, s), 8.59 (1H, t).
LC/MS t = 2.61 min, Molecular ion observed ($MH^+$) = 265 consistent with the molecular formula $C_{14}H_{17}{}^{35}ClN_2O$

**Intermediate 22: 6-Chloro-N-cyclopentylmethyl-nicotinamide**

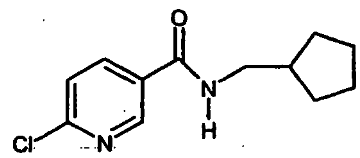

This was prepared in the same manner as the compound in Intermediate 19 from C-cyclopentylmethylamine hydrochloride (prepared as in J Med Chem 1997, 40, 3207) (10g) to give the title compound 12.2g
NMR (MeOD) δ 1.26 -1.35 (2H, m), 1.51-1.72 (4H, m), 1.73- 1.86 (2H, m), 2.16-2.28 (1H,m), 3.29-3.36 (2H, m), 7.55 (1H, d), 8.18 (1H, dd), 8.77 (1H, dd).
LC / MS t= 2.64 min, molecular ion observed [MH+] = 239 consistent with molecular formula $C_{12}H_{15}ClN_2O$

**Intermediate 23: 6-Chloro-N-cyclopentylmethyl-4-cyclopropyl-nicotinamide**

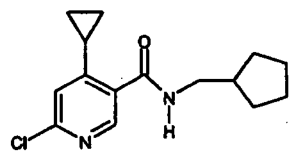

This was prepared from 6-chloro-N-cyclopentylmethyl-nicotinamide (12.1 g) in a manner similar that described in Intermediate 20 to give chloro-N-cyclopentylmethyl-4-cyclopropyl-nicotinamide (9.55g).
NMR (MeOD) δ 0.86-0.92 (2H, m), 1.12-1.19 (2H, m), 1.27-1.35 (2H, m), 1.53-1.73 (4H, m), 1.77-1.86 (2H, m), 2.15-2.26 (2H, m), 3.28-3.36 (2H, m), 4.87 (1H, s), 6.99(1H, s), 8.20 (1H, s) LC / MS t= 2.81 min, molecular ion observed [MH+] = 279 consistent with molecular formula
$C_{15}H_{19}ClN_2O$

**Intemediate 24: 3-Amino-3-cyclopropyl-acrylic acid methyl ester**

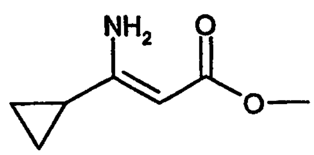

To a stirred solution of 3-cyclopropyl-3-oxo-propionic acid methyl ester (10g, ex Butt Park,) in methanol (200ml) was added ammonium acetate (26g) and the mixture was stirred at room temperature for 18 hours overnight. The methanol was evaporated under reduced pressure, and the residue treated with dichloromethane (100ml). The suspension was stirred for 30 minutes at room temperature. The solid formed was filtered, and washed with dichloromethane. The dichloromethane was evaporated under reduced pressure to afford the title product (10g) as a clear oil, which solidified on standing.
NMR NMR ($CDCl_3$) δ 0.60 - 0.85 (4H,m), 1.29 - 1.39 (1H, m), 3.55 (3H, s), 4.40 (1H, s), 8.28 - 8.85 (bs partially exchanged NH)

**Intermediate 25: 4-(1-Amino-1-cyclopropyl-methylene)-pent-2-enedioic acid dimethyl ester**

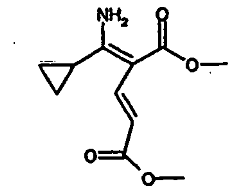

To 3-amino-3-cyclopropyl-acrylic acid methyl ester (8.8g) in toluene (100ml) was added propynoic acid methyl ester (6ml). The mixture was heated to 85°C for 47 hours, and when cool, was evaporated under reduced pressure. The residue was taken up into toluene (30ml) and subjected to microwave irradiation at 110°C for 30 min. The toluene was removed under reduced pressure and the residue was chromatographed using a Biotage (ethyl acetate 40% / isohexane 60%) to afford the title compound (10.6g).
NMR ($CDCl_3$) δ 0.70 - 0.77 (2H, m), 0.98 -1.06 (2H, m), 1.93 - 2.03 (1H, m), 3.56 (3H, s), 3.71 (3H, s), 6.13 (1H, d), 8.00 (1H, d)

**Intermediate 26: 2-Cyclopropyl-6-oxo-1,6-dihydro-pyridine-3-carboxylic acid methyl ester**

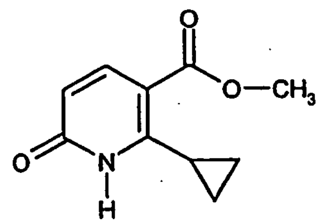

To a solution of 4-(1-amino-1-cyclopropyl-methylene)-pent-2-enedioic acid dimethyl ester (1.5g) in dimethylformamide (10ml) was added sodium tert-butoxide (100mg) and the mixture was refluxed for 6.5 hours. The mixture was purified by Biotage chromatography over silica gel, using ethyl acetate (70%) / isohexane (30%) to afford the title compound (1.1g) as an off white solid NMR (DMSO) δ 0.97 - 1.16 (4H, m), 2.99 - 3.10 (1H, m), 3.78 (3H, s), 6.14 - 6.26 (1H, m), 7.79 - 7.88 (1H, m), 11.0 (1H, s).

**Intermediate 27: 6-Chloro-2-cyclopropyl-nicotinic acid methyl ester**

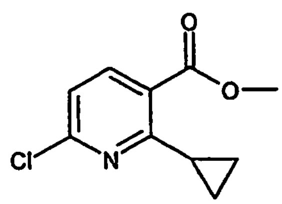

To 2-cyclopropyl-6-oxo-1,6-dihydro-pyridine-3-carboxylic acid methyl ester (1.1g) was added phenyl dichlorophosphate (10ml). The suspension was heated to 180°C and stirred at 180°C for 10 minutes. The dark mixture was allowed to cool to room temperature, and an excess of ice was added. After 15 minutes a saturated solution of sodium hydrogen carbonate (80ml) was added carefully. The mixture was extracted with ethyl acetate (2x 50ml), and the combined, dried ($Na_2SO_4$) organic extracts were evaporated under reduced pressure to give a pale yellow oil. This purified by Biotage chromatography over silica gel with ethyl acetate (60%) / isohexane (40%), to afford the title compound (1.23g) as a white solid.
NMR ($CDCl_3$) δ 1.04 - 1.12 (2H, m), 1.19 - 1.25 (2H, m), 3.04 - 3.12 (1H, m), 3.94 (3H, s), 7.10 (1H, d), 8.07 (1H, s).

**Intermediate 28: 6-Chloro-2-cyclopropyl-nicotinic acid**

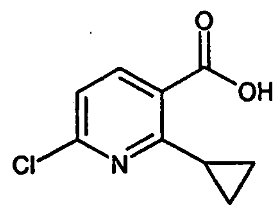

To 6-chloro-2-cyclopropyl-nicotinic acid methyl ester (1.23g) was added tetrahydrofuran (9ml) and water (3ml) followed by lithium hydroxide (0.72g). The mixture was stirred vigorously at room temperature overnight, and then evaporated under reduced pressure. To the residue was added water (50ml), which was then acidified to pH1 using conc. hydrochloric acid. The white precipitate that formed was filtered and washed with water (50ml) and dried to afford the title compound (1g) NMR (DMSO) δ 0.95 - 1.09 (4H, m), 3.03 - 3.12 (1H, m), 7.31 (1H, d), 8.12 (1H, s), 13.50 (1H, s). LC / MS t= 2.58 min, [MH$^+$] = 198 consistent with molecular formula $C_9H_8ClNO_2$

**Intermediate 29: 6-Chloro-2-cyclopropyl-N-(tetrahydro-pyran-4-ylmethyl)-nicotinamide**

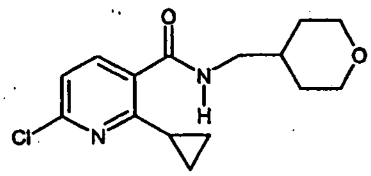

To a solution of 6-chloro-2-cyclopropyl-nicotinic acid (2.1 g) in dimethylformamide (20ml) was added 1-hydroxybenzotriazole (730mg), 1-[3-(dimethylamino)propyl]-3-ethylcarbodiimide hydrochloride (2.31g), N-ethyl morpholine(3.2ml) followed by (tetrahydro-pyran-4-yl)-methylamine (1.9g). The mixture was stirred at room temperature overnight. Water (100ml) was added and the mixture was extracted with ethyl acetate (2x 100ml). The combined organic layers were washed with 10% sodium hydrogen carbonate (100ml), and brine (50ml). The dried ($Na_2SO_4$) organic layer was evaporated under reduced pressure. The residue was purified by Biotage chromatography over silica using ethyl acetate(60%) / isohexane (40%) to give the title compound (2.81g) as a white solid.
NMR (MeOD) δ 0.96 - 1.10 (4H, m), 1.28 - 1.41 (2H, m), 1.65 - 1.73 (2H, m), 1.80 - 1.94 (1H, m), 2.24 - 2.33 (1H, m); 3.24 - 3.29 (2H, m), 3.37 - 3.47 (2H, m), 3.92 - 4.00 (2H, m), 7.16 (1H, d), 7.62 (1H, d).
LC / MS t= 2.39 min, molecular ion observed [MH$^+$] = 295 consistent with molecular formula $C_{15}H_{19}ClN_2O_2$

**Intermediate 30: 6-(2,4-Dichloro-phenylamino)-4-trifluoromethyl-nicotinic acid methyl ester**

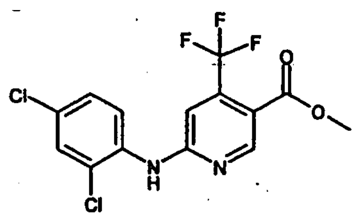

A mixture of methyl-6-chloro-4-(trifluoromethyl)-nicotinate (2.0 g, 8.37 mmol ex Fluorochem) and 2,4-dichloroaniline (4.05 g, 25 mmol) was heated at 130°C for 15 h, to afford the title compound that was used for the next step

without further purification.
MS m/z (ESI+): 365 (MH+).

**Intermediate 31: 6-(2,4-Dichloro-phenylamino)-4-trifluoromethyl-nicotinic acid**

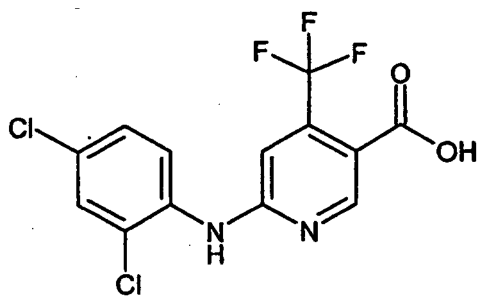

A solution of potassium hydroxide (1.4 g, 25 mmol) in 20 mL of EtOH / $H_2O$ (1:1) was added to the crude mixture from Intermediate 30 and the resulting mixture was stirred under reflux for 3h. The solution was concentrated in vacuo, diluted with water and washed three times (3 x 15 mL) with diethyl ether. Upon acidification of the aqueous layer to pH1 with 37% HCl, the title compound precipitated out as hydrochloride salt that was filtered and dried under vacuum. The solid (2.89 g, 7.5 mmol) was then suspended in dichloromethane (20 mL), in the presence of PS-diisopropylethylamine (1.93 g, 7.5 mmol, loading 3.88 mmol/g, ex Argonaut Technologies) and stirred at room temperature for 30 min. After filtration of the resin and evaporation in vacuo of the solvent, the title compound was isolated as a white solid (2.62 g).
$^1$H NMR (300 MHz, DMSO-$d_6$) δ: 13.16 (s br, 1H); 9.49 (s, 1H); 8.67 (s, 1H); 7.94 (d, 1H); 7.67 (d, 1H); 7.43 (dd, 1H); 7.40 (s, 1H).
MS m/z (ESI+): 351 (MH+).

**Intermediate 32: [6-(2,4-Dichloro-phenylamino)-4-trifluoromethyl-pyridin-3-yl]-methanol**

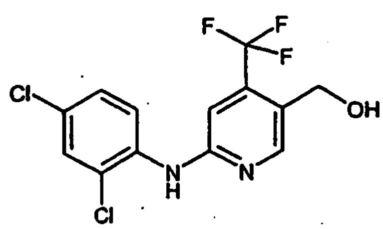

To a solution of 6-(2,4-dichloro-phenylamino)-4-trifluoromethyl-nicotinic acid (250mg) in DME (4mL), N-methylmorpholine (144mg) and isobutyl chloroformate (94μl) were added and the reaction mixture was stirred under nitrogen for 30min. The precipitate was filtered off, discarded, and sodium borohydride (55mg) was added to the filtrate at -15° and the reaction mixture was stirred at this temperature for 30 min. The solvent was removed in vacuo, and a solution of the residue in ethyl acetate was washed with aqueous 1M sodium hydroxide, followed by aqueous 5% sodium bicarbonate, water, and the dried ($MgSO_4$) organic layer was concentrated to dryness to give the product as a yellow oil (200mg).
MS m/z (ESI+): 338 (MH+).

**Intermediate 33: 6-(2,4-Dichloro-phenylamino)-4-trifluoromethyl-pyridine-3-carbaldehyde**

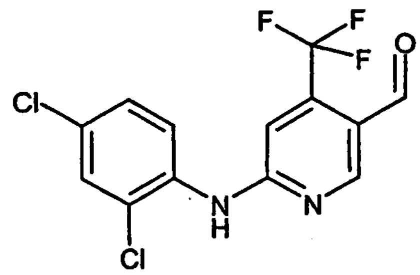

Manganese (IV) oxide (610mg) and sodium chloride (180mg) were added to a solution of [6-(2,4-dichloro-phenylamino)-4-trifluoromethyl-pyridin-3-yl]-methanol (200mg) in dichloromethane (5ml) and the suspension was stirred at room temperature overnight. The solid was filtered off and the solvent removed under reduced pressure to afford the title compound (190mg).
MS m/z (ESI+): 336 (MH+)

**Intermediate 34: 6-Chloro-N-cyclopropylmethyl-nicotinamide**

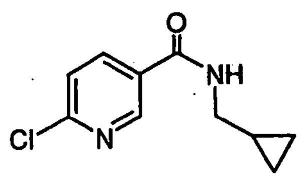

Prepared in a manner similar to Intermediate 11, using C-cyclopropyl-methylamine in place of cyclohexanemethanamine to give the title compound.
LC/MS t= 2.02 min. Molecular ion observed [$MH^+$] 211 consistent with molecular formula $C_{10}H_{11}ClN_2O$.

**Intermediate 35: 6-Chloro-N-cyclopropylmethyl-4-isopropyl-nicotinamide**

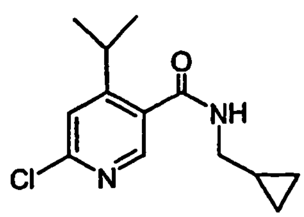

Prepared in a manner similar to intermediate 6, from 6-Chloro-N-cyclopropylmethyl-nicotinamide using an extraction with dichloromethane followed by chromatography instead of warming with t-butyl methyl ether, to give the title compound.
LC/MS t= 2.60 min. Molecular ion observed [$MH^+$] 253 consistent with molecular formula $C_{13}H_{17}ClN_2O$.

**Intermediate 36: 6-Chloro-N -isobutyl-nicotinamide**

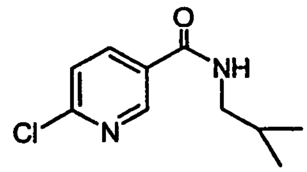

Prepared in a manner similar to intermediate 11, using isobutylamine in place of cyclohexanemethanamine to give the title compound.
LC/MS t= 2.51 min. Molecular ion observed [$MH^+$] 213 consistent with molecular formula $C_{10}H_{13}ClN_2O$.

**Intermediate 37: 6-Chloro-N -isobutyl-4-isopropyl-nicotinamide**

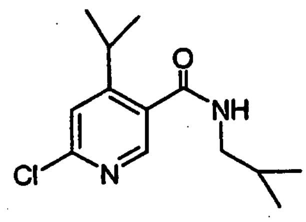

Prepared in a manner similar to intermediate 6, from 6-chloro-N -isobutyl-nicotinamide using an extraction with ethyl acetate followed by chromatography instead of warming with t-butyl methyl ether, to give the title compound.
LC/MS t= 2.75 min. Molecular ion observed [$MH^+$] 255 consistent with molecular formula $C_{13}H_{19}ClN_2O$.

**Intermediate 38: 6-Chloro-4-isopropyl-nicotinic acid.**

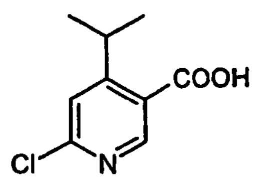

Method A

2M Isopropylmagnesium bromide in tetrahydrofuran (48 ml) was added dropwise over 1 hour to a solution of 6-chloronicotinic acid (6.0 g) in dry tetrahydrofuran (100 ml) at 0° under nitrogen and the solution stirred at 0° for 3 hours then at room temperature for 15 hours. It was cooled to -60° and acetic acid (48 ml), tetrahydrofuran (40 ml) and manganese (III) acetate dihydrate (20.4 g) added successively. The mixture was stirred at -70° for 30 minutes then at room temperature for 1 hour. The suspension was filtered through Celite and the filtrate evaporated under reduced pressure. The residue was partitioned between dichloromethane (150 ml) and water (120 ml) and the aqueous layer separated and washed with dichloromethane (2 x 50 ml). The combined organic layers were dried ($MgSO_4$) and evaporated under reduced pressure to afford, after silica gel chromatography using 3:1 isohexane:ethyl acetate, 6-chloro-4-isopropyl-nicotinic acid (2.31g).
NMR (DMSO-d6) $\delta$ 1.21 (6H, d), 3.76 (1H, m), 7.60 (1H, s), 8.67 (1H, s), 13.55 (1H, br s).
LC/MS t = 2.6 min, [$MH^+$] 200 consistent with molecular formula $C_9H_{10}{}^{35}ClN0_2$

Method B

To a stirred solution of 6-chloronicotinic acid (12.6g) in anhydrous tetrahydrofuran (300ml), under nitrogen, at 0˚C was added isopropylmagnesium chloride (60ml of a 2M solution in THF) over 10 minutes. The mixture was then allowed to warm to room temperature. Isopropylmagnesium chloride (50ml of 2M solution in THF) was added as 5ml aliquots at 30 minutes intervals. The mixture was cooled to 0˚C and methanol (20ml) was added slowly over 10 minutes. The mixture was stirred for 5 minutes and 2,3-dichloro-5,6-dicyano-1,4-benzoquinone (20g) was added portionwise over 10 minutes. The mixture was allowed to warm to room temperature and stirred for 2 hours. The mixture was evaporated and the residue in dichloromethane (750ml), was washed with water(300ml) containing aqueous 1M hydrochloric acid (120ml). The organic phase was allowed to stand overnight. The precipitate which formed was filtered off and discarded. The filtrate was evaporated and the resultant residue purified over silica gel using a Flash 75 biotage system eluting with dichlormethane /containing methanol (1.25%) and acetic acid (0.5%), to give 6-chloro-4-isopropyl-nicotinic acid (11.8g, 74%)
Molecular ion observed m/z= 200 [MH+] consistent with formula $C_9\ H_{10}\ {}^{35}ClN\ O_2$

**Intermediate 39: 6-(3-Chloro-phenylamino)-4-isopropyl-nicotinic acid.**

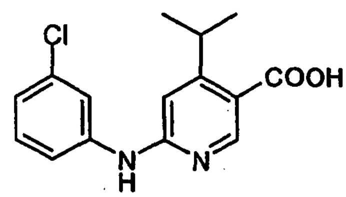

Method A.

A mixture of 6-chloro-4-isopropyl-nicotinic acid (0.50 g) and 3-chloroaniline (265 mg) was stirred at 120˚ for 1½ hours. Isopropanol was added and the mixture chilled. Insoluble solid was filtered off, washed successively with isopropanol and ether and dried *in vacuo* at 50˚ to afford 6-(3-chloro-phenylamino)-4-isopropyl-nicotinic acid (0.51 g). NMR (DMSO-d6) δ 1.19 (6H, d), 3.93 (1H, m), 6.85 (1H, s), 6.99 (1H, d), 7.31 (1H, t), 7.53 (1H, d), 8.00 (1H, s), 8.64 (1H, s), 9.73 (1H, s), 12.6 (1H, br s).
LC/MS t = 3.63 min, [MH$^+$] 291, consistent with molecular formula $C_{15}H_{15}{}^{35}ClN_2O_2$

Method B.

6-Chloro-4-isopropyl-nicotinic acid (2g) was added 3-chloroaniline (5ml), under nitrogen, and mixture was heated to 150˚C for 2 hours. Water (20ml) was added followed by 2M sodium hydroxide (200ml). The mixture was extracted with ether (4x100ml) and this was discarded. The aqueous phase was acidified to pH1 using aqueous 2M hydrochloric acid. The precipitate formed was filtered off, washed with water (50ml) and dried to give the title compound (2.7g, 88%)
NMR (MeOD) δ 1.23 (3H, s), 1.25 (3H, s), 3.97-4.04(1H, m), 6.80(1H, s), 6.95-6.98 (1H, m), 7.24 (1H, s), 7.43-7.45(1H, m), 7.82-7.83(1H, m), 8.71(1H, s). (NB No acid proton orNH seen)

**Intermediate 40: 6-(3-Chloro-phenylamino)-N-cyclopropyl-4-isopropyl-nicotinamide.**

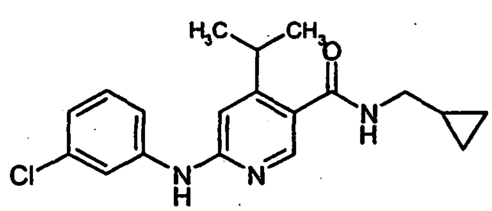

To a solution of 6-(3-chloro-phenylamino)-4-isopropyl-nicotinic acid) (48 mg) in dimethylformamide (2.5 ml) was added successively N-ethylmorpholine (69 μl), cyclopropanemethylamine (17 μl), 1-hydroxybenzotriazole hydrate (40 mg) and 1-(3-dimethylamino-propyl)-3-ethylcarbodiimide hydrochloride (40 mg). The solution was stirred for 3 hours and allowed to stand overnight. Dimethylformamide was removed under reduced pressure and ethyl acetate (8 ml) added. The solution was washed sequentially with 5% sodium bicarbonate solution (5 ml), water (5 ml) and brine (2 x 5 ml), dried ($MgSO_4$) and evaporated to afford the title compound (43 mg).
LC/MS t = 3.47 min, [$MH^+$] 344, consistent with molecular formula $C_{19}H_{22}{}^{35}ClN_30$

**Intermediate 41 : 6-(3-Chloro-phenylamino)-N-cyclobutylmethyl-4-isopropyl-nicotinamide**

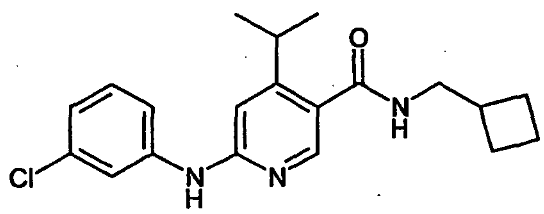

A mixture of 6-chloro-N-cyclobutylmethyl-4-isopropyl-nicotinamide (80mg) and 3-chloroaniline (0.5r was irradiated under microwave conditions at 180°C for 30mins. The mixture was diluted with dichloromethane (2ml) and chromatographed on silica gel. The excess aniline was removed by elution with dichloromethane and then elution with dichloromethane/ether (5:1) gave the title compound (38mg NMR (DMSO-$d_6$) δ 1.16 (6H, m), 1.74 (2H, m), 1.82 (2H, m), 2.00 (2H, m), 2.52 (1H, m excess), 3.23 (2H, t), 3.40 (1H, m), 6.78 (1H, s), 6.92 (1H, d), 7.27 (1H, t), 7.46 (1H, d), 8.04 (1H, s), 8.10 (1H, s), 8.33 (1H, t), 9.41 (1H, s)
LC/MS t = 3.65min, [$MH^+$] 358 consistent with the molecular formula $C_{20}H_{24}{}^{35}ClN_3O$

Intermediates Table

Intermediates 42 to 76 were prepared from the above described appropriate Intermediates and commercially available amines by the methods set out below

Method A: As for Intermediate 41: 6-(3-Chloro-phenylamino)-N-cyclobutylmethyl-4-isopropyl-nicotinamide

Method G: As for Method A, but with 2 equivalents of methanesulphonic acid present in the reaction mixture prior to microwave irradiation.

Purification Method B: MDAP as detailed at the beginning of the experimental

Purification Method F: Chromatography on silica gel as detailed in the preparation of Intermediate 41 (6-(3-Chloro-phenylamino)-N-cyclobutylmethyl-4-isopropyl-nicotinamide)

Purification Method H: Biotage Horizon as detailed at the beginning of the experimental

| Intermediate no. | Chemical Name | Structure | 1. Preparation method A, or G<br>2. Time. | Purification method<br>B, F, or H | 1.Retention Time (min).<br>2.[MH+]<br>3. Molecular formula |
|---|---|---|---|---|---|
| 42 | 6-(3-Bromo-phenylamino)-N-cyclobutylmethyl-4-isopropyl-nicotinamide | 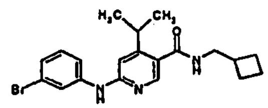 | A<br>30 min | F | 3.70<br>402<br>$C_{20}H_{24}{}^{79}BrN_3O$ |
| 43 | N-Cyclobutylinethyl-6-(2,4-dichloro-phenylamino)-4-isopropyl-nicotinamide | 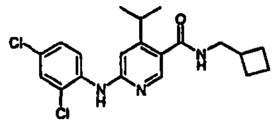 | G<br>1hour | F | 3.75<br>392<br>$C_{20}H_{23}{}^{35}Cl_2N_3O$ |
| 44 | N-Cyclobutylinethyl-6-(3,4-dichloro-phenylamino)-4-isopropyl-nicotinamide | 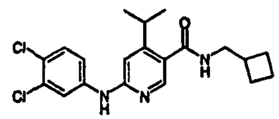 | G<br>1hour | Crude product purified by trituration with 1:1 DCM/Ether | 3.89<br>392<br>$C_{20}H_{23}{}^{35}Cl_2N_3O$ |
| 45 | 6-(3-Chloro-4-fluoro-phenylamino)-N-cyclobutylinethyl-4-isopropyl-nicotinamide | 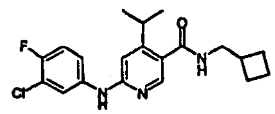 | G<br>1hour | H | 3.63<br>376<br>$C_{20}H_{23}{}^{35}ClFN_3O$ |
| 46 | N-cyclobutylmethyl-6-(2-fluoro-3-trifluoromethyl-phenylamino)-4-isopropyl-nicotinamide | 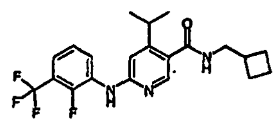 | G<br>1hour | H | 3.64<br>410<br>$C_{21}H_{23}F_4N_3O$ |
| 47 | N-Cyclobutylinethyl-6-(2,5-dichloro-phenylamino)-4-isopropyl-nicotinamide | 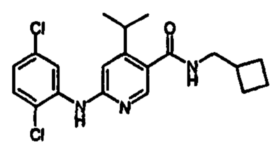 | G<br>1hour | H | 3.78<br>392<br>$C_{20}H_{23}{}^{35}Cl_2N_3O$ |
| 48 | N-Cyclobutylinethyl-6-(3,5-difluoro-phenylamino)-4-isopropyl-nicotinamide | 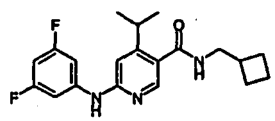 | G<br>1hour | H | 3.57<br>360<br>$C_{20}H_{23}F_2N_3O$ |

(continued)

| Intermediate no. | Chemical Name | Structure | 1. Preparation method A, or G 2. Time. | Purification method B, F, or H | 1.Retention Time (min). 2.[MH+] 3. Molecular formula |
|---|---|---|---|---|---|
| 49 | 6-(2,4-Dichloro-phenylamino)-N-isobutyl-4-isopropyl-nicotinamide | 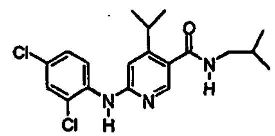 | G 30 min | H | 3.70 380 $C_{19}H_{23}{}^{35}Cl_2N_3O$ |
| 50 | 6-(2-Chloro-4-fluoro-phenylamino)-N-cyclobutylmethyl-4-isopropyl-nicotinamide | 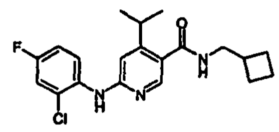 | G 1hour | F & triturate with $Et_2O$ . | 3.37 376 $C_{20}H_{23}{}^{35}ClFN_3O$ |
| 51 | 6-(4-Chloro-2-fluoro-phenylamino)-N-cyclobutylinethyl-4-isopropyl-nicotinamide | 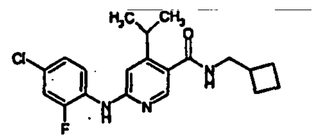 | G 30 min | H | 3.58 376 $C_{20}H_{23}{}^{35}ClFN_3O$ |
| 52 | 6-(2-Bromo-4-trifluoromethoxy-phenylamino)-N-cyclobutylmethyl-4-isopropyl-nicotinamide | 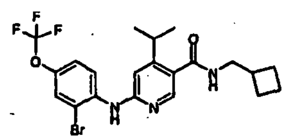 | G 1hour | H | 3.84 486 $C_{21}H_{23}{}^{79}BrF_3N_3O_2$ |
| 53 | 6-(4-Chloro-2-methyl-phenylamino)-N-cyclobutylmethyl-4-isopropyl-nicotinamide | 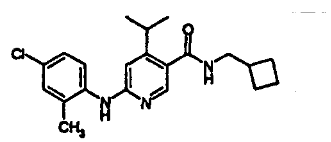 | G 1hour | H | 3.35 372 $C_{21}H_{26}{}^{35}ClN_3O$ |
| 54 | 6-(3-Chloro-4-fluoro-phenylamino)-N-cyclobutylmethyl-4-cyclopropyl-nicotinamide | 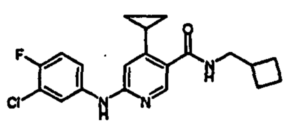 | G 30 min | H | 3.7 374 $C_{20}H_{21}{}^{35}ClFN_3O$ |

(continued)

| Intermediate no. | Chemical Name | Structure | 1. Preparation method A, or G 2. Time. | Purification method B, F, or H | 1.Retention Time (min). 2.[MH+] 3. Molecular formula |
|---|---|---|---|---|---|
| 55 | 6-(3-Chloro-2-methyl-phenylamino)-N-cyclobutylmethyl-4-cyclopropyl-nicotinamide | 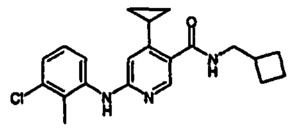 | G<br>30 min | H | 3.4<br>370<br>$C_{21}H_{24}{}^{35}ClN_3O$ |
| 56 | N-Cyclopropylmethyl-4-isopropyl-6-(3-trifluoromethyl-phenylamino)-nicotinamide | 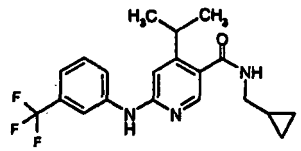 | G<br>30 min | B | 3.54<br>378<br>$C_{20}H_{22}F_3N_3O$ |
| 57 | N-Cyclopropylmethyl-6-(3,5-difluoro-phenylamino)-4-isopropyl-nicotinamide | 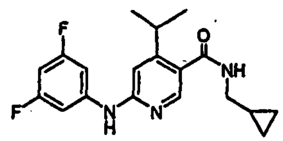 | G<br>30 min | B | 3.43<br>346<br>$C_{19}H_{21}F_2N_3O$ |
| 58 | N-Cyclopropylmethyl-6-(2-fluoro-4-trifluoromethyl-phenylamino)-4-isopropyl-nicotinamide | 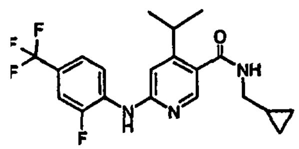 | G<br>30 min | B | 3.51<br>396<br>$C_{20}H_{21}F_4N_3O$ |
| 58 | 6-(3-Chloro-4-methyl-phenylamino)-N-cyclopropylinethyl-4-isopropyl-nicotinamide | 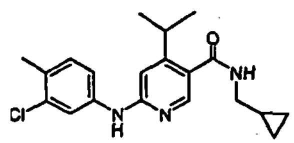 | G<br>30 min | B | 3.59<br>358<br>$C_{20}H_{24}ClN_3O$ |

(continued)

| Intermediate no. | Chemical Name | Structure | 1. Preparation method A, or G 2. Time. | Purification method B, F, or H | 1.Retention Time (min). 2.[MH+] 3. Molecular formula |
|---|---|---|---|---|---|
| 60 | 6-(3-Chloro-4-fluoro-phenylamino)-N-cyclopropylmethyl-4-isopropyl-nicotinamide | 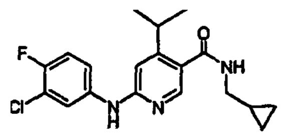 | G 30 min | B | 3.48 362 $C_{19}H_{21}ClFN_3O$ |
| 61 | N-Cyclobutylmethyl-6-(2,4-difluoro-phenylamino)-4-isopropyl-nicotinamide | 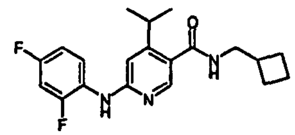 | G 1hour | F | 3.24 360 $C_{20}H_{23}F_2N_3O$ |
| 62 | 6-(4-Chloro-2-methyl-phenylamino)-N-cyclobutylmethyl-4-cyclopropyl-nicotinamide | 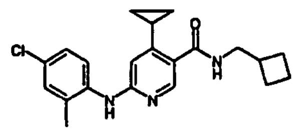 | G 30 min | B | 3.5 370 $C_{21}H_{24}{}^{35}ClN_3O$ |
| 63 | N-Cyclopropylmethyl-6-(3,4-difluoro-phenylamino)-4-isopropyl-nicotinamide | 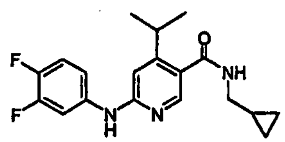 | G 30 min | B | 3.31 346 $C_{19}H_{21}F_2N_3O$ |
| 64 | 6-(3-Bromo-phenylamino)-2-cyclopropyl-N-(tetrahydro-pyran-4-ylmethyl)-nicotinamide | 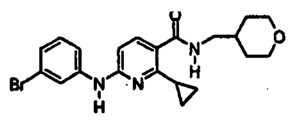 | G 30 min | B | 3.32 432 $C_{21}H_{24}{}^{81}BrN_3O_2$ |
| 65 | 2-Cyclopropyl-6-(3,5-difluoro-phenylamino)-N-(tetrahydro-pyran-4-ylmethyl)-nicotinamide | 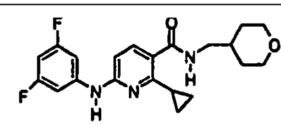 | G 30 min | B | 3.21 388 $C_{21}H_{23}F_2N_3O_2$ |

(continued)

| Intermediate no. | Chemical Name | Structure | 1. Preparation method A, or G<br>2. Time. | Purification method<br>B, F, or H | 1.Retention Time (min).<br>2.[MH+]<br>3. Molecular formula |
|---|---|---|---|---|---|
| 66 | 2-Cyclopropyl-6-(3,4-dichloro-phenylamino)-N-(tetrahydro-pyran-4-ylmethyl)-nicotinamide | 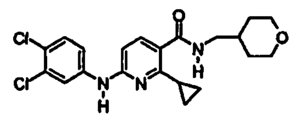 | G<br>30 min | B | 3.56<br>420<br>$C_{21}H_{23}Cl_2N_3O_2$ |
| 67 | 6-(3-Chloro-phenylamino)-2-cyclopropyl-N-(tetrahydro-pyran-4-ylmethyl)-nicotinamide | 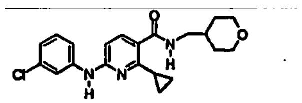 | G<br>30 min | B | 3.24<br>386<br>$C_{21}H_{24}Cl\ N_3O_2$ |
| 68 | 4-Cyclopentyl-6-(2,4-dichloro-phenylamino)-N-(tetrahydro-pyran-4-ylmethyl)-nicotinamide | 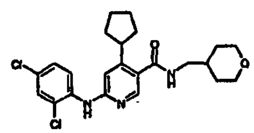 | G<br>30 min | B | 3.67<br>448<br>$C_{23}H_{27}{}^{35}Cl_2N_3O_2$ |
| 69 | 6-(3-Chloro-4-fluoro-phenylamino)-N-cyclopentylmethyl-4-cyclopropyl-nicotinamide | 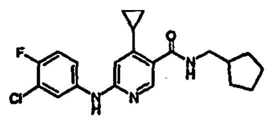 | G<br>30 min | H | 3.74<br>388<br>$C_{21}H_{23}{}^{35}ClFN_3O$ |
| 70 | N-Cyclopentylmethyl-4-cyclopropyl-6-(4-fluoro-3-trifluoromethyl-phenylamino)-nicotinamide | 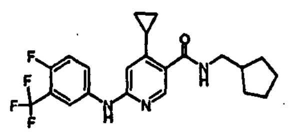 | G<br>30 min | H | 3.80<br>422<br>$C_{22}H_{23}F_4N_3O$ |
| 71 | 6-(2-Fluoro-3-trifluoromethyl-phenylamino)-N-isobutyl-4-isopropyl-nicotinamide | 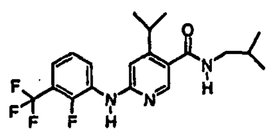 | G<br>30 min | H | 3.37<br>398<br>$C_{20}H_{23}F_4N_3O$ |

(continued)

| Intermediate no. | Chemical Name | Structure | 1. Preparation method A, or G 2. Time. | Purification method B, F, or H | 1.Retention Time (min). 2.[MH+] 3. Molecular formula |
|---|---|---|---|---|---|
| 72 | 6-(3,4-Dichloro-phenylamino)-N-isobutyl-4-isopropyl-nicotinamide | 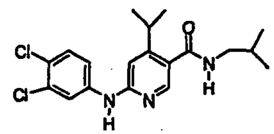 | G 30 min | H | 3.72 380 $C_{19}H_{23}{}^{35}Cl_2N_3O$ |
| 73 | 6-(3-Bromo-phenylamino)-N-isobutyl-4-isopropyl-nicotinamide | 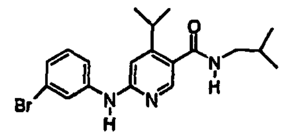 | G 30 min | H | 3.63 392 $C_{19}H_{24}{}^{81}BrN_3O$ |
| 74 | 6-[(3-Bromo-phenyl)-methyl-amino]-4-isopropyl-N-(tetrahydro-pyran-4-ylmethyl)-nicotinamide | 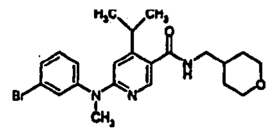 | G 2hrs | F | 3.31 446 $C_{22}H_{28}{}^{79}BrN_3O_2$ |
| 75 | 6-[(3-Chloro-phenyl) amino]-N-(cyclopentylmethyl)-4-isopropyl-nicotinamide | 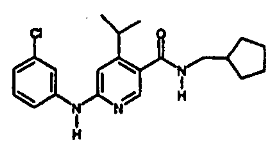 | G 30 min | B | 3.76 372 $C_{21}H_{26}N_3ClO$ |
| 76 | N-Isobutyl-4-isopropyl-6-(3-trifluoromethyl-phenylamino)-nicotinamide | 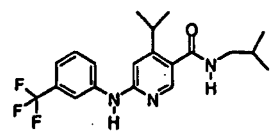 | G 30 min | H | 3.53 380 $C_{20}H_{24}F_3N_3O$ |

**Intermediate 77: (6-Chloro-4-isopropyl-pyridin-3-yl)-methanol**

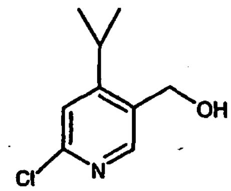

To a cooled to -20°C solution of 6-chloro-4-isopropyl-nicotinic acid (9.38g) in 1,2-dimethoxyethane (150ml), under nitrogen, was added isobutyl chloroformate (6.3ml) dropwise followed by N-methyl morpholine (5.3ml) dropwise. The mixture was stirred at -20°C for 30 minutes. The mixture was quickly filtered, the solid obtained was washed with 1,2-dimethoxyethane (20ml). The combined filtrates were allowed to warm to between +5°C to 7°C and stirred for 30 minutes. To this was added a solution of sodium borohydride (3.56g) in water (30ml) dropwise, maintaining the temperature below +10°C. After 10 minutes the mixture was poured into water (150ml). The aqueous layer was extracted with ethyl acetate (2x150ml) and the combined extracts were dried ($MgSO_4$) and evaporated to give an oil. This was purified using a flash biotage system over silica gel, eluting with DCM/ Ether (5:1) to give an oil which solidified on standing, to give the title compound (5.96g, 68%)
Molecular ion observed m/z= 186 [MH+] consistent with formula $C_9$ $H_{12}$ $^{35}$ClNO

**Intermediate 78: 6-Chloro-4-isopropyl-pyridine-3-carbaldehyde**

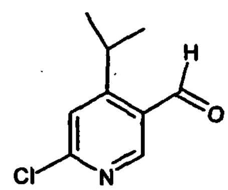

To a solution of (6-chloro-4-isopropyl-pyridin-3-yl)-methanol (5.9g) in dichloromethane (300ml), under nitrogen, was added sodium chloride (28g) and magnesium (IV) oxide (28g) and the mixture stirred at room temperature for 48 hours. The mixture was filtered through a pad of Kieselguhr. The filtrate was evaporated in vacuo and the residue was purified over silica gel using biotage system eluting with dichloromethane to the title compound as a light brown oil (4.83g, 82%)
Molecular ion observed m/z= 184 [MH+] consistent with formula $C_9$ $H_{10}$ $^{35}$Cl N O

**Intermediate 79: 1-(6-Chloro-4-isopropyl-pyridin-3-yl)-ethanol**

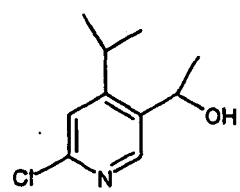

To a cooled (-60°C to -70°C) solution of 6-chloro-4-isopropyl-pyridine-3-carbaldehyde (1.5g) in tetrahydrofuran (20ml), under nitrogen, was added methylmagnesium bromide (3M in ether, 7.2ml) dropwise. After 1 hour the cold mixture was quenched by slow addition of saturated ammonium chloride (50ml) and allowed to warm to room temperature.

The mixture was extracted with dichloromethane (2x50ml), the dichloromethane extracts were combined, dried ($MgSO_4$) and evaporated. The residue was purified over silica gel using a biotage system to give the title compound (1.42g , 86%). Molecular ion observed m/z= 200 [MH+] consistent with formula $C_{10} H_{14} {}^{35}Cl N O$

**Intermediate 80: 1-(6-Chloro-4-isopropyl-pyridin-3-yl)-ethanone**

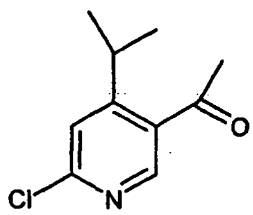

To a solution of 1-(6-chloro-4-isopropyl-pyridin-3-yl)-ethanol (1.42g) in dichloromethane (30ml), under nitrogen, was added sodium chloride (4.7g) and manganese (IV) oxide (4.7g) and the mixture was stirred overnight at room temperature followed by heating at reflux 3 hours. The mixture was filtered through a pad of Kieselguhr, and the filtrate was evaporated in vacuo to give the title compound (1.1g, 78%)
Molecular ion observed m/z= 198 [MH+] consistent with formula $C_{10} H_{12} {}^{35}Cl N O$

**Intermediate 81: [1-(6-Chloro-4-isopropyl-pyridin-3-yl)-ethyl]-cyclobutylmethyl-amine**

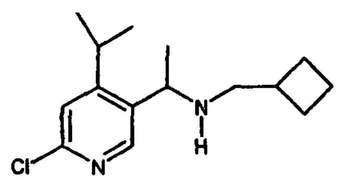

To a stirred solution of 1-(6-chloro-4-isopropyl-pyridin-3-yl)-ethanone (100mg) in a reacti-vial was added tetraethyl orthosilicate (1ml) followed by concentrated sulfuric acid (20μl). The reaction was exothermic and a white precipitate formed. The mixture was sealed and heated to 150°C for 5 hours. To the mixture was added sodium triacetoxyborohydride (150mg) portionwise. The mixture was stirred until no further effervescence was observed. The mixture was sealed and heated to 100°C for 1hour. It was then evaporated in vacuo and the residue was purified by MDAP to give the title compound (70mg, 52%).
Molecular ion observed m/z= 267 [MH+] consistent with formula $C_{15} H_{23} {}^{35}ClN_2$

**Intermediate 82: [6-(3-Chloro-phenylamino)-4-isopropyl-pyridin-3-yl]-methanol**

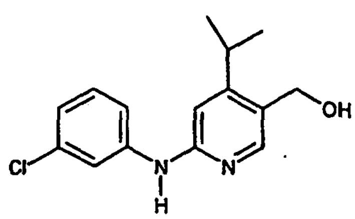

The tittle compound (2.5g, 97%) was prepared in a similar manner to that described for Intermediate 3 using 6-(3-chloro-phenylamino)-4-isopropyl-nicotinic acid (2.7g). Molecular ion observed m/z= 277 [MH+] consistent with formula ($C_{15} H_{17} {}^{35}Cl N_2 O$)

**Intermediate 83: 6-(3-Chloro-phenylamino)-4-isopropyl-pyridine-3-carbaldehyde**

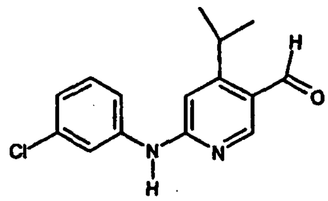

6-(3-Chloro-phenylamino)-4-isopropyl-pyridine-3-carbaldehyde was prepared in a similar manner to that described for Intermediate 78, using [6-(3-chloro-phenylamino)-4-isopropyl-pyridin-3-yl]-methanol (2.5g) to give the title compound (1.54g, 62%).
Molecular ion observed m/z= 275 [MH+] consistent with formula $C_{15}$ $H_{15}$ $^{35}Cl$ $N_2$ O

**Intermediate 84: 6-(2,4-Dichloro-phenylamino)-4-isopropyl-nicotinic acid**

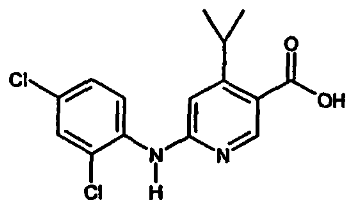

The title compound (1.5g, 46%) was prepared in a similar manner to Intermediate 39, using 6-chloro-4-isopropyl-nicotinic acid (2g) and 2,4-dichloroaniline (excess).
Molecular ion observed m/z= 325 [MH+] consistent with formula $C_{15}$ $H_{14}$ $^{31}Cl_2$ $N_2$ $O_2$

**Intermediate 85: [6-(2,4-Dichloro-phenylamino)-4-isopropyl-pyridin-3-yl]-methanol**

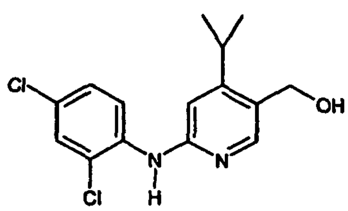

The title compound (1.5g, 72%) was prepared in a similar manner to Intermediate 3 from 6-(2,4-dichloro-phenylamino)-4-isopropyl-nicotinic acid (2.18g)
Molecular ion observed m/z= 311 [MH+] consistent with formula $C_{15}H_{16}{}^{35}Cl_2N_2O$

**Intermediate 86: 6-(2,4-Dichloro-phenylamino)-4-isopropyl-pyridine-3-carbaldehyde**

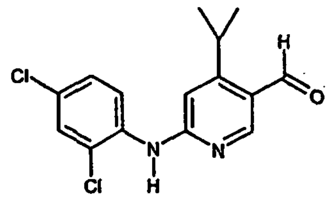

The title compound (1g, 72%) was prepared in a similar manner to Intermediate 78, using [6-(2,4-dichloro-phenylamino)-4-isopropyl-pyridin-3-yl]-methanol (1.4g).
Molecular ion observed m/r= 309 [MH+] consistent with formula $C_{15}H_{14}{}^{35}Cl_2\ N_2O$

**Intermediate 87: tert-Butyl-(6-chloro-4-isopropyl-pyridin-3-ylmethyl)-amine**

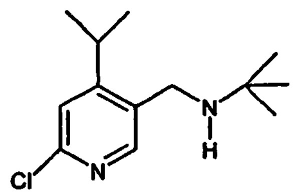

Prepared in a similar manner to Example 60 using 6-chloro-4-isopropyl-pyridine-3-carbaldehyde (1g) and tert-butylamine (478mg) to give the title compound (410mg, 31%)
Molecular ion observed m/z= 241 [$MH^+$] consistent with formula $C_{13}H_{21}{}^{35}Cl\ N_2$

**Example 1: (3-Chlorophenyl)-5-(cyclobutylaminomethyl-4-cyclopropyl-pyridin-2-yl)-amine acetate**

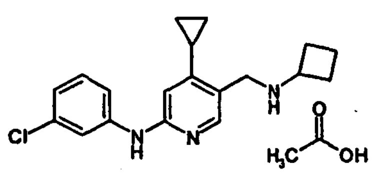

6-(3-Chloro-phenylamino)-4-cyclopropyl-pyridine-3-carbaldehyde (90mg) in methanol (2ml) was added to 4A molecular sieves (70 mg). Aminocyclobutane (94 mg) was added, and the tube shaken for 1 hour. Glacial acetic acid (132 μl) in dichloromethane (0.5ml) and MP-cyanoborohydride polymer reagent (Part number 800406, Argonaut Technologies Inc, 342 mg) were added and the reaction mixture shaken overnight. The polymer was filtered off, washed with methanol, and the filtrate evaporated in vacuo. The residue was purified by triturating the crude product with ether and water to yield the title compound (63 mg).
LC/MS t=2.45 min. Molecular ion observed [MH+] 328 consistent with molecular formula $C_{19}H_{22}{}^{35}ClN_3$
The products in Table 1 were prepared in a similar manner to the preparation of Example 1 from 6-(3-chloro-phenylamino)-4-cyclopropyl-pyridine-3-carbaldehyde and commercially available starting materials except for purification of crude product which was purified by the methods give in the table below.

Purification method:

A Triturate crude product with ether and water
B MDAP then add excess ethereal HCl, and evaporate.
C MDAP
D Biotage Horizon then add excess ethereal HCl, and evaporate

| Example | Name | Structure | Purification method | RT, ( MH+), molecular formula of free base |
|---|---|---|---|---|
| 2 | (3-Chlorophenyl)-(4-cyclopropyl-5-cyclopropylaminomethyl -pyridin-2-yl)-amine acetate | 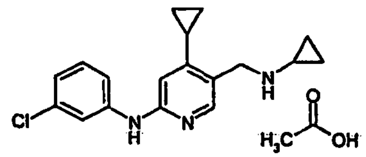 | A | 2.30<br>314<br>$C_{18}H_{20}{}^{35}ClN_3$ |
| 3 | (3-Chlorophenyl)-{4-cyclopropyl-5-[(cyclopropylmethyl-amino)-methyl]-pyridin-2-yl}-amine acetate | 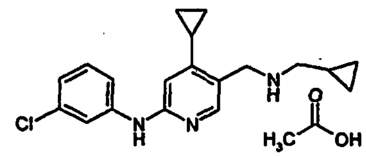 | A | 2.43<br>328<br>$C_{19}H_{22}{}^{35}ClN_3$ |
| 4 | (3-Chlorophenyl)-(4-cyclopropyl-5-morpholin-4-ylmethyl-pyridin-2-yl)-amine dihydrochloride | 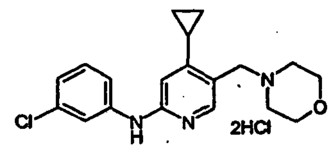 | B | 2.34<br>344<br>$C_{19}H_{22}{}^{35}ClN_3O$ |
| 5 | (3-Chlorophenyl)-[4-cyclopropyl-5-(isobutylamino-methyl)-pyridin-2-yl]-amine acetate | 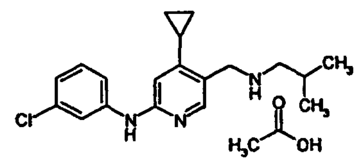 | A | 2.38<br>330<br>$C_{19}H_{24}{}^{35}ClN_3$ |
| 6 | (3-Chlorophenyl)-{4-cyclopropyl-5-[(2-methoxy-ethylamino)-methyl]-pyridin-2-yl}-amine dihydrochloride | 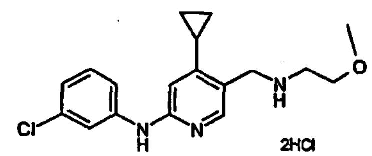 | B | 2.31<br>332<br>$C_{18}H_{22}{}^{35}ClN_3O$ |

(continued)

| Example | Name | Structure | Purification method | RT, ( MH+), molecular formula of free base |
|---|---|---|---|---|
| 7 | (3-Chlorophenyl)-[4-cyclopropyl-5-isopropylamino-methyl)-pyridin-2-yl]-amine acetate | 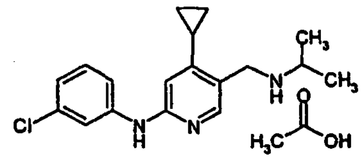 | A | 2.29<br>316<br>$C_{18}H_{22}{}^{35}ClN_3$ |
| 8 | (3-Chlorophenyl)-(4-cyclopropyl-5-{[(tetrahydro-pyran-4-ylmethyl)-amino]-methyl}-pyridin-2-yl)-amine formate | 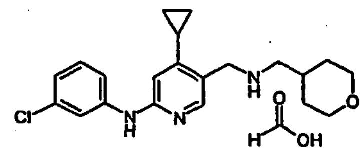 | C | 2.37<br>372<br>$C_{21}H_{26}{}^{35}ClN_3O$ |
| 9 | N-(2-{[6-(3-Chlorophenylamino)-4-cyclopropyl-pyridin-3-ylmethyl]-amino}-ethyl)-acetamide dihydrochloride | 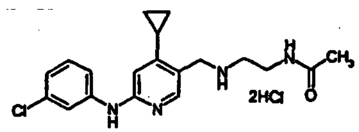 | D | 2.23<br>359<br>$C_{19}H_{23}{}^{35}ClN_4O$ |
| 10 | 4-({[6-(3-Chloro-phenylamino)-4-cyclopropyl-pyridin-3-ylmethyl]-amino}-methyl)-tetrahydro-pyran-4-ol | 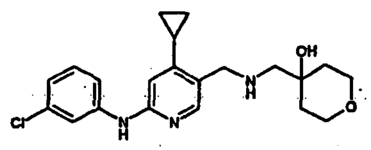 | D | 2.64<br>386<br>$C_{22}H_{28}{}^{35}ClN_3O$ |
| 11 | (3-Chlorophenyl)-{4-cyclopropyl-5-[(tetrahydro-pyran-4-ylamino)-methyl]-pyridin-2-yl}-amine acetate | 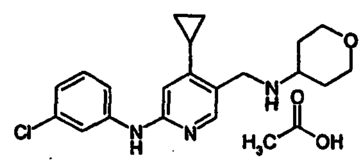 | A | 2.30<br>358<br>$C_{20}H_{24}{}^{35}ClN_3O$ |

**Example 12: {5-[(Cyclobutylmethyl-amino)-methyl]-4-isopropyl-pyridin-2-yl}-m-tolyl-amine**

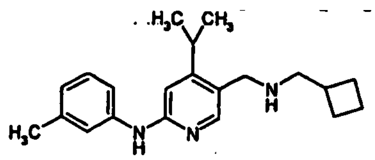

A solution of borane-tetrahydrofuran complex (1M in tetrahydrofuran, 0.3ml) was added to a solution of N-cyclobutylmethyl-4-isopropyl-6-m-tolylamino-nicotinamide, (33mg) in dry tetrahydrofuran (2ml) under nitrogen at room temperature. The solution was refluxed overnight then cooled to room temperature. Methanol (1ml) was added, the mixture cooled in ice and a 1M solution of hydrogen chloride in diethyl ether (1ml) added. The mixture was refluxed for 2hrs then evaporated to dryness and re-evaporated twice from methanol. The residue was purified by MDAP to give the title compound (4.5mg)
LC/MS t = 2.44 min, Molecular ion observed [$MH^+$]324, consistent with molecular formula $C_{21}H_{29}N_3$.

**Example 13: (3-Chloro-phenyl)-{5-[(cyclobutylmethyl-amino)-methyl]-4-cyclopropyl-pyridin-2-yl}-amine**

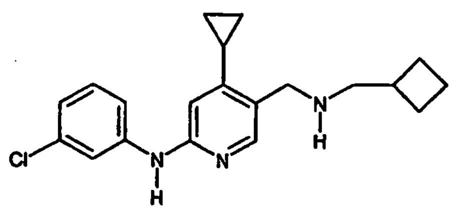

To a solution of 6-(3-chloro-phenylamino)-N-cyclobutylmethyl4-cyclopropyl-nicotinamide, (100mg) in anhydrous tetrahydrofuran (2ml) was added 1.5M borane THF complex (0.95ml, 5eq) dropwise under nitrogen at room temperature. The mixture was stirred at room temperature for 30 minutes followed by heating at reflux for 18 hours. On cooling, methanol (3ml) was added slowly, and the mixture was stirred at room temperature for 1 hour. The mixture was evaporated under reduced pressure, and the residue was treated with 2N hydrochloric acid (3ml). The mixture was heated to 60° for 1 hour. On cooling, it was neutralised to pH 7.4 with aqueous saturated sodium hydrogen carbonate and extracted with ethyl acetate (3x 10ml). The ethyl acetate layers were combined, dried ($MgSO_4$), evaporated under reduced pressure and the residue was purified using MDAP to give (3-chloro-phenyl)-{5-[(cyclobutylmethyl-amino)-methyl]-4-cyclopropyl-pyridin-2-yl}-amine (38mg) as white solid.
LC/MS t = 2.58 min, molecular ion observed $[MH]^+$ 342, consistent for $C_{20}H_{24}{}^{35}ClN_3$
NMR (MeOD) δ 0.77-0.84 (2H, m), 1.10-1.19 (2H, m), 1.8-2.07 (5H, m), 2.15-2.25 (2H, m), 2.68-2.79 (1H, m), 3.15 (2H, d), 4.30 (2H, s), 6.41 (1H, s), 6.91 (1H, dd), 7.20 (1H, t), 7.35 (1H, dd), 7.84 (1H, t), 8.15 (1H, s), 8.49 (1H, br s).

The compounds in the table below were prepared using precursors that are all either described herein or are commercially available. The method used for the preparation is given in column 4 of the table.

Method A is as for Example 13.
Method B as for Method A, except that the mixture was neutralised with 2N sodium hydroxide instead of neutralised with aqueous saturated sodium hydrogen carbonate

| Example No | Name | Structure | Prep. Meth | Ret Time (min) MH+ Molecular Formula of free base |
|---|---|---|---|---|
| 14 a) | (3-Chloro-phenyl)-{5-[(cyclobutylmethyl-amino)-methyl]-4-isopropyl-pyridin-2-yl}-amine, compound with formic acid (1:1) | 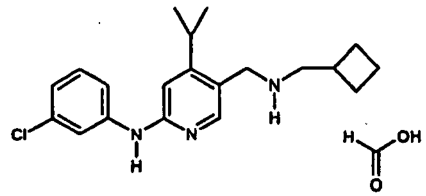 | A | 2.60<br>344<br>$C_{20}H_{26}{}^{35}ClN_3$ |
| 15a) | (3-Bromo-phenyl)-{5-[(cyclobutylmethyl-amino)-methyl]-4-isopropyl-pyridin-2-yl}-amine | 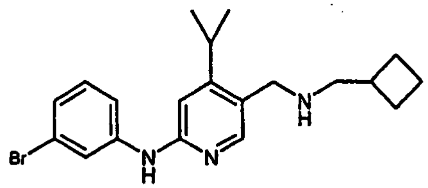 | A | 2.69<br>390<br>$C_{20}H_{26}{}^{81}BrN_3$ |
| 16 | {5-[(Cyclobutylmethyl-amino)-methyl]-4-isopropyl-pyridin-2-yl}-(2,4-dichloro-phenyl)-amine, compound with formic acid (1:1) | 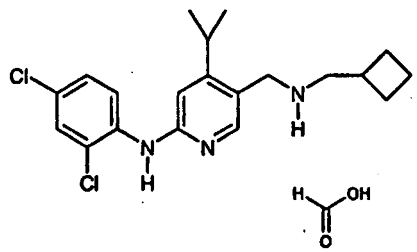 | A | 2.76<br>378<br>$C_{20}H_{25}{}^{35}Cl_2N_3$ |
| 17 | {5-[(Cyclobutylmethyl-amino)-methyl]-4-isopropyl-pyridin-2-yl}-(3,4-dichloro-phenyl)-amine, compound with formic acid (1:1.2) | 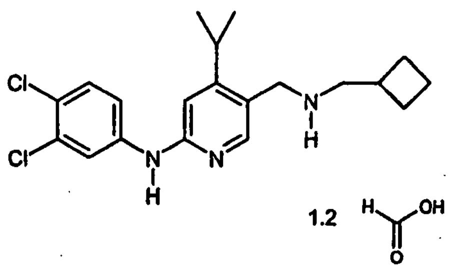 1.2 | A | 2.90<br>378<br>$C_{20}H_{25}{}^{35}Cl_2N_3$ |

(continued)

| Example No | Name | Structure | Prep. Meth | Ret Time (min) MH+ Molecular Formula of free base |
|---|---|---|---|---|
| 18 | (3-Chloro-4-fluoro-phenyl)-{5-[(cyclobutylmethyl-amino)-methyl]-4-isopropyl-pyridin-2-yl}-amine, compound with formic acid (1:1.3) | 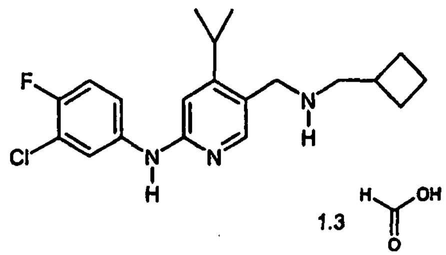 1.3 | A | 2.40<br>362<br>$C_{20}H_{25}{}^{35}ClFN_3$ |
| 19a) | {5-[(Cyclobutylmethyl-amino)-methyl]-4-isopropyl-pyridin-2-yl}-(2-fluoro-3-trifluoromethyl-phenyl)-amine, compound with formic acid (1:1) | 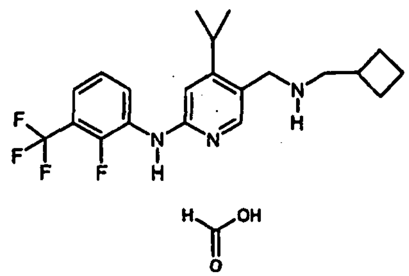 | B | 2.74<br>396<br>$C_{21}H_{25}F_4N_3$ |
| 20 | {5-[(Cyclobutylmethyl-amino)-methyl]-4-isopropyl-pyridin-2-yl}-(2,5-dichloro-phenyl)-amine, compound with formic acid (1:1) | 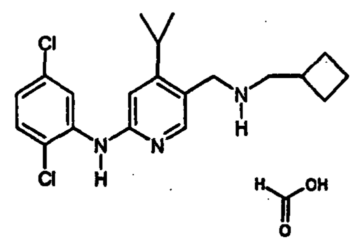 | A | 2.77<br>378<br>$C_{20}H_{25}{}^{35}Cl_2N_3$ |
| 21 | {5-[(Cyclobutylmethyl-amino)-methyl]-4-isopropyl-pyridin-2-yl}-(3,5-difluoro-phenyl)-amine, compound with formic acid (1:1.3) | 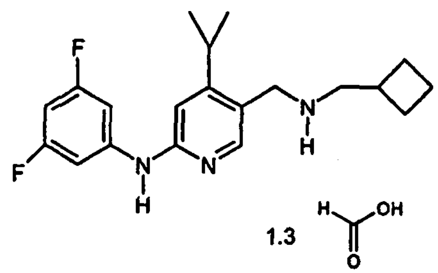 1.3 | A | 2.66<br>346<br>$C_{20}H_{25}F_2N_3$ |

(continued)

| Example No | Name | Structure | Prep. Meth | Ret Time (min) MH+ Molecular Formula of free base |
|---|---|---|---|---|
| 22 | (3-Chloro-phenyl)-{5-[(cyclopropylmethyl-amino)-methyl]-4-isopropyl-pyridin-2-yl}-amine, compound with formic acid (1: 1.25) | 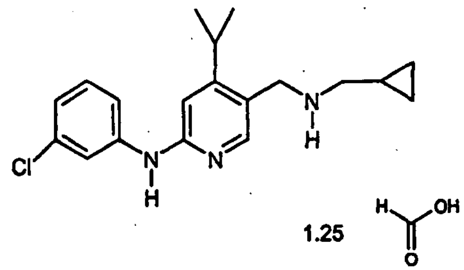 1.25 | A | 2.53<br>330<br>$C_{19}H_{24}{}^{35}ClN_3$ |
| 23 | (2,4-Dichloro-phenyl)-[5-(isobutylamino-methyl)-4-isopropyl-pyridin-2-yl]-amine, compound with formic acid (1:1) | 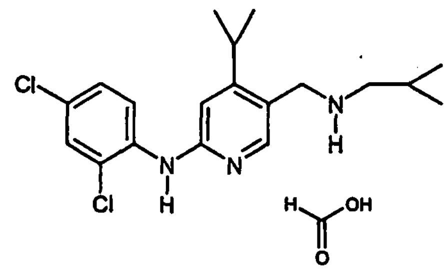 | A | 2.74<br>366<br>$C_{19}H_{25}{}^{35}Cl_2N_3$ |
| 24 | (2-Chloro-4-fluoro-phenyl)-{5-[(cyclobutylmethyl-amino)-methyl]-4-isopropyl-pyridin-2-yl}-amine, compound with formic acid (1:1.75) | 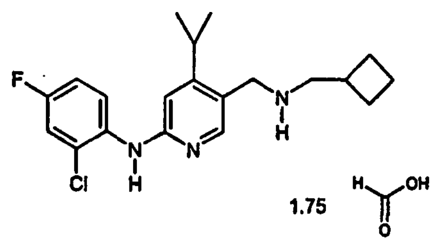 1.75 | A | 2.50<br>362<br>$C_{20}H_{25}{}^{35}ClFN_3$ |
| 25 | (4-Chloro-2-fluoro-phenyl)-{5-[(cyclobutylmethyl-amino)-methyl]-4-isopropyl-pyridin-2-yl}-amine, compound with formic acid (1:1.67) | 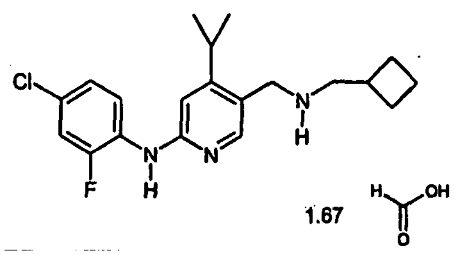 1.67 | A | 2.40<br>362<br>$C_{20}H_{25}{}^{35}ClFN_3$ |

(continued)

| Example No | Name | Structure | Prep. Meth | Ret Time (min) MH+ Molecular Formula of free base |
|---|---|---|---|---|
| 26 | (2-Bromo-4-trifluoromethoxy-phenyl)-{5-[(cyclobutylmethyl-mino)-methyl]-4-isopropyl-pyridin-2-yl}-amine, compound with formic acid (1:1) | 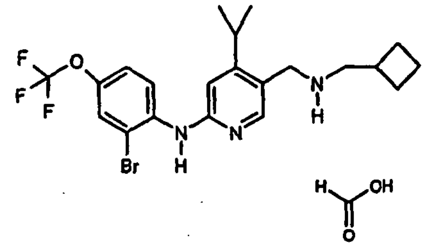 | A | 2.92<br>474<br>$C_{21}H_{25}{}^{81}BrF_3N_3O$ |
| 27 | (4-Chloro-2-methyl-phenyl)-{5-[(cyclobutylmethyl-amino)-methyl]-4-isopropyl-pyndin-2-yl}-amine, compound with formic acid (1:1.33) | 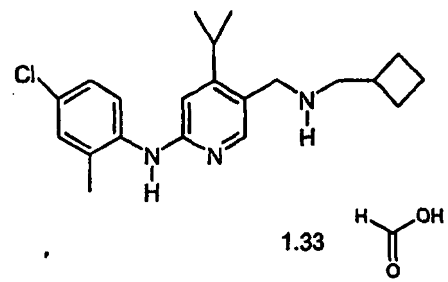 1.33 | A | 2.60<br>358<br>$C_{21}H_{28}{}^{35}ClN_3$ |
| 28 | (3-Chloro-4-fluoro-phenyl)-{5-[(cyclobutylmethyl-amino)-methyl]-4-cyclopropyl-pyridin-2-yl}-amine, compound with formic acid (1:1.5) | 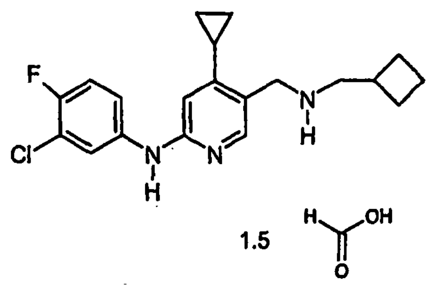 1.5 | A | 2.66<br>360<br>$C_{20}H_{23}{}^{35}ClFN_3$ |

(continued)

| Example No | Name | Structure | Prep. Meth | Ret Time (min) MH+ Molecular Formula of free base |
|---|---|---|---|---|
| 29 | (3-Chloro-2-methyl-phenyl)-{5-[(cyaobutylmethyl-amino)-methyl]-4-cyclopropyl-pyridin-2-yl}-amine, compound with formic acid (1:1.57) | 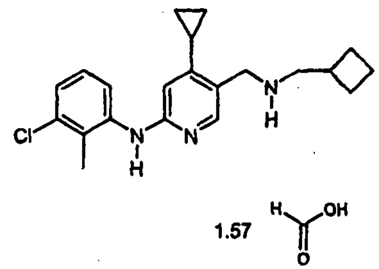 1.57 | A | 2.50<br>356<br>$C_{21}H_{26}{}^{35}ClN_3$ |
| 30 | {5-[(Cyclopropylmethyl-amino)-methyl]-4-isopropyl-pyridin-2-yl}-(3-trifluoromethyl-phenyl)-amine, compound with formic acid (1:1) | 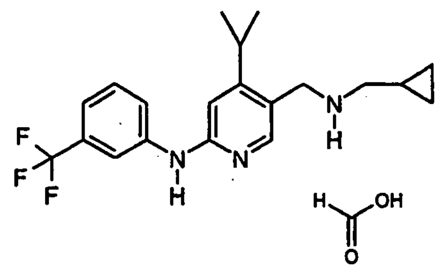 | A | 2.65<br>364<br>$C_{20}H_{24}F_3N_3$ |
| 31 | {5-[(Cyclopropylmethyl-amino)-methyl]-4-isopropyl-pyridin-2-yl}-(3,5-difluoro-phenyl)-amine, compound with formic acid (1:1) | 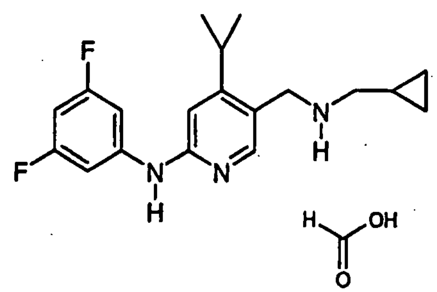 | A | 2.50<br>332<br>$C_{19}H_{23}F_2N_3$ |

(continued)

| Example No | Name | Structure | Prep. Meth | Ret Time (min) MH+ Molecular Formula of free base |
|---|---|---|---|---|
| 32 | {5-[(Cyclopropylmethyl-amino)-methyl]-4-isopropyl-pyridin-2-yl}-(2-fluoro-3-trifluoromethyl-phenyl)-amine, compound with formic acid (1:1) | 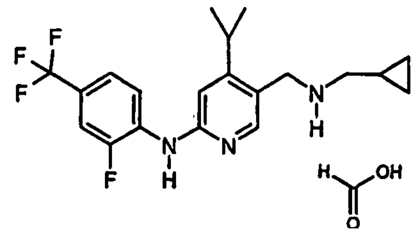 | A | 2.65<br>382<br>$C_{20}H_{23}F_4N_3$ |
| 33 | (3-Chloro-4-methyl-phenyl)-{5-[(cyclopropylmethyl-amino)-methyl]-4-isopropyl-pyridin-2-yl}-amine, compound with formic acid (1:1) | 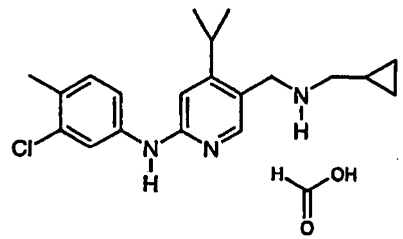 | A | 2.65<br>344<br>$C_{20}H_{26}{}^{35}ClN_3$ |
| 34 | (3-Chloro-4-fluoro-phenyl)-{5-[(cyclopropylmethyl-amino)-methyl]-4-isopropyl-pyridin-2-yl}-amine, compound with formic acid (1:1) | 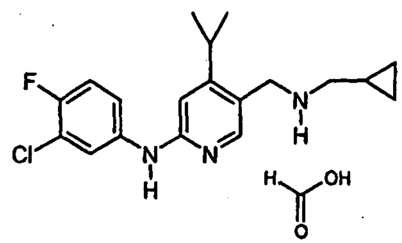 | A | 2.57<br>348<br>$C_{19}H_{23}{}^{35}ClFN_3$ |
| 35 | {5-[(Cyclobutylmethyl-amino)-methyl]-4-isopropyl-pyridin-2-yl}-(2,4-difluoro-phenyl)-amine, compound with formic acid (1:1.57) | 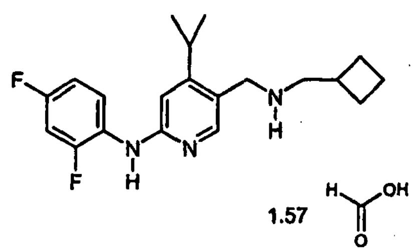 1.57 | A | 2.34<br>346<br>$C_{20}H_{25}F_2N_3$ |

(continued)

| Example No | Name | Structure | Prep. Meth | Ret Time (min) MH+ Molecular Formula of free base |
|---|---|---|---|---|
| 36 | (4-Chloro-2-methyl-phenyl)-{5-[(cyclobutylmethyl-amino)-methyl]-4-cyclopropyl-pyridin-2-yl}-amine, compound with formic acid (1:1.58) | 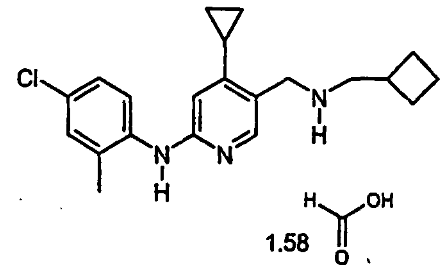 1.58 | A | 2.52<br>356<br>$C_{21}H_{26}{}^{35}ClN_3$ |
| 37 | {5-[(Cyclopropylmethyl-amino)-methyl]-4-isopropyl-pyridin-2-yl}-(3,4-difluoro-phenyl)-amine, compound with formic acid (1:1.15) | 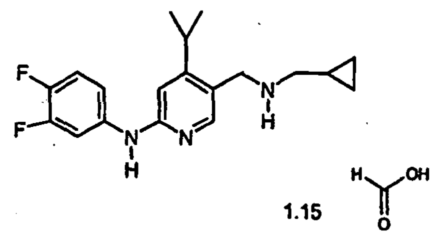 1.15 | A | 2.41<br>332<br>$C_{19}H_{23}F_2N_3$ |
| 38 | 2-(2-(2,4-Dichloro-phenylamino)-5-{[(tetrahydro-pyran-4-ylmethyl)-amino]-methyl}-pyridin-4-yl)-propan-2-ol, compound with formic acid (1:1) | 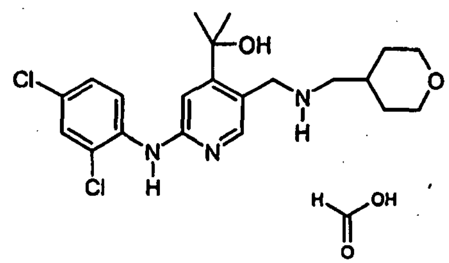 | A | 2.47<br>424<br>$C_{21}H_{27}{}^{35}Cl_2\ N_3\ O_2$ |
| 39 | (3-Bromo-phenyl)-(6-cyclopropyl-5-{[(tetrahydro-pyran-4-ylmethyl)-amino]-methyl}-pyridin-2-yl)-amine, compound with formic acid (1:1.33) | 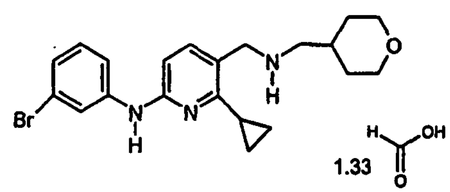 1.33 | A | 2.56<br>418<br>$C_{21}H_{26}{}^{81}BrN_3O$ |

(continued)

| Example No | Name | Structure | Prep. Meth | Ret Time (min) MH+ Molecular Formula of free base |
|---|---|---|---|---|
| 40 | (6-Cyclopropyl-5-{[(tetrahydro-pyran-4-ylmethyl)-amino]-methyl}-pyridin-2-yl)-(3,5-difluoro-phenyl)-amine, compound with formic acid (1:1) | 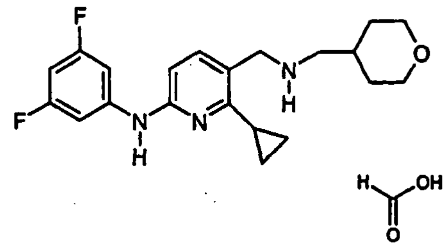 | A | 2.58<br>372<br>$C_{21}H_{25}F_2N_3O$ |
| 41 | (6-Cyclopropyl-5-{[(tetrahydro-pyran-4-ylmethyl)-amino]-methyl}-pyridin-2-yl)-(3,4-dichloro-phenyl)-amine, compound with formic acid (1:1.43) | 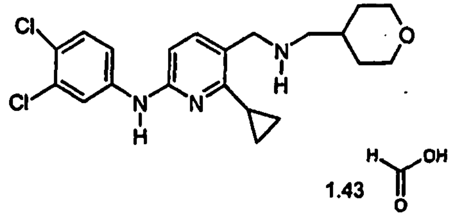 1.43 | A | 2.83<br>406<br>$C_{21}H_{25}{}^{35}Cl_2N_3O$ |
| 42 | (3-Chloro-phenyl)-(6-cyclopropyl-5-{[ (tetrahydro-pyran-4-ylmethyl)-amino]-methyl}-pyridin-2-yl)-amine, compound with formic acid (1: 1.37) | 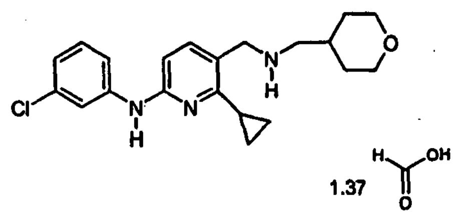 1.37 | A | 2.62<br>372<br>$C_{21}H_{26}{}^{35}ClN_3O$ |
| 43 | (4-Cyclopentyl-5-{[(tetrahydro-pyran-4-ylmethyl)-amino]-methyl}-pyridin-2-yl)-(2,4-dichloro-phenyl)-amine, compound with formic acid (1:0.78) | 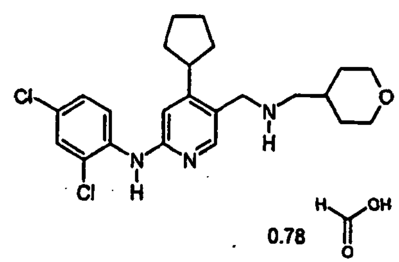 0.78 | A | 2.74<br>434<br>$C_{23}H_{29}{}^{35}Cl_2N_3O$ |

(continued)

| Example No | Name | Structure | Prep. Meth | Ret Time (min) MH+ Molecular Formula of free base |
|---|---|---|---|---|
| 44 | (3-Chloro-4-fluoro-phenyl)-{5-[(cyclopentylmethyl-amino)-methyl]-4-cyclopropyl-pyridin-2-yl}-amine, compound with formic acid (1:1.4) | 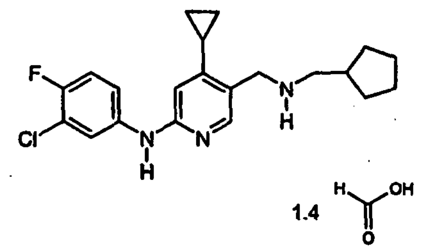 | B | 281<br>374<br>$C_{21}H_{25}{}^{35}ClFN_3$ |
| 45 | {5-[(Cyclopentylmethyl-amino)-methyl]-cyclopropyl-pyridin-2-yl}-(4-fluoro-3-trifluoromethyl-phenyl)-amine, compound with formic acid (1:1.25) | 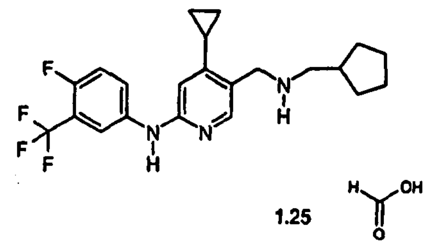 | B | 2.91<br>408<br>$C_{22}H_{25}F_4N_3$ |
| 46 | (2-Fluoro-3-trifluoromethyl-phenyl)-5-[(isobutylamino-methyl)-4-isopropyl-pyridin-2-yl]-amine, compound with formic acid (1:1.13) | 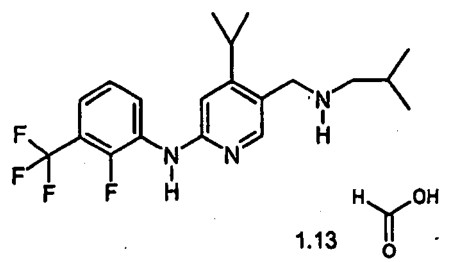 | B | 2.72<br>384<br>$C_{20}H_{25}F_4N_3$ |
| 47 | (3,4-Dichloro-phenyl)-[5-(isobutylamino-methyl)-4-isopropyl-pyridin-2-yl]-amine, compound with formic acid (1:1) | 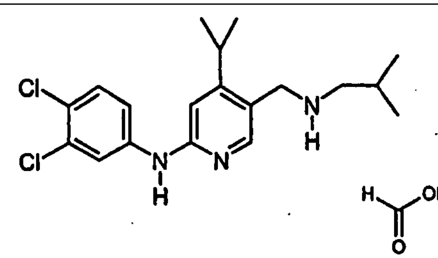 | B | 2.85<br>366<br>$C_{19}H_{25}{}^{35}Cl_2N_3$ |

(continued)

| Example No | Name | Structure | Prep. Meth | Ret Time (min) MH+ Molecular Formula of free base |
|---|---|---|---|---|
| 48 | (3-Bromo-phenyl)-[5-(isobutylamino-methyl)-4-isopropyl-pyridin-2-yl]-amine, compound with formic acid (1:33) | 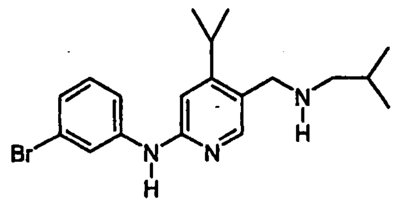 1.33 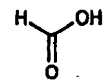 | B | 2.69<br>378<br>$C_{19}H_{26}{}^{81}BrN_3$ |
| 49 | (3-Bromo-phenyl)-(4-isopropyl-5-{[ (tetrahydro-pyran-4-ylmethyl)-amino]-methyl}-pyridin-2-yl)-methyl-amine, compound with formic acid (1:1) | 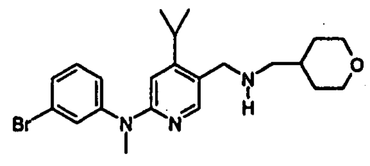 1.1 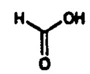 | B | 2.53<br>434<br>$C_{22}H_{30}{}^{81}BrN_3O$ |
| 50 | (3-Chloro-phenyl)-{5-[(cyclopentylmethyl-amino)-methyl]-4-isopropyl-pyridin-2-yl}-amine, compound with formic acid (1:1) | 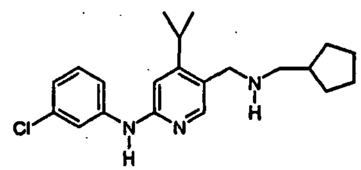 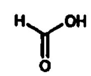 | B | 2.78<br>358<br>$C_{21}H_{28}{}^{35}ClN_3$ |
| 51 | [5-(Isobutylamino-methyl)-4-isopropyl-pyridin-2-yl]-(3-trifluoromethyl-phenyl)-amine, compound with formic acid (1:1,25) | 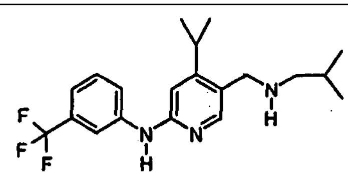 1:1,25 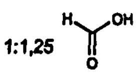 | B | 2.73<br>366<br>$C_{20}H_{26}F_3N_3$ |

**Example 52: (3-Chloro-phenyl)-{4-cyclopropyl-5-[(4-fluoro-benzylamino)-methyl]-pyridin-2-yl}-amine dihydrochloride**

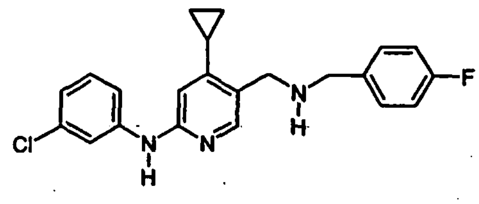

2HCl

This was prepared from 6-(3-chloro-phenylamino)-4-cyclopropyl-pyridine-3-carbaldehyde and 4-fluorobenzylamine by the method used for Example 1, but the final product was treated with hydrogen chloride in 1,4-dioxan, stood for 1 hour at room temperature, and then evaporated to give the title compound (28mg).
LC/MS t = 2.76 min, molecular ion observed [MH+] 382, consistent with $C_{22}H_{21}{}^{35}ClFN_3$

**Example 53: (3-Chloro-4-trifluoromethoxyphenyl)-(4-isopropyl-5-{[(tetrahydropyran-4-ylmethyl)-amino]-methyl}-pyridin-2-yl)-amine**

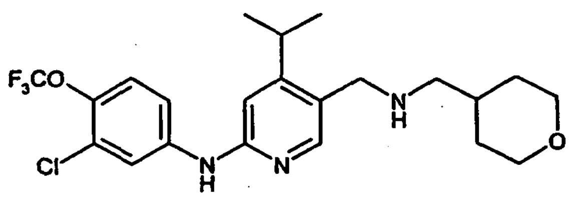

To a solution of 6-(3-chloro-4-trifluoromethoxyphenylamino)- 4-isopropyl-N-(tetrahydropyran-4-ylmethyl)-nicotinamide, (60 mg) in dry tetrahydrofuran (1 ml) was added borane-tetrahydrofuran complex (1.0M in tetrahydrofuran, 0.64 ml) and the solution stirred at 70°C for 4h. Further borane-tetrahydrofuran complex (1.0M in tetrahydrofuran, 0.64 ml) was added and stirring at 70°C continued for 24h. The solution was quenched with methanol and then 6N hydrochloric acid (six drops) was added. Stirring at 70°C was continued for 1h and then the solvent was removed under reduced pressure. The residue was purified using the MDAP system detailed at the beginning of the experimental section to afford the title compound as an off white foam (11 mg).
NMR (MeOD) δ 1.29-1.40 (8H, d,m), 1.72-1.75 (2H, d), 2.01 (1H, m), 3.05 (2H, d), 3.20 (1H, m), 3.45 (2H, m), 3.96 (2H, dd), 4.28 (2H, s), 6.90 (1H, s), 7.35 (1H, d), 7.50 (1H, dd), 8.04 (1H, d), 8.25 (1H, s).
LC/MS t = 2.6 min, molecular ion observed [$MH^+$] 458, consistent with molecular formula $C_{22}H_{27}{}^{35}Cl\ F_3N_3O_2$

**Example 54: (2,4-Dichlorophenyl)-{5-[(4-fluoro-benzylamino)-methyl]-4-trifluoromethyl-pyridin-2-yl}-amine**

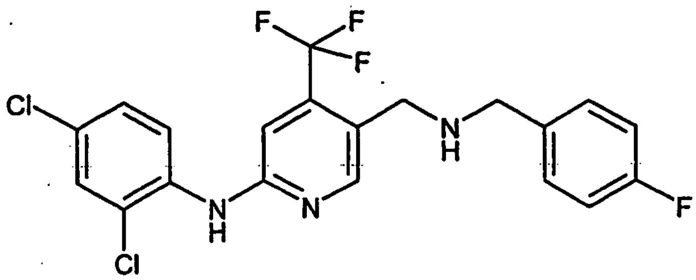

6-(2,4-Dichloro-phenylamino)-4-trifluoromethyl-pyridine-3-carbaldehyde , (100mg) and 4-fluorobenzylamine (45mg) were dissolved in dry methanol (5ml) in the presence of 3A molecular sieves and the reaction mixture was stirred at room temperature for 2h. The mixture was diluted with DCM (1ml), then sodium cyano borohydride (27mg) and acetic acid (60μl) were added and the solution was stirred at room temperature for 30 min. The molecular sieves were filtered off, and the filtrate was washed with aqueous 5% potassium carbonate, water, and the dried ($MgSO_4$) organic layer was concentrated under reduced pressure. A solution of the residue in DCM was treated with 5% hydrochloric acid, and the precipitate that formed was collected and recrystallised from methanol/ether to yield the title compound (30mg).
NMR (300MHz, DMSO-d6) δ 9.49 (br s, 1H), 9.19 (s, 1H), 8.51 (s, 1H), 7.97 (d, 1H); 7.66 (d, 1H), 7.59 (m, 2H), 7.43 (dd, 1H), 7.38 (s, 1H), 7.28 (dd, 2H), 4.21 (br s, 2H), 4.12 (br s, 2H).
MS m/z (EI+); TSQ700: source 180° ; 70V; 200uA: 445.1,443.1,318.9,307.0,285.1. MS m/z (ESI+): 445 (MH+).

**Example 55: (3-Chloro-phenyl)-(4-cyclopropyl-5-propylaminomethyl-pyridin-2-yl)-amine dihydrochloride**

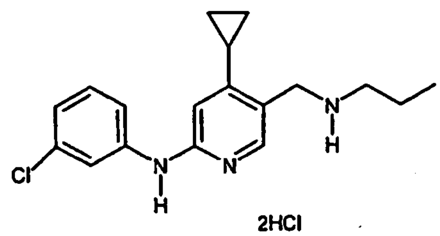

(3-Chloro-phenyl)-(4-cyclopropyl-5-propylaminomethyl-pyridin-2-yl)-amine was prepared as in Example 1 using 6-(3-chloro-phenylamino)-4-cyclopropyl-pyridine-3-carbaldehyde (100mg) and propylamine (78mg). The title compound was prepared by treatment with 1M HCl in ether (2ml), the mixture was stirred for 10 minutes at room temperature and evaporated in vacuo to give the title compound (55mg, 47%)
Molecular ion observed m/z = 316 [$MH^+$] consistent with formula $C_{18}$ $H_{22}$ $^{35}Cl$ $N_3$
NMR (DMSO) δ 0.72-0.78 (2H, m), 0.91-0.94 (3H, m), 1.07-1.11 (2H, m), 1.66-1.76 (2H, m), 2.19-2.26 (1H, m), 2.80-3.00 (2H, m), 4.22-4.24 (2H, m), 6.56 (1H, s), 7.00-7.02 (1H, m), 7.29-7.33 (1H, m), 7.43-7.45 (1H, m), 7.90 (1H, s), 8.28 (1H, s), 9.21 (2H, bs), 9.85 (1H, s).

**Example 14b): (3-Chloro-phenyl)-{5-[(cyclobutylmethyl-amino)-methyl]-4-isopropyl-pyridin-2-yl}-amine dihydrochloride**

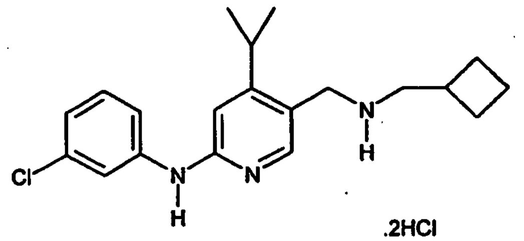

.2HCl

To a stirred solution of 6-(3-chloro-phenylamino)-N-cyclobutylmethyl-4-isopropyl-nicotinamide (100mg), under nitrogen, in anhydrous tetrahydrofuran (2ml) was added 1.5M borane THF complex (0.95ml, 5eq) drop wise at room temperature. The mixture was stirred at room temperature for 30 minutes followed by heating to reflux for 18 hours. On cooling, methanol (3ml) was added slowly. The mixture was stirred at room temperature for 1 hour and evaporated under reduced pressure on a Buchi. To the residue was added aqueous 2N hydrochloric acid (3ml). The mixture was heated to 60˚C for 1 hour. On cooling it was basified with saturated sodium hydrogen carbonate and extracted with ethyl acetate (3x 10ml). The ethyl acetate layers were combined, dried ($MgSO_4$), evaporated in vacuo and the residue was purified using Mass directed autoprep to give (3-chloro-phenyl)-{5-[(cyclobutylmethyl-amino)-methyl]-4-isopropyl-pyridin-2-yl}-amine as the formate salt. This was treated with 1M HCl in ether(2ml), the mixture was stirred for 10 minutes at room temperature and evaporated in vacuo to give the title compound (113.4mg).
Molecular ion observed m/z= 342 [MH+] consistent with formula $C_{20}$ $H_{24}$ $N_3$ $^{35}Cl$
NMR (MeOD) δ 1.12-1.19 (6H, m), 1.81-2.09 (5H, m), 2.15-2.24(2H, m), 2.66-2.79 (1H, m), 3.15 (2H, d), 4.30 (2H, s), 6.41 (1H,s), 6.89-6.93 (1H ,m), 7.18-7.27(1H, m), 7.33-7.37 (1H, m), 7.84-7.87 (1H, m), 8.16 (1H, s), 8.50 (1H, bs).

The compounds in the table below were prepared using one of the following methods as indicated in column 4 of the table.

Method A: The compounds were prepared in a manner similar to Example 14b).

Method B : As for Method A, above, but the product obtained after MDAP was treated with 3M HCl in 1,4-dioxane and the mixture was evaporated in vacuo. Ether was added and the resulting solid was filtered off, washed with ether, and dried to give the product.

Method C : As for Method A, above, but the product obtained after MDAP was treated with 3M HCl in 1,4-dioxane and the mixture was evaporated in vacuo. The resulting solid was taken up into 1,4-dioxane (2ml), treated with water (0.1ml), and freeze dried to give the compound.

| Example no | Structure | Name | Method | MH+ Mol Formula NMR |
|---|---|---|---|---|
| 15b) | 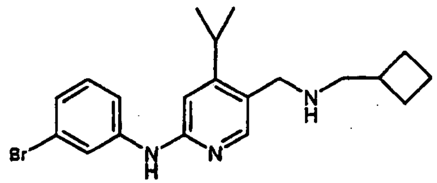 2HCl | (3-Bromo-phenyl)-{5-[(cyclobutylinethyl-amino)-methyl]-4-isopropyl-pyridin-2-yl}-amine dihydrochloride | B | 390<br>$C_{20}H_{26}{}^{81}BrN_3$<br>NMR (MeOD) δ 1.24 (3H, s), 1.26 (3H, s), 1.63-1.78 (2H, m), 1.80-2.00 (2H, m), 2.03-2.13 (2H, m), 2.48-2.60 (1H, m), 2.68(2H, d), 3.11-3.24 (1H, m), 3.69 (2H, s), 6.78 (1H, s), 6.99-7.04 (1H, m), 7.12(1H, t), 7.36-7.40 (1H, m), 7.88 (1H, t), 8.01 (1H, s). |
| 19b) | 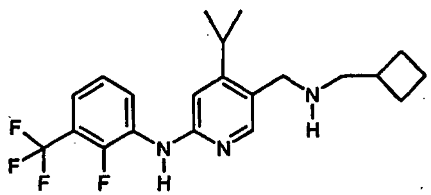 2HCl | 5-{[(cyclobutylmethyl)amin o]methyl}-*N*-[2-fluoro-3-(trifluoromethyl)phenyl]-4-(1-methylethyl)-2-pyridinamine dihydrochloride | C | 396<br>$C_{21}H_{2S}F_4N_3$<br>NMR (MeOD) δ 1.28 (3H, s), 1.30 (3H, s), 1.75-2.34 (6H, m), 2.54-2.82 (1H, m), 3.07-3.20 (3H, m), 3.27-3.32 (2H, m), 6.97 (1H, s), 7.22-7.33 (2H, m), 8.17 (1H, s), 8.27 (1H, s). |

**Example 56: {[1-(Cyclobntylmethyl-amino)-ethyl]-4-isopropyl-pyridin-2-yl}-(2-fluoro-3-trifluoromethyl-phenyl)-amine dihydrochloride**

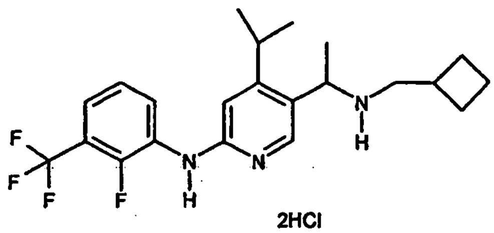

2HCl

This was prepared in a similar manner to the preparation of the intermediate 10 using [1-(6-chloro-4-isopropyl-pyridin-3-yl)-ethyl]-cyclobutylmethyl-amine (70mg) and 2-fluoro-3-(trifluoromethyl)aniline (94mg). Purification by MDAP gave a residue which was treated with 3M HCl in 1,4-dioxane and the resultant mixture was evaporated in vacuo. The resulting solid was taken up into 1,4-dioxane(2ml) treated with water (0.1 ml), and freeze dried to give the title compound (57mg , 52%)
Molecular ion observed m/z= 410 [MH+] consistent with formula $C_{22}$ $H_{27}$ $F_4$ $N_3$
NMR NMR (MeOD) δ 1.27 (3H, d), 1.38 (3H, d), 1.71 (3H, d), 1.78- 2.06 (4H, m), 2.12-.24 (2H, m), 2.65 (2H, s), 3.00-3.10 (1H, m), 3.16-3.27(1H, m), 4.66-4.77 (1H, m), 7.18 (1H, s), 7.50-7.56 (1H, m), 7.73-7.80 (1H, m), 7.85-7.93 (1H, m), 8.19 (1H, s).

**Example 57: (3-Chloro-phenyl)-[5-(4,4-difluoro-piperidin-1-ylmethyl)-4-isopropyl-pyridin-2-yl]-amine diydrochloride**

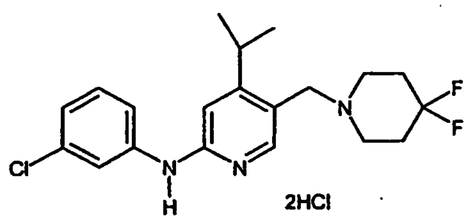

2HCl

To 6-(3-chloro-phenylamino)-4-isopropyl-pyridine-3-carbaldehyde(100mg) in an Alltech tube was added powdered 4A molecular sives(70mg), 4,4-Difluoro-piperidine (180mg) followed by methanol (1.5ml). The mixture was capped and shaken for 4 hours. Glacial acetic acid (0.15ml) was added followed by polymer supported sodium cyanoborohydride (0.34g). The mixture was then shaken overnight at room temperature. The mixture was filtered and the filtrate evaporated in vacuo. The residue was purified by MDAP and treated with 1M HCl in ether. The resulting solid was filtered off, treated with water and 1,4-dioxan and then freeze-dried to give the title compound (73mg, 53%).
Molecular ion observed m/z= 380 [MH+] consistent with formula $C_{20}H_{24}$ $^{35}ClF_2N_3$
NMR (MeOD) δ 1.23 - 1.28 (6H, m), 1.92 - 2.01 (4H, m), 2.63 - 2.65 (4H, m), 3.29 - 3.37 (1H, m), 3.57 (2H, s), 6.78 (1H, s), 6.87-6.90 (1H, m), 7.18-7.22 (1H,m), 7.31-7.34 (1H, m), 7.72 - 7.73(1H, m), 7.93 (1H, s), 8.13 (1H, s)

The following compounds were prepared by similar manner as described for Example 57, using the appropriate amine.

| Example no | Structure | Name | MH+<br>Mol Formula<br>NMR |
|---|---|---|---|
| 58 | 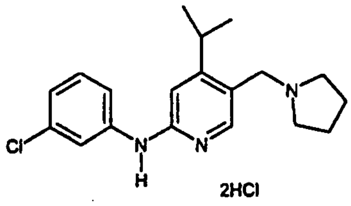 | (3-Chloro-phenyl)-(4-isopropyl-5-pyrrolidin-1-ylmethyl-pyridin-2-yl)-amine. dihydrochloride | 380<br>$C_{19}H_{24}ClN_3$<br>NMR (MeOD) δ 1.27-1.29 (6H, m), 2.07-2.11 (4H, m), 3.13-3.20 (1H, m), 4.36 (2H,s), 6.82 (1H, s), 6.91-6.93 (1H, m), 7.20-7.24 (1H, m), 7.36-7.39 (1H, m), 7.87-7.88 (1H,m), 8.20 (1H, s), 8.39 (1H,s) |
| 59 | 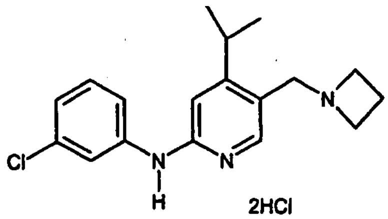 | (5-Azetidin-1-ylmethyl-4-isopropyl-pyridin-2-yl)-(3-chloro-phenyl)-amine. dihydrochloride | 316<br>$C_{18}H_{22}ClN_3$<br>NMR (MeOD) δ 1.26-1.28 (6H, m), 2.44-2.52 (2H, m), 3.07-3.14 (1H, m), 4.10-4.14 (4H, t), 4.35 (2H, s), 6.80 (1H,s), 6.90-6.93 (1H, m), 7.19-7.23(1H, t), 7.35-7.38 (1H, m), 7.87-7.88 (1H, m), 8.15 (1H, s), 8.37 (>1H, bs) |
| 60 | 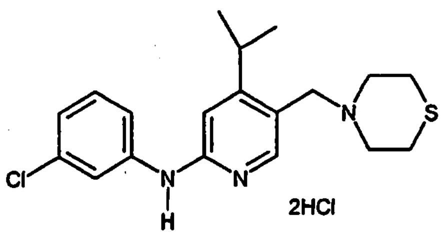 | (3-Chloro-phenyl)-(4-isopropyl-5-thiomorpholin-4-ylmethyl-pyridin-2-yl)-amine. dihydrochloride | 362<br>$C_{19}H_{24}ClN_3S$<br>NMR (MeOD) δ 1.24-1.27 (6H, m) 2.65-2.72 (4H, m), 2.91-2.94 (4H,m), 3.69 (2H, s), 6.79 (1H, s), 6.88-6.90(1H, m), 7.18-7.22 (1H, t), 7.32-7.35 (1H, s), 7.75-7.76(1H, m), 8.19 (1H, s). |

**Example 61: (2,4-Dichloro-phenyl)-(4-isopropyl-5-thiomorpholin-4-ylmethyl-pyridin-2-yl)-amine dihydrochloride**

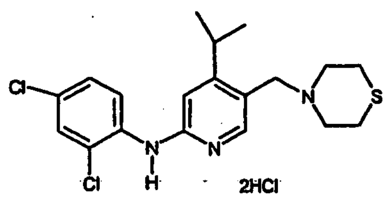

The above compound was prepared by a similar manner to Intermediate 81, from 6-(2,4-dichlorophenylamino)-4-isopropyl-pyridine-3-carbaldehyde (100mg) and thiomorpholine (133mg). The reaction mixture was purified by MDAP and the residue was treated with 1M HCl in ether (2ml). The resultant mixture was stirred for 10 minutes at room

temperature and evaporated in vacuo to give the title compound ( 11mg, 8%).
Molecular ion observed m/z= 396 [MH+] consistent with formula $C_{19} H_{23} {}^{35}Cl_2 N_3 S$
NMR (DMSO) δ 1.17-1.19 (6H, m), 2.54-2.59 (8H, m), 3.13-3.26 (1H, m), 3.32 (2H, s), 7.10 (1H, s), 7.55-7.56 (1H, m), 7.86 (1H, s), 8.20-8.28 (1H, m), 8.31(1H, s).

The following compounds were prepared using a similar method as described for Example 55.

In Example 63 the amine used was tetrahydro-2*H*-thiopyran-4-amine 1,1-dioxide hydrochloride which may be prepared as described by N Sakai in WO 2003/072554.

| Example No | Structure | Name | MH+ Molecular Formula NMR data |
|---|---|---|---|
| 62 | 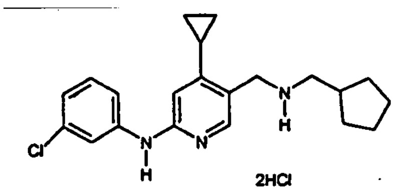 2HCl | (3-Chloro-phenyl)-{5-[(cyclopentylmethyl-amino)-methyl]-4-cyclopropyl-pyridin-2-yl}-amine di-Hydrochloride | 356 $C_{21} H_{26} {}^{35}Cl N_3$ NMR (DMSO) δ 0.72-0.78 (2H, m), 0.91-0.94 (3H, m), 1.07-1.11 (2H, m), 1.66-1.76 (2H, m), 2.19-2.26 (1H, m), 2.80-3.00 (2H, m), 4.22-4.24 (2H, m), 6.56 (1H, s), 7.00-7.02 (1H, m), 7.29-7.33(1H, m), 7.43-7.45 (1H, m), 7.90 (1H, s), 8.28 (1h, s), 9.21 (2H, s), 9.85 (1H, m). |
| 63 | 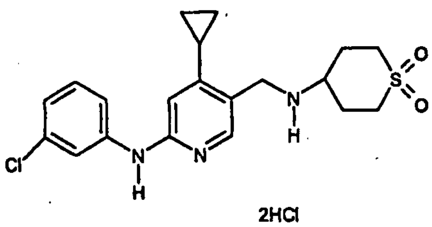 2HCl | (3-Chloro-phenyl)-{4-cyclopropyl-5-[(1,1-dioxo-hexahydro-1$l^6$-thiopyran-4-ylamino)-methyl]-pyridin-2-yl}-amine di-hydrochloride | 406 $C_{20} H_{24} {}^{35}Cl N_3 O_2 S$ NMR (DMSO) δ 0.71-0.75 (2H, m), 1.05-1.09 (2H, m), 2.13-2.24(3H, m), 2.50-2.59 (2H, m), 3.23-3.33 (4H, m), 3.52 (1H, bs), 4.28 (2H, s), 6.53 (1H, s), 6.95-6.97 (1H, d), 7.27-7.31 (1H, t), 7.44-7.46 (1H, d), 7.97 (1H, s), 8.13 (1H, s), 9.45 (2H, s), 9.60 (1H, s). |
| 64 | 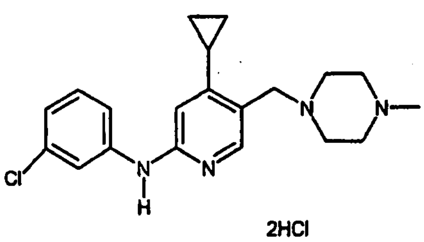 2HCl | (3-Chloro-phenyl)-[4-cyclopropyl-5-(4-methyl-piperazin-1-ylmethyl)-pyridin-2-yl]-amine di-hydrochloride | 357 $C_{20} H_{25} {}^{35}Cl N_4$ NMR (DMSO at 393K) δ 0.71-0.75 (2H, m), 1.05-1.09 (2H, m), 2.08-2.16 (1H, m), 2.68 (3H, s), 2.79 (4H, bs), 3.07 (4H, bs), 4.69 (2H, s), 6.43 (1H, s), 6.80 (1H, d), 7.20 (1H, t), 7.40 (1H, d), 7.79 (1H, s), 8.02 (1H, s) |

**Example 65: [5-(tert-Butylamino-methyl)-4-isopropyl-pyridin-2-yl]-(3-chloro-phenyl)-amine dihydrochloride**

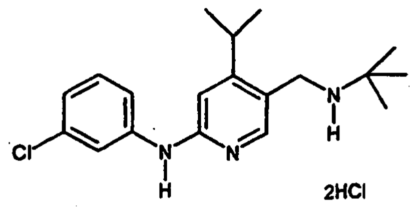

A mixture of tert-butyl-(6-chloro-4-isopropyl-pyridin-3-ylmethyl)-amine (100mg), 3-chloroaniline (105mg), cesium carbonate (190mg), tris(dibenzylideneacetone)di-palladium(0) ($Pd_2(dba)_3$) (20mg), 4,5-bis(diphenylphosphino)-9,9-dimethyl xanthene (Xantphos) (24mg) in 1,4-dioxane(1ml) was irradiated under microwave conditions at 150°C for 30 minutes. Further quantities of cesium carbonate (190mg), $Pd_2(dba)_3$ (20mg) and Xantphos (24mg) were added and the mixture was again irradiated under microwave conditions at 150°C for 30 minutes. Ethyl acetate was added and the mixture was washed with water. The ethyl acetate layer was dried ($Na_2SO_4$) and the solvent was evaporated in vacuo. The residue was purified using MDAP .The residue was treated with 3M HCl in 1,4-dioxane and the mixture was evaporated in vacuo. The resulting solid was taken up into 1,4-dioxane (2ml), treated with water (0.1ml), and freeze dried to give the title compound. (58mg, 43%)
Molecular ion observed m/z = 332 [$MH^+$] consistent with formula $C_{19}$ $H_{26}$ $^{35}Cl$ $N_3$
NMR (MeOD) δ 1.29 (3H, s), 1.31 (3H, s), 1.49 (9H, s), 2.99-3.08 (1H, m), 4.17 (2H, s), 6.82 (1H, s), 6.91 (1H, dd), 7.21 (1H, t), 7.39 (1H, dd), 7.88 (1H, t), 8.19 (1H, s)

Formulations for pharmaceutical use incorporating compounds of the present invention can be prepared in various forms and with numerous excipients. Examples of such formulations are given below.

**Example 66: Inhalant Formulation**

A compound of formula (I) or a pharmaceutically acceptable derivative thereof, (1 mg to 100 mg) is aerosolized from a metered dose inhaler to deliver the desired amount of drug per use.

**Example 67: Tablet Formulation**

| Tablets/Ingredients | | Per Tablet |
|---|---|---|
| 1. | Active ingredient | 40 mg |
| | (Compound of formula (I) or pharmaceutically acceptable derivative) | |
| 2. | Corn Starch | 20 mg |
| 3. | Alginic acid | 20 mg |
| 4. | Sodium Alginate | 20 mg |
| 5. | Mg stearate | 1.3 mg |

Procedure for tablet formulation:

Ingredients 1, 2, 3 and 4 are blended in a suitable mixer/blender. Sufficient water is added portionwise to the blend with careful mixing after each addition until the mass is of a consistency to permit its conversion to wet granules. The wet mass is converted to granules by passing it through an oscillating granulator using a No. 8 mesh (2.38 mm) screen. The wet granules are then dried in an oven at 140°F (60°C) until dry. The dry granules are lubricated with ingredient No. 5, and the lubricated granules are compressed on a suitable tablet press.

**Example 68: Parenteral Formulation**

A pharmaceutical composition for parenteral administration is prepared by dissolving an appropriate amount

of a compound of formula (I) in polyethylene glycol with heating. This solution is then diluted with water for injections Ph Eur. (to 100 ml). The solution is then rendered sterile by filtration through a 0.22 micron membrane filter and sealed in sterile containers.

## Claims

**1.** A compound of formula (I);

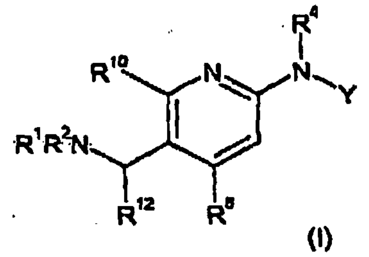

(I)

wherein:

Y is phenyl, unsubstituted or substituted with one, two or three substituents;
$R^1$ is selected from hydrogen, $C_{1-6}$, alkyl, $C_{3-6}$ cycloalkyl, or halosubstituted $C_{1-6}$ alkyl;
$R^2$ is $(CH_2)_mR^5$ where m is 0 or 1;
or $R^1$ and $R^2$ together with N to which they are attached form an unsubstituted or substituted 4- to 8- membered non-aromatic heterocyclyl ring;
$R^3$ is an unsubstituted or substituted 4- to 8- membered non-aromatic heterocyclyl group, an unsubstituted or substituted $C_{3-8}$ cycloalkyl group, an unsubstituted or substituted straight or branched $C_{1-10}$ alkyl, an unsubstituted or substituted $C_{5-7}$ cycloalkenyl, $R^5$ or $R^3$ is an optionally substituted 5- to 6- membered aromatic heterocyclyl group, or group A:

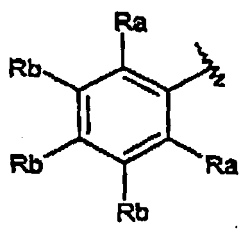

(A)

$R^4$ is selected from hydrogen, $C_{1-6}$ alkyl, $C_{3-6}$ cycloalkyl, or halosubstituted $C_{1-6}$ alkyl, $COCH_3$, or $SO_2Me$;
$R^5$ is

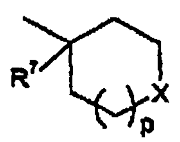

wherein p is 0, 1 or 2, and X is $CH_2$, O, S, SO or $SO_2$;
$R^6$ is halo, a substituted or unsubstituted $(C_{1-6})$alkyl, substituted or unsubstituted $(C_{3-6})$cycloalkyl, or a 4- to 7- membered non-aromatic heterocyclic group and $R^{10}$ is hydrogen or $R^{10}$ is halo, a substituted or unsubstituted $(C_{1-6})$alkyl, substituted or unsubstituted $(C_{3-6})$cycloalkyl, or a 4- to 7- membered non aromatic heterocyclic group and $R^6$ is hydrogen:
$R^7$ is OH, $C_{1-6}$alkoxy, $NR^{8a}R^{8b}$, $NHCOR^9$, $NHSO_2R^9$, $SO_9R^9$
$R^{8a}$ is H or $C_{1-6}$alkyl;

$R^{8b}$ is H or $C_{1-6}$alkyl;
$R^9$ is $C_{1-6}$alkyl;
$R^{12}$ is hydrogen or $C_{1-6}$alkyl;
q is 0, 1 or 2;
Ra can be independently selected from hydrogen, fluoro, chloro or trifluoromethyl;
Rb can be independently selected from hydrogen, $C_{1-6}$ alkyl, $C_{1-6}$ alkoxy, halo$C_{1-6}$ alkoxy, hydroxy, cyano, halo, sulfonyl, $CONH_2$, COOH or $NHCOOC_{1-6}$alkyl;
or a pharmaceutically acceptable salt, ester, salt of such ester or solvate thereof.

**2.** A compound as claimed in Claim 1 wherein the compound of formula (I) is a compound of formula (Ia):

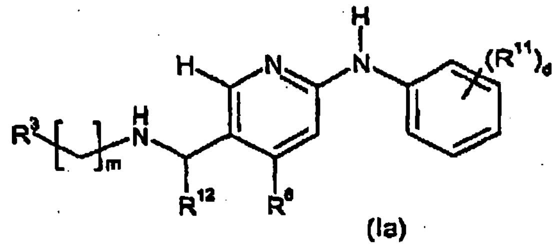

(Ia)

wherein:

$R^3$ is an unsubstituted or substituted 4- to 8- membered non-aromatic heterocyclyl group, an unsubstituted or substituted $C_{3-8}$ cycloalkyl group or a straight or branched $C_{1-6}$alkyl group;
$R^6$ is isopropyl, cyclopropyl, trifluoromethyl, *t*-butyl or cyclopentyl;
$R^{11}$ is selected from halo, cyano, methyl, trifluoromethyl, methoxy or trifluoromethoxy;
$R^{12}$ is hydrogen or $C_{1-6}$alkyl;
d is 0, 1, 2 or 3;
m is 0 or 1;

or a pharmaceutically acceptable salt, ester, salt of such ester or solvate thereof.

**3.** A compound as claimed in Claim 1 wherein the compound of formula (I) is a compound of formula (Ib):

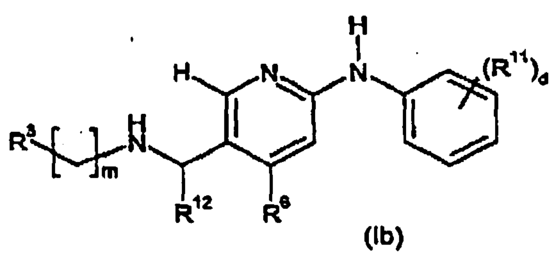

(Ib)

wherein:

$R^3$ is an optionally substituted 5- to 6- membered aromatic heterocyclyl group, or group A;
$R^6$ is isopropyl, cyclopropyl, trifluoromethyl, *t*-butyl or cyclopentyl;
$R^{11}$ is selected from halo, cyano, methyl, trifluoromethyl, methoxy or trifluoromethoxy;
$R^{12}$ is hydrogen or $C_{1-6}$alkyl;
d is 0, 1, 2 or 3;
m is 0 or 1;

or a pharmaceutically acceptable salt, ester, salt of such ester or solvate thereof.

**4.** A compound as claimed in Claim 1 wherein the compound of formula (I) is a compound of formula (Ic):

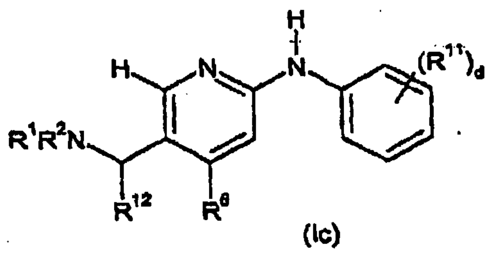

(Ic)

wherein:

$R^1$ and $R^2$ together with N to which they are attached form an unsubstituted or substituted 4- to 8- membered non-aromatio heterocyclyl ring;
$R^6$ is isopropyl, cyclopropyl, trifluoromethyl, *t*-butyl or cyclopentyl;
$R^{11}$ is selected from halo, cyano, methyl, trifluoromethyl, methoxy or trifluoromethoxy;
$R^{12}$ is hydrogen or $C_{1-6}$alkyl;
d is 0, 1, 2 or 3;

or a pharmaceutically acceptable salt, ester, salt of such ester or solvate thereof.

**5.** A compound as claimed in Claim 1 selected from Example 1 to 65.

**6.** A pharmaceutical composition comprising a compound as claimed in any one of claims 1 to 5 or a pharmaceutically acceptable salt, ester, salt of such ester or solvate thereof and a pharmaceutical carrier or diluent.

**7.** A pharmaceutical composition as claimed in claim 6 further comprising a second therapeutic agent.

**8.** A pharmaceutical composition as claimed in claim 7 wherein the second therapeutic agent is a PDE4 inhibitor.

**9.** A compound, salt, ester, salt of such ester or solvate as claimed in any one of claims 1 to 5 for use in the treatment of a condition which is mediated by the activity of cannabinoid 2 receptors.

**10.** A compound, salt, ester, salt of such ester or solvate as claimed in any one of claims 1 to 5 for use in the treatment or prevention of a condition such as an immune disorder, an inflammatory disorder, pain, rheumatoid arthritis, multiple sclerosis, osteoarthritis or osteoporosis.

**11.** Use of a compound, salt, ester, salt of such ester or solvate as claimed in any one of claims 1 to 5 for the manufacture of a therapeutic agent for the treatment or prevention of a condition such as an immune disorder, an inflammatory disorder, pain, rheumatoid arthritis, multiple sclerosis, osteoarthritis or osteoporosis.

**12.** The compound, salt, ester, salt of such ester or solvate as claimed in claim 10 or the use as claimed in claim 11 wherein the pain is selected from inflammatory pain, visceral pain, cancer pain, neuropathic pain, lower back pain, muscular skeletal, post operative pain, acute pain and migraine.

**13.** The compound, salt, ester, salt of such ester or solvate as claimed in claim 10 or the use as claimed in claim 11 wherein the inflammatory disorder is selected from Crohn's disease, ulcerative colitis or inflammatory bowel disease.

## Patentansprüche

**1.** Verbindung der Formel (I);

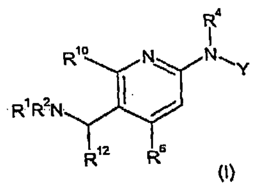

(I)

wobei:

Y Phenyl, unsubstituiert oder substituiert mit einem, zwei oder drei Substituenten ist;
$R^1$ ausgewählt ist aus einem Wasserstoffatom, $C_{1-6}$-Alkyl, $C_{3-6}$-Cycloalkyl oder halogensubstituiertem $C_{1-6}$-Alkyl;
$R^2$ $(CH_2)_mR^3$ ist, wobei m 0 oder 1 ist;
oder $R^1$ und $R^2$ zusammen mit N, an welches sie gebunden sind, einen unsubstituierten oder substituierten 4- bis 8-gliedrigen nicht-aromatischen heterocyclischen Ring bilden;
$R^3$ ein unsubstituierter oder substituierter 4- bis 8-gliedriger nicht-aromatischer heterocyclischer Rest, ein unsubstituierter oder substituierter $C_{3-8}$-Cycloalkylrest, ein unsubstituiertes oder substituiertes, geradkettiges oder verzweigtes $C_{1-10}$-Alkyl, ein unsubstituiertes oder substituiertes $C_{5-7}$-Cycloalkenyl, $R^5$ ist, oder $R^3$ ein gegebenenfalls substituierter 5- bis 6-gliedriger aromatischer heterocyclischer Rest oder Rest A ist:

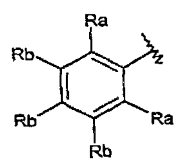

(A)

$R^4$ ausgewählt ist aus einem Wasserstoffatom, $C_{1-6}$-Alkyl, $C_{3-6}$-Cycloalkyl oder halogensubstituiertem $C_{1-6}$-Alkyl, $COCH_3$, oder $SO_2Me$;
$R^5$

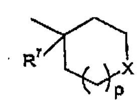

ist,
wobei p 0, 1 oder 2 ist und X $CH_2$, O, S, SO oder $SO_2$ ist;
$R^6$ ein Halogen, ein substituiertes oder unsubstituiertes $(C_{1-6})$Alkyl, substituiertes oder unsubstituiertes $(C_{3-6})$Cycloalkyl oder ein 4- bis 7-gliedriger nicht-aromatischer heterocyclischer Rest ist und $R^{10}$ ein Wasserstoffatom ist oder $R^{10}$ ein Halogen, ein substituiertes oder unsubstituiertes $(C_{1-6})$Alkyl, substituiertes oder unsubstituiertes $(C_{3-6})$Cycloalkyl oder ein 4- bis 7-gliedriger nicht-aromatischer heterocyclischer Rest ist und $R^6$ ein Wasserstoffatom ist;
$R^7$ OH, $C_{1-6}$-Alkoxy, $NR^{8a}R^{8b}$, $NHCOR^9$, $NHSO_2R^9$, $SO_qR^9$ ist;
$R^{8a}$ H oder $C_{1-6}$-Alkyl ist;
$R^{8b}$ H oder $C_{1-6}$-Alkyl ist;
$R^9$ $C_{1-6}$-Alkyl ist;
$R^{12}$ ein Wasserstoffatom oder $C_{1-6}$-Alkyl ist;
q 0, 1 oder 2 ist;
Ra unabhängig ausgewählt sein kann aus einem Wasserstoffatom, einem Fluoratom, einem Chloratom oder Trifluormethyl;
Rb unabhängig ausgewählt sein kann aus einem Wasserstoffatom, $C_{1-6}$-Alkyl, $C_{1-6}$-Alkoxy, Halo-$C_{1-6}$-alkoxy, Hydroxy, Cyano, Halo, Sulfonyl, $CONH_2$, COOH oder $NHCOOC_{1-6}$-alkyl;

oder ein pharmazeutisch verträgliches Salz, Ester, Salz eines solchen Esters oder Solvat davon.

**2.** Verbindung wie in Anspruch 1 beansprucht, wobei die Verbindung der Formel (I) eine Verbindung der Formel (Ia) ist:

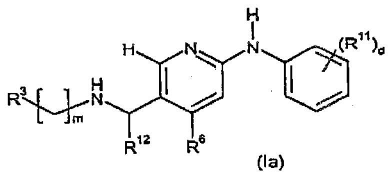

(Ia)

wobei:

$R^3$ ein unsubstituierter oder substituierter 4- bis 8-gliedriger nicht-aromatischer heterocyclischer Rest, ein unsubstituierter oder substituierter $C_{3-8}$-Cycloalkylrest oder ein geradkettiger oder verzweigter $C_{1-6}$-Alkylrest ist;
$R^6$ Isopropyl, Cyclopropyl, Trifluormethyl, *t*-Butyl oder Cyclopentyl ist;
$R^{11}$ ausgewählt ist aus Halogen, Cyano, Methyl, Trifluormethyl, Methoxy oder Trifluormethoxy;
$R^{12}$ ein Wasserstoffatom oder $C_{1-6}$-Alkyl ist;
d 0, 1, 2 oder 3 ist;
m 0 oder 1 ist;

oder ein pharmazeutisch verträgliches Salz, Ester, Salz eines solchen Esters oder Solvat davon.

**3.** Verbindung wie in Anspruch 1 beansprucht, wobei die Verbindung der Formel (I) eine Verbindung der Formel (Ib) ist:

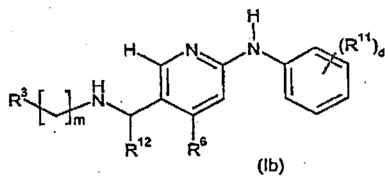

(Ib)

wobei:

$R^3$ ein gegebenenfalls substituierter 5- bis 6-gliedriger aromatischer heterocyclischer Rest oder Rest A ist;
$R^6$ Isopropyl, Cyclopropyl, Trifluormethyl, *t*-Butyl oder Cyclopentyl ist;
$R^{11}$ ausgewählt ist aus Halogen, Cyano, Methyl, Trifluormethyl, Methoxy oder Trifluormethoxy;
$R^{12}$ ein Wasserstoffatom oder $C_{1-6}$-Alkyl ist;
d 0, 1, 2 oder 3 ist;
m 0 oder 1 ist;

oder ein pharmazeutisch verträgliches Salz, Ester, Salz eines solchen Esters oder Solvat davon.

**4.** Verbindung wie in Anspruch 1 beansprucht, wobei die Verbindung der Formel (I) eine Verbindung der Formel (Ic) ist:

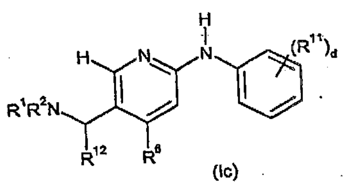

(Ic)

wobei:

$R^1$ und $R^2$ zusammen mit N, an welches sie gebunden sind, einen unsubstituierten oder substituierten 4- bis 8-gliedrigen nicht-aromatischen heterocyclischen Ring bilden;
$R^6$Isopropyl, Cyclopropyl, Trifluormethyl, *t*-Butyl oder Cyclopentyl ist;

$R^{11}$ ausgewählt ist aus Halogen, Cyano, Methyl, Trifluormethyl, Methoxy oder Trifluormethoxy;
$R^{12}$ ein Wasserstoffatom oder $C_{1-6}$-Alkyl ist;
d 0, 1, 2 oder 3 ist;

oder ein pharmazeutisch verträgliches Salz, Ester, Salz eines solchen Esters oder Solvat davon.

**5.** Verbindung wie in Anspruch 1 beansprucht, ausgewählt aus Beispiel 1 bis 65.

**6.** Arzneimittel, umfassend eine Verbindung wie in einem der Ansprüche 1 bis 5 beansprucht, oder ein pharmazeutisch verträgliches Salz, Ester, Salz eines solchen Esters oder Solvat davon und einen pharmazeutischen Träger oder ein pharmazeutisches Verdünnungsmittel.

**7.** Arzneimittel wie in Anspruch 6 beansprucht, weiter umfassend einen zweiten Wirkstoff.

**8.** Arzneimittel wie in Anspruch 7 beansprucht, wobei der zweite Wirkstoff ein PDE4-Inhibitor ist.

**9.** Verbindung, Salz, Ester, Salz eines solchen Esters oder Solvat wie in einem der Ansprüche 1 bis 5 beansprucht zur Verwendung in der Behandlung eines Zustandes, welcher durch die Aktivität des Cannabinoid-2-Rezeptors vermittelt wird.

**10.** Verbindung, Salz, Ester, Salz eines solchen Esters oder Solvat wie in einem der Ansprüche 1 bis 5 beansprucht zur Verwendung in der Behandlung oder Vorbeugung eines Zustandes, wie einer Immunstörung, einer entzündlichen Erkrankung, Schmerz, rheumatoider Arthritis, Multipler Sklerose, Osteoarthritis oder Osteoporose.

**11.** Verwendung einer Verbindung, eines Salzes, eines Esters, eines Salzes eines solchen Esters oder eines Solvats wie in einem der Ansprüche 1 bis 5 beansprucht zur Herstellung eines Arzneimittels zur Behandlung oder Vorbeugung eines Zustandes, wie einer Immunstörung, einer entzündlichen Erkrankung, Schmerz, Rheumatoider Arthritis, Multipler Sklerose, Osteoarthritis oder Osteoporose.

**12.** Verbindung, Salz, Ester, Salz eines solchen Esters oder Solvat wie in Anspruch 10 beansprucht oder Verwendung wie in Anspruch 11 beansprucht, wobei der Schmerz ausgewählt ist aus entzündlichem Schmerz, viszeralem Schmerz, Schmerz welcher durch Krebs verursacht wird, neuropathischem Schmerz, Schmerz im unteren Rückenbereich, postoperativem Schmerz der Skelettmuskulatur, akutem Schmerz und Migräne.

**13.** Verbindung, Salz, Ester, Salz eines solchen Esters oder Solvat wie in Anspruch 10 beansprucht oder Verwendung wie in Anspruch 11 beansprucht, wobei die entzündliche Erkrankung ausgewählt ist aus Crohn-Krankheit, Colitis ulcerosa oder entzündlicher Darmerkrankung.

## Revendications

**1.** Composé de formule (I) :

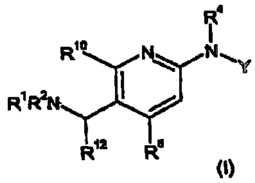

(I)

dans laquelle :

Y représente un groupe phényle, non substitué ou substitué avec un, deux ou trois substituants ;
$R^1$ est choisi entre un atome d'hydrogène, des groupes alkyle en $C_1$ à $C_6$, cycloalkyle en $C_3$ à $C_6$ et alkyle en $C_1$ à $C_6$ à substituant halogéno ;
$R^2$ représente un groupe $(CH_2)_mR^3$ dans lequel m est égal à 0 ou 1 ;
ou bien $R^1$ et $R^2$, conjointement avec l'atome de N auquel ils sont fixés, forment un noyau hétérocyclyle non

aromatique tétra- à octogonal non substitué ou substitué ;
$R^3$ représente un groupe hétérocyclyle non aromatique tétra- à octogonal non substitué ou substitué, un groupe cycloalkyle en $C_3$ à $C_8$ non substitué ou substitué, un groupe alkyle en $C_1$ à $C_{10}$ droit ou ramifié substitué ou non substitué, un groupe cycloalcényle en $C_5$ à $C_7$ non substitué ou substitué, $R^5$ ou $R^3$ représente un groupe hétérocyclyle aromatique penta- ou hexagonal facultativement substitué, ou groupe A :

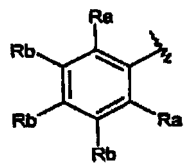

(A)

$R^4$ est choisi entre un atome d'hydrogène, des groupes alkyle en $C_1$ à $C_6$, cycloalkyle en $C_3$ à $C_6$, alkyle en $C_1$ à $C_6$ à substituant halogéno, $COCH_3$ et $SO_2Me$ ;
$R^5$ représente un groupe

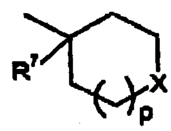

dans lequel p est égal à 0, 1 ou 2 et X représente un groupe $CH_2$, O, S, SO ou $SO_2$ ;
$R^6$ représente un groupe halogéno, alkyle en $C_1$ à $C_6$ substitué ou non substitué, cycloalkyle en $C_3$ à $C_6$ substitué ou non substitué ou un groupe hétérocyclique non aromatique tétra- à heptagonal et $R^{10}$ représente un atome d'hydrogène, ou bien $R^{10}$ représente un groupe halogéno, alkyle en $C_1$ à $C_6$ substitué ou non substitué, cycloalkyle en $C_3$ à $C_6$ substitué ou non substitué ou un groupe hétérocyclique non aromatique tétra- à heptagonal et $R^6$ représente un atome d'hydrogène ;
$R^7$ représente un groupe OH, alkoxy en $C_1$ à $C_6$, $NR^{8a}R^{8b}$, $NHCOR^9$, $NHSO_2R^9$ ou $SO_qR^9$ ,
$R^{8a}$ représente H ou un groupe alkyle en $C_1$ à $C_6$ ;
$R^{8b}$ représente H ou un groupe alkyle en $C_1$ à $C_6$ ;
$R^9$ représente un groupe alkyle en $C_1$ à $C_6$ ;
$R^{12}$ représente un atome d'hydrogène ou un groupe alkyle en $C_1$ à $C_6$ ;
q est égal à 0, 1 ou 2 ;
Ra peut être choisi indépendamment entre un atome d'hydrogène, des groupes fluoro, chloro et trifluorométhyle ;
Rb peut être choisi indépendamment entre un atome d'hydrogène, des groupes alkyle en $C_1$ à $C_6$, alkoxy en $C_1$ à $C_6$, halogénalkoxy en $C_1$ à $C_6$, hydroxy, cyano, halogéno, sulfonyle, $CONH_2$, COOH et NHCOO(alkyle en $C_1$ à $C_6$) ;

ou un de ses sels ou esters pharmaceutiquement acceptables, un sel d'un tel ester, ou un de leurs produits de solvatation.

**2.** Composé suivant la revendication 1, le composé de formule (I) étant un composé de formule (Ia) :

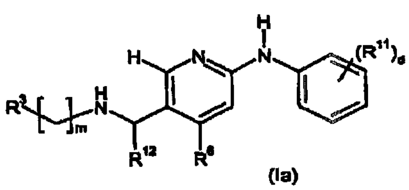

(Ia)

dans laquelle :

$R^3$ représente un groupe hétérocyclyle non aromatique tétra- à octogonal non substitué ou substitué, un groupe cycloalkyle en $C_3$ à $C_8$ non substitué ou substitué ou un groupe alkyle en $C_1$ à $C_6$ droit ou ramifié ;
$R^6$ représente un groupe isopropyle, cyclopropyle, trifluorométhyle, tertiobutyle ou cyclopentyle ;
$R^{11}$ est choisi entre des groupes halogéno, cyano, méthyle, trifluorométhyle, méthoxy et trifluorométhoxy ;
$R^{12}$ représente un atome d'hydrogène ou un groupe alkyle en $C_1$ à $C_6$ ;
d est égal à 0, 1, 2 ou 3 ;
m est égal à 0 ou 1 ;

ou un de ses sels ou esters pharmaceutiquement acceptables, un sel d'un tel ester ou un de leurs produits de solvatation.

**3.** Composé suivant la revendication 1, le composé de formule (I) étant un composé de formule (Ib) :

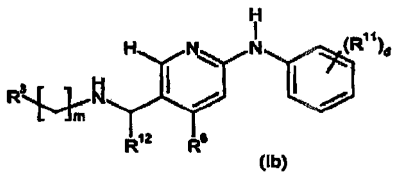

(Ib)

dans laquelle :

$R^3$ représente un groupe hétérocyclyle aromatique penta-ou hexagonal facultativement substitué, ou groupe A ;
$R^6$ représente un groupe isopropyle, cyclopropyle, trifluorométhyle, tertiobutyle ou cyclopentyle ;
$R^{11}$ est choisi entre des groupes halogéno, cyano, méthyle, trifluorométhyle, méthoxy et trifluorométhoxy ;
$R^{12}$ représente un atome d'hydrogène ou un groupe alkyle en $C_1$ à $C_6$ ;
d est égal à 0, 1, 2 ou 3 ;
m est égal à 0 ou 1 ;

ou un de ses sels ou esters pharmaceutiquement acceptables, un sel d'un tel ester ou un de leurs produits de solvatation.

**4.** Composé suivant la revendication 1, le composé de formule (I) étant un composé de formule (Ic) :

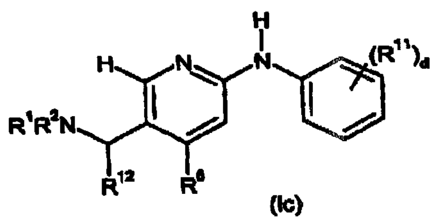

(Ic)

dans laquelle :

$R^1$ et $R^2$, conjointement avec l'atome de N auquel ils sont fixés, forment un noyau hétérocyclyle non aromatique tétra- à octogonal non substitué ou substitué ;
$R^6$ représente un groupe isopropyle, cyclopropyle, trifluorométhyle, tertiobutyle ou cyclopentyle ;
$R^{11}$ est choisi entre des groupes halogéno, cyano, méthyle, trifluorométhyle, méthoxy et trifluorométhoxy ;
$R^{12}$ représente un atome d'hydrogène ou un groupe alkyle en $C_1$ à $C_6$ ;
d est égal à 0, 1, 2 ou 3 ;

ou un de ses sels ou esters pharmaceutiquement acceptables, un sel d'un tel ester ou un de leurs produits de solvatation.

**5.** Composé suivant la revendication 1, choisi parmi les exemples 1 à 65.

**6.** Composition pharmaceutique comprenant un composé suivant l'une quelconque des revendications 1 à 5 ou un

de ses sels ou esters pharmaceutiquement acceptables, un sel d'un tel ester ou un de leurs produits de solvatation et un support ou diluant pharmaceutiquement acceptable.

**7.** Composition pharmaceutique suivant la revendication 6, comprenant en outre un second agent thérapeutique.

**8.** Composition pharmaceutique suivant la revendication 7, dans laquelle le second agent thérapeutique est un inhibiteur de PDE4.

**9.** Composé, sel, ester, sel d'un tel ester ou produit de solvatation suivant l'une quelconque des revendications 1 à 5, destiné à être utilisé dans le traitement d'une affection qui est soumise à une médiation par l'activité des récepteurs de cannabinoïdes 2.

**10.** Composé, sel, ester, sel d'un tel ester ou produit de solvatation suivant l'une quelconque des revendications 1 à 5, destiné à être utilisé dans le traitement ou la prévention d'une affection telle qu'un trouble immunitaire, un trouble inflammatoire, la douleur, la polyarthrite rhumatoïde, la sclérose en plaques, l'arthrose ou l'ostéoporose.

**11.** Utilisation d'un composé, sel, ester, sel d'un tel ester ou produit de solvatation suivant l'une quelconque des revendications 1 à 5, pour la production d'un agent thérapeutique destiné au traitement ou à la prévention d'une affection telle qu'un trouble immunitaire, un trouble inflammatoire, la douleur, la polyarthrite rhumatoïde, la sclérose en plaques, l'arthrose ou l'ostéoporose.

**12.** Composé, sel, ester, sel d'un tel ester ou produit de solvatation suivant la revendication 10 ou utilisation suivant la revendication 11, la douleur étant choisie entre la douleur inflammatoire, la douleur viscérale, la douleur due au cancer, la douleur neuropathique, la douleur dans le bas du dos, la douleur musculo-squelettique, la douleur postopératoire, la douleur aiguë et la migraine.

**13.** Composé, sel, ester, sel d'un tel ester ou produit de solvatation suivant la revendication 10 ou utilisation suivant la revendication 11, le trouble inflammatoire étant choisi entre la maladie de Crohn, la colite ulcérative et la maladie intestinale inflammatoire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5112820 A
- EP 576357 A
- US 4826689 A, to Violanto & Fischer
- US 5145684 A, Liversidge
- US 5298262 A, Na & Rajagopalan
- US 5302401 A, Liversidge
- US 5336507 A, Na & Rajagopalan
- US 5340564 A, Illig & Sarpotdar
- US 5346702 A, Na Rajagopalan
- US 5352459 A, Hollister
- US 5354560 A, Lovrecich
- US 5384124 A, Courteille
- US 5429824 A, June
- US 5503723 A, Ruddy
- US 5510118 A, Bosch
- US 5518 A, Bruno
- US 5518738 A, Eickhoff
- US 5534270 A, De Castro
- US 5536508 A, Canal
- US 5552160 A, Liversidge
- US 5560931 A, Eickhoff
- US 5560932 A, Bagchi
- US 5565188 A, Wong
- US 5571536 A, Eickhoff
- US 5573783 A, Desieno & Stetsko
- US 5580579 A, Ruddy
- US 5585108 A, Ruddy
- US 5587143 A, Wong
- US 5591456 A, Franson
- US 5622938 A, Wong
- US 5662883 A, Bagchi
- US 5665331 A, Bagchi
- US 5718919 A, Ruddy
- US 5747001 A, Wiedmann
- WO 9325190 A
- WO 9624336 A
- WO 9714407 A
- WO 9835666 A
- WO 9965469 A
- WO 0018374 A
- WO 0027369 A
- WO 0030615 A
- WO 0141760 A
- WO 0200196 A
- US 5474995 A
- US 5633272 A
- US 5466823 A
- US 6310099 B
- US 6291523 B
- WO 9625405 A
- WO 9738986 A
- WO 9803484 A
- WO 9714691 A
- WO 9912930 A
- WO 0026216 A
- WO 0052008 A
- WO 0038311 A
- WO 0158881 A
- WO 0218374 A
- US 5998428 A
- US 5449686 A
- US 5552438 A
- WO 9916766 A
- WO 11294 A
- WO 9947505 A
- WO 0113953 A
- WO 2003072554 A, N Sakai


### Non-patent literature cited in the description


- **L.E. HOLLISTER.** Health Aspects of Cannabis. *Pharmacological Reviews,* 1986, vol. 38, 1-20
- **WIRTH et al.** Antiinflammatory Properties ofCannabichrome. *Life Science,* 1990, vol. 26, 1991-1995
- **MATSUDA et al.** Structure of a Cannabinoid Receptor and Functional Expression of the Cloned cDNA. *Nature,* 1990, vol. 346, 561-564
- **MUNRO.** Molecular Characterization of a Peripheral Receptor for Cannabinoids. *Nature,* 1993, vol. 365, 61-65
- **BERGE ; BIGHLEY ; MONKHOUSE.** *J. Pharm. Sci.,* 1977, vol. 66, 1-19
- **LACHMAN et al.** The Theory and Practice of Industrial Pharmacy. 1986, 45
- **LANDELLS, L.J. et al.** *Eur Resp J [Annu Cong Eur Resp Soc,* 19 September 1998, vol. 12 (28
- **FUJI, K. et al.** *J Pharmacol Exp Ther,* 1998, vol. 284 (1), 162
- **BROWN et al.** *Yeast,* 2000, vol. 16, 11-22

- **GUTHRIE ; FINK.** *Methods in Enzymology,* 1991, vol. 194
- *J Med Chem,* 1997, vol. 40, 3207